(19) Europäisches Patentamt
European Patent Office
Office européen des brevets

(11) EP 4 293 011 A1

(12) **EUROPEAN PATENT APPLICATION**
published in accordance with Art. 153(4) EPC

(43) Date of publication:
**20.12.2023 Bulletin 2023/51**

(21) Application number: **21925845.6**

(22) Date of filing: **21.12.2021**

(51) International Patent Classification (IPC):
***C07C 309/67*** *(2006.01)* ***C07C 309/68*** *(2006.01)*
***C07C 309/73*** *(2006.01)* ***C08F 220/12*** *(2006.01)*
***G03F 7/004*** *(2006.01)* ***G03F 7/039*** *(2006.01)*
***G03F 7/20*** *(2006.01)*

(52) Cooperative Patent Classification (CPC):
**C07C 309/67; C07C 309/68; C07C 309/73; C08F 220/12; G03F 7/004; G03F 7/039; G03F 7/20**

(86) International application number:
**PCT/JP2021/047175**

(87) International publication number:
**WO 2022/172602 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.02.2021 JP 2021021928**

(71) Applicant: **FUJIFILM Corporation**
**Tokyo 106-8620 (JP)**

(72) Inventors:
- **MIYOSHI Taro**
  **Haibara-gun, Shizuoka 421-0396 (JP)**
- **YAMAGUCHI Shuhei**
  **Haibara-gun, Shizuoka 421-0396 (JP)**
- **FUKUZAKI Eiji**
  **Haibara-gun, Shizuoka 421-0396 (JP)**
- **YOSHIOKA Tomoaki**
  **Haibara-gun, Shizuoka 421-0396 (JP)**

(74) Representative: **HGF**
**HGF Limited**
**1 City Walk**
**Leeds LS11 9DX (GB)**

# (54) ACTINIC LIGHT-SENSITIVE OR RADIATION-SENSITIVE RESIN COMPOSITION, RESIST FILM, PATTERN FORMATION METHOD, AND METHOD FOR PRODUCING ELECTRONIC DEVICES

(57) An object of the present invention is to provide an actinic ray-sensitive or radiation-sensitive resin composition having excellent sensitivity to exposure. Another object of the present invention is to provide a resist film, a pattern forming method, and a method for manufacturing an electronic device, which relate to the actinic ray-sensitive or radiation-sensitive resin composition. The actinic ray-sensitive or radiation-sensitive resin composition contains a resin which is decomposed by action of acid to increase polarity, in which at least one of a requirement that the actinic ray-sensitive or radiation-sensitive resin composition further contains a compound represented by Formula (1), or a requirement that the resin which is decomposed by action of acid to increase polarity has a residue formed by removing one hydrogen atom from the compound represented by Formula (1) is satisfied.

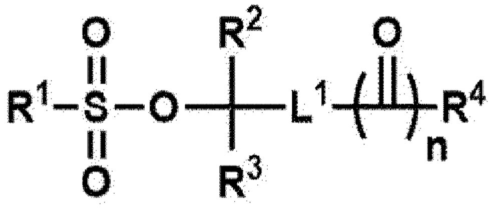

(1)

## Description

## BACKGROUND OF THE INVENTION

1. Field of the Invention

The present invention relates to an actinic ray-sensitive or radiation-sensitive resin composition, a resist film, a pattern forming method, and a method for manufacturing an electronic device.

2. Description of the Related Art

Examples of a pattern forming method include the following methods.

An actinic ray-sensitive or radiation-sensitive resin film (hereinafter, also referred to as "resist film") formed of an actinic ray-sensitive or radiation-sensitive resin composition is exposed to light to cause a change in solubility of the resist film in a developer in a region reflecting the exposed pattern. Thereafter, development is performed using a developer (for example, alkali water-based developer, organic solvent-based developer, or the like) to remove an exposed portion or non-exposed portion of the resist film, thereby obtaining a desired pattern.

For example, JP2008-111120A discloses a photoresist composition containing a photoresist polymer including a repeating unit represented by the following chemical formula, a photoacid generator generating an acid, and an organic solvent, in which, with respect to 100 parts by mass of the photoresist polymer, a content of the photoacid generator is 0.1 to 20 parts by mass and a content of the organic solvent is 300 to 5000 parts by mass (Claims 3 and 6).

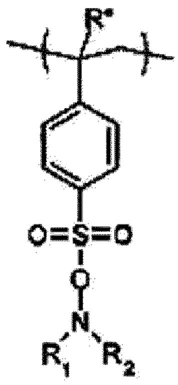

## SUMMARY OF THE INVENTION

The present inventors have studied the photoresist composition (actinic ray-sensitive or radiation-sensitive resin composition) disclosed in JP2008-111120A, and have found that there is room for improvement in sensitivity of the photoresist composition to exposure.

An object of the present invention is to provide an actinic ray-sensitive or radiation-sensitive resin composition having excellent sensitivity to exposure.

Another object of the present invention is to provide a resist film, a pattern forming method, and a method for manufacturing an electronic device, which relate to the actinic ray-sensitive or radiation-sensitive resin composition.

The present inventors have found that the above-described objects can be achieved by the following configurations.

[1] An actinic ray-sensitive or radiation-sensitive resin composition comprising:

a resin which is decomposed by action of acid to increase polarity,
in which at least one of a requirement that the actinic ray-sensitive or radiation-sensitive resin composition further contains a compound represented by Formula (1) described later, or
a requirement that the resin which is decomposed by action of acid to increase polarity has a residue formed by removing one hydrogen atom from the compound represented by Formula (1) is satisfied.

[2] The actinic ray-sensitive or radiation-sensitive resin composition according to [1],
in which the residue includes at least one selected from the group consisting of a group represented by Formula

(2a) described later and a group represented by Formula (2b) described later.
[3] The actinic ray-sensitive or radiation-sensitive resin composition according to [1],
in which $R^4$ represents an aromatic ring group.
[4] The actinic ray-sensitive or radiation-sensitive resin composition according to any one of [1] to [3], further comprising:
a nitrogen-containing basic compound.
[5] The actinic ray-sensitive or radiation-sensitive resin composition according to any one of [1] to [4],

in which the resin which is decomposed by action of acid to increase polarity includes a repeating unit b having a group which is decomposed by action of acid to increase polarity, and
a content of the repeating unit b is 15% by mole or more with respect to all repeating units.

[6] The actinic ray-sensitive or radiation-sensitive resin composition according to any one of [1] to [5],

in which the resin which is decomposed by action of acid to increase polarity includes a repeating unit b having a group which is decomposed by action of acid to increase polarity, and
the repeating unit b includes at least one selected from the group consisting of repeating units represented by Formulae (M1) to (M5) described later.

[7] The actinic ray-sensitive or radiation-sensitive resin composition according to [6],
in which the repeating unit b includes at least one selected from the group consisting of the repeating unit represented by Formula (M4) and the repeating unit represented by Formula (M5).
[8] A resist film formed of the actinic ray-sensitive or radiation-sensitive resin composition according to any one of [1] to [7].
[9] A pattern forming method comprising:

a step of forming a resist film on a substrate using the actinic ray-sensitive or radiation-sensitive resin composition according to any one of [1] to [7];
a step of exposing the resist film; and
a step of developing the exposed resist film using a developer.

[10] A method for manufacturing an electronic device, comprising:
the pattern forming method according to [9].

According to the present invention, it is possible to provide an actinic ray-sensitive or radiation-sensitive resin composition having excellent sensitivity to exposure.
In addition, according to the present invention, it is possible to provide a resist film, a pattern forming method, and a method for manufacturing an electronic device, which relate to the actinic ray-sensitive or radiation-sensitive resin composition.

## DESCRIPTION OF THE PREFERRED EMBODIMENTS

Hereinafter, the present invention will be described in detail.
Description of configuration requirements described below may be made on the basis of representative embodiments of the present invention in some cases, but the present invention is not limited to such embodiments.
Meanings of the following notations in the present specification are described.
In notations for a group (atomic group), in a case where the group is cited without specifying that it is substituted or unsubstituted, the group includes both a group having no substituent and a group having a substituent as long as it does not impair the spirit of the present invention. For example, an "alkyl group" includes not only an alkyl group having no substituent (unsubstituted alkyl group), but also an alkyl group having a substituent (substituted alkyl group).
"Organic group" refers to a group including at least one carbon atom.
A substituent is a monovalent substituent unless otherwise specified.
"Actinic ray" or "radiation" means, for example, a bright line spectrum of a mercury lamp, far ultraviolet rays typified by an excimer laser, extreme ultraviolet rays (EUV light), X-rays, electron beams (EB), or the like.
"Light" means actinic ray or radiation.
Unless otherwise specified, "exposure" encompasses not only exposure by a bright line spectrum of a mercury lamp, far ultraviolet rays typified by an excimer laser, extreme ultraviolet rays, X-rays, EUV light, or the like, but also drawing by particle beams such as electron beams and ion beams.

"~" is used to mean that numerical values described before and after a range are included as the lower limit and the upper limit.

A bonding direction of divalent groups cited in the present specification is not limited unless otherwise specified. For example, in a case where Y in a compound represented by Formula "X-Y-Z" is -COO-, Y may be -CO-O- or -O-CO-. In addition, the above-described compound may be "X-CO-O-Z" or "X-O-CO-Z".

(Meth)acrylate represents acrylate and methacrylate.

(Meth)acrylic represents acrylic and methacrylic.

A weight-average molecular weight (Mw), a number-average molecular weight (Mn), and a dispersity (hereinafter, also referred to as "molecular weight distribution") (Mw/Mn) of a resin are defined as values expressed in terms of polystyrene by means of gel permeation chromatography (GPC) measurement (solvent: tetrahydrofuran, flow amount (amount of a sample injected): 10 μL, columns: TSK gel Multipore HXL-M manufactured by Tosoh Corporation, column temperature: 40°C, flow rate: 1.0 mL/min, and detector: differential refractive index detector) using a GPC apparatus (HLC-8120GPC manufactured by Tosoh Corporation).

A compositional ratio (molar ratio, mass ratio, or the like) of a resin is measured by $^{13}$C-nuclear magnetic resonance (NMR).

An acid dissociation constant (pKa) represents a pKa in an aqueous solution, and is specifically a value determined by computation from a value based on a Hammett's substituent constant and database of publicly known literature values, using the following software package 1. Any of the pKa values described in the present specification indicates values determined by computation using the software package.

Software Package 1: Advanced Chemistry Development (ACD/Labs) Software V 8.14 for Solaris (1994 - 2007 ACD/Labs).

On the other hand, the pKa can also be determined by a molecular orbital computation method. Examples of a specific method therefor include a method for performing calculation by computing $H^+$ dissociation free energy in an aqueous solution based on a thermodynamic cycle. With regard to a computation method for $H^+$ dissociation free energy, the $H^+$ dissociation free energy can be computed by, for example, density functional theory (DFT), but various other methods have been reported in literature and the like, and are not limited thereto. There are a plurality of software applications capable of performing DFT, and examples thereof include Gaussian 16.

As described above, the pKa means a value determined by computation from a value based on a Hammett's substituent constant and database of publicly known literature values, using the software package 1, but in a case where the pKa cannot be calculated by the method, a value obtained by Gaussian 16 based on density functional theory (DFT) shall be adopted.

In addition, the pKa means a "pKa in an aqueous solution" as described above, but in a case where the pKa in an aqueous solution cannot be calculated, a "pKa in a dimethyl sulfoxide (DMSO) solution" is adopted.

"Solid content" is intended to be components which form the resist film, and does not include a solvent. In addition, even in a case where a component is liquid, the component is included in the solid content as long as the component forms the resist film.

1 inch means 25.4 mm.

[Actinic Ray-Sensitive or Radiation-Sensitive Resin Composition]

The actinic ray-sensitive or radiation-sensitive resin composition (hereinafter, also referred to as "resist composition") according to the embodiment of the present invention is a resist composition containing a resin which is decomposed by action of acid to increase polarity, in which at least one of a requirement that the actinic ray-sensitive or radiation-sensitive resin composition further contains a compound (hereinafter, also referred to as "compound (1)") represented by Formula (1), or a requirement that the resin which is decomposed by action of acid to increase polarity has a residue (hereinafter, also referred to as "specific group") formed by removing one hydrogen atom from the compound represented by Formula (1) is satisfied.

Hereinafter, the resin which has the specific group and is decomposed by action of acid to increase polarity is also simply referred to as "resin A".

A working mechanism by which sensitivity to exposure is improved by adopting such configurations is not always clear, but is presumed to be as follows by the present inventors.

First, in a resist film formed of a typical chemically amplified resist composition, an acid generated from a photoacid generator by exposure acts on a resin which is decomposed by action of acid to increase polarity, and causes decomposition of a group (hereinafter, also referred to as "acid-decomposable group") in the resin, which is decomposed by action of acid to increase polarity, thereby causing a change in polarity in the exposed portion. In this case, the present inventors have found that sensitivity of the resist composition to exposure varies depending on ease of decomposition

of the light-received photoacid generator.

On the other hand, the resist composition according to the embodiment of the present invention contains the compound (1) or the resin A. Since the compound (1) or the specific group in the resin A is easily decomposed during exposure, an acid is likely to be generated. As a result, it is presumed that the sensitivity of the resist composition to exposure is excellent.

Hereinafter, the fact that the sensitivity to exposure is more excellent is also referred to that the effect of the present invention is more excellent.

Hereinafter, the resist composition according to the embodiment of the present invention will be described in detail.

The resist composition may be either a positive tone resist composition or a negative tone resist composition. In addition, the resist composition may be either a resist composition for alkali development or a resist composition for organic solvent development.

The resist composition may be a non-chemically amplified resist composition, or may have a mechanism as a chemically amplified resist composition in combination with the resist composition.

Hereinafter, various components of the resist composition will be described in detail.

[Compound (1)]

The compound (1) is a compound represented by Formula (1).

The compound (1) can also function as a photoacid generator described later.

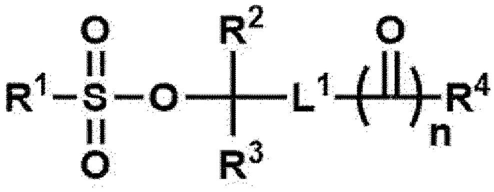

(1)

In Formula (1), $R^1$ and $R^4$ each independently represent a substituent, $R^2$ and $R^3$ each independently represent a hydrogen atom or a substituent, $L^1$ represents a single bond or a divalent linking group, and n represents an integer of 1 or more.

$R^1$ and $R^4$ each independently represent a substituent.

The above-described substituent is not particularly limited, and examples thereof include a hydroxyl group, a thiol group, an amino group, a sulfonic acid group, an organic group, and a group formed by a combination of these groups. Among these, an organic group is preferable, and an alkyl group, an alkoxy group, an alkoxycarbonyl group, an alkenyl group, a cyano group, a cycloalkyl group, or an aromatic ring group is more preferable.

The above-described alkyl group may be linear or branched.

The number of carbon atoms in the above-described alkyl group is preferably 1 to 5.

Examples of the above-described alkyl group include a methyl group, an ethyl group, an n-propyl group, an isopropyl group, an n-butyl group, an isobutyl group, and a t-butyl group.

Examples of an alkyl group moiety in the above-descried alkoxy group and the above-described alkoxycarbonyl group include the same groups as those in the above-described alkyl group.

The above-described alkenyl group may be linear or branched.

The number of carbon atoms in the above-described alkenyl group is preferably 1 to 5.

Examples of the above-described alkenyl group include a vinyl group.

The number of ring member atoms in the above-described cycloalkyl group is preferably 3 to 15.

As the above-described cycloalkyl, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable, and a polycyclic cycloalkyl group is more preferable.

In the above-described cycloalkyl group, one or more (for example, one to three) of methylene groups constituting the ring may be replaced with a heteroatom (for example, -O-, -S-, and the like), $-SO_2-$, $-SO_3-$, an alkoxycarbonyl group, a carbonyl group, or a vinylidene group. In addition, in the above-described cycloalkyl group, one or more (for example, one or two) of ethylene groups constituting the cycloalkane ring may be replaced with a vinylene group.

The above-described aromatic ring group may be monocyclic or polycyclic.

The number of ring member atoms in the above-described aromatic ring group is preferably 5 to 15.

Ring member atoms of the above-described aromatic ring group may have one or more (for example, one to five) heteroatoms (for example, oxygen atom, sulfur atom, nitrogen atom, and/or the like).

Examples of the above-described aromatic ring group include aryl groups such as a benzene ring group, a naphthalene ring group, and an anthracene ring group, and thiazole ring groups such as a benzothiazole ring group.

The alkyl group, the alkoxy group, the alkoxycarbonyl group, the alkenyl group, the cycloalkyl group, and the aromatic ring group described above may further have a substituent.

Examples of the above-described substituent include a halogen atom (for example, a fluorine atom and the like), a hydroxyl group, a nitro group, a cyano group, a cycloalkyl group, and an aromatic ring group. Specifically, the above-described alkyl group may have a fluorine atom as the substituent and may be a perfluoroalkyl group. Examples of the cycloalkyl group and the aromatic ring group as the substituent include the above-described cycloalkyl group and the above-described aromatic ring group, which can be adopted as the substituent represented by $R^1$ and $R^4$.

As described above, the alkyl group, the alkoxy group, the alkoxycarbonyl group, the alkenyl group, the cycloalkyl group, and the aromatic ring group described above may further have a substituent, and this substituent may further have a substituent (hereinafter, also referred to as "substituent X"). For example, the cycloalkyl group and the aromatic ring group described above may further have a substituent.

Examples of the above-described substituent X include a halogen atom, a hydroxyl group, a nitro group, a cyano group, an alkyl group, an alkoxy group, an alkoxycarbonyl group, and an alkenyl group. Examples of the alkyl group, the alkoxy group, the alkoxycarbonyl group, and the alkenyl group as the substituent X include the above-described alkyl group, the above-described alkoxy group, the above-described alkoxycarbonyl group, and the above-described alkenyl group, which can be adopted as the substituent represented by $R^1$ and $R^4$. The alkyl group, the alkoxy group, the alkoxycarbonyl group, and the alkenyl group as the substituent X may further have the above-described substituent.

As $R^1$, an alkyl group, a cycloalkyl group, or an aromatic ring group is preferable, and an alkyl group, a cycloalkyl group, or an aryl group is more preferable.

As $R^4$, an alkyl group, an alkoxy group, or an aromatic ring group is preferable, and from the viewpoint of more excellent sensitivity, an aromatic ring group is more preferable and an aryl group is still more preferable.

$R^2$ and $R^3$ each independently represent a hydrogen atom or a substituent.

Examples of the above-described substituent include the substituent in $R^1$ and $R^4$ described above.

As $R^2$ and $R^3$, a hydrogen atom, an alkyl group, or an aryl group is preferable.

At least two of $R^2$ to $R^4$ may be bonded to each other to form a ring.

It is preferable that $R^2$ or $R^3$, and $R^4$ are bonded to each other to form a ring. The ring formed by the above-described bonding may be monocyclic or polycyclic. The number of ring member atoms in the above-described ring is preferably 4 to 15.

$L^1$ represents a single bond or a divalent linking group.

Examples of the above-described divalent linking group include -CO-, -O-, -S, -SO-, $-SO_2-$, $-NR^N-$, a hydrocarbon group (for example, an alkylene group, a cycloalkylene group, an alkenylene group, an arylene group, and the like), and a group formed by a combination of these groups. $R^N$ represents a substituent (for example, the substituent which can be included in $R^1$ to $R^4$ described above). The above-described hydrocarbon group may further have a substituent, and preferably has a halogen atom as the substituent.

The above-described alkylene group may be linear or branched.

The number of carbon atoms in the above-described alkylene group is preferably 1 to 4.

The above-described cycloalkylene group may be monocyclic or polycyclic.

The number of carbon atoms in the above-described cycloalkylene group is preferably 3 to 15.

One or more (for example, one or two) of $-CH_2-$'s constituting a ring structure of the above-described cycloalkylene group may be replaced with a heteroatom (for example, -O-, -S-, and the like), $-SO_2-$, $-SO_3-$, an alkoxycarbonyl group, or a carbonyl group.

As $L^1$, a single bond or an alkylene group is preferable, and a single bond is more preferable.

n represents an integer of 1 or more.

n is preferably 1 to 5, more preferably 1 to 3, still more preferably 1 or 2, and particularly preferably 1.

Examples of the compound (1) include the following compounds.

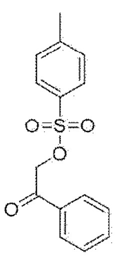 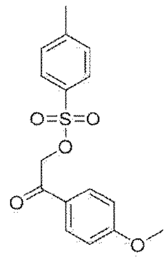 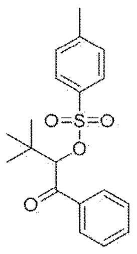 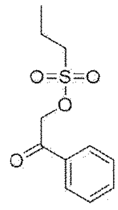 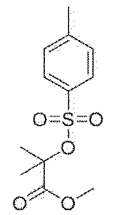 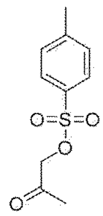

B-1 B-2 B-3 B-4 B-5 B-6

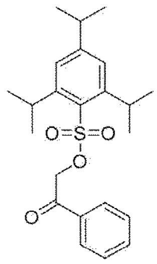 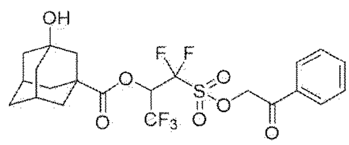 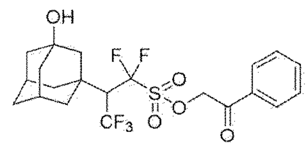

B-7 B-8 B-9

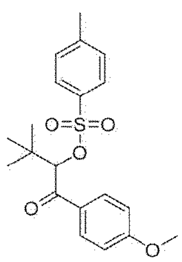 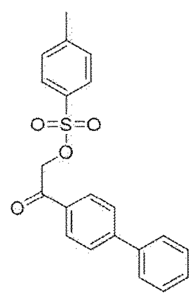

B-10 B-11

In a case where the resist composition contains the compound (1), a content of the compound (1) is preferably 0.5% by mass or more and more preferably 1% by mass or more with respect to the total solid content of the resist composition. The upper limit thereof is preferably 40% by mass or less and more preferably 30% by mass or less with respect to the total solid content of the resist composition.

The compound (1) may be used alone or in combination of two or more kinds thereof. In a case where two or more kinds thereof are used, a total content thereof is preferably within the suitable content range.

[Resin which is Decomposed by Action of Acid to Increase Polarity]

The resin which is decomposed by action of acid to increase polarity (hereinafter, also simply referred to as "acid-decomposable resin") has an acid-decomposable group. The "acid-decomposable group" means a group which is decomposed by action of acid to form a polar group. The acid-decomposable group preferably has a structure in which a polar group is protected by a leaving group which is eliminated by action of acid. The acid-decomposable group may be decomposed by action of acid to form a polar group.

The acid-decomposable group will be described later.

In the pattern forming method according to the embodiment of the present invention, typically, in a case where an alkali developer is adopted as a developer, a positive tone pattern is suitably formed, and in a case where an organic developer is adopted as a developer, a negative tone pattern is suitably formed.

As described above, the acid-decomposable resin may be a resin having the specific group. That is, the acid-decomposable resin may be the resin A.

The resin A is preferably a resin which has a repeating unit (hereinafter, also simply referred to as "repeating unit a") having the specific group that is a non-ionic group which is decomposed by irradiation (exposure) with actinic ray or radiation.

The specific group is decomposed by irradiation with actinic ray or radiation to generate a polar group.

That is, the resin A has a repeating unit having a group which is decomposed by exposure to generate a polar group. For example, the resin A usually has an increased polarity by the exposure, an increased solubility in an alkali developer, and a decreased solubility in an organic solvent.

(Specific Group)

The specific group is a residue formed by removing one hydrogen atom from the above-described compound (1).
The specific group is preferably a residue formed by removing one hydrogen atom from $R^1$ or $R^4$ in the compound (1), more preferably includes at least one selected from the group consisting of a group represented by Formula (2a) and a group represented by Formula (2b), and is still more preferably a group represented by Formula (2a).
An another suitable aspect of the specific group, a residue formed by removing one hydrogen atom from the compound (1) in which $R^4$ is an aromatic ring group is preferable.

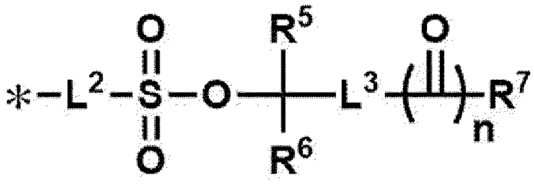

(2a)

In Formula (2a), $R^5$ and $R^6$ each independently represent a hydrogen atom or a substituent, $R^7$ represents a substituent, $L^2$ represents a divalent linking group, $L^3$ represents a single bond or a divalent linking group, n represents an integer of 1 or more, and * represents a bonding position.
$R^5$, $R^6$, $R^7$, $L^3$, and n have the same meanings as $R^2$, $R^3$, $R^4$, $L^1$, and n in (1) described above, and suitable aspects thereof are also the same.
$L^2$ represents a divalent linking group.
As $L^2$, a group formed by removing one hydrogen atom from $R^1$ in Formula (1) is preferable.
A suitable aspect of $R^1$ is as described above.

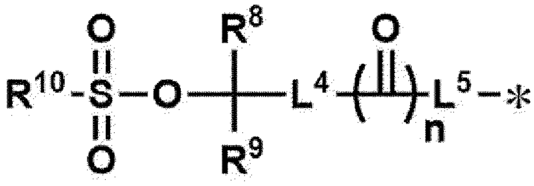

(2b)

In Formula (2b), $R^8$ and $R^9$ each independently represent a hydrogen atom or a substituent, $R^{10}$ represents a substituent, $L^4$ represents a single bond or a divalent linking group, $L^5$ represents a divalent linking group, n represents an integer of 1 or more, and * represents a bonding position.
$R^8$, $R^9$, $R^{10}$, $L^4$, and n have the same meanings as $R^2$, $R^3$, $R^1$, $L^1$, and n in (1) described above, and suitable aspects thereof are also the same.
$L^5$ represents a divalent linking group.
As $L^5$, a group formed by removing one hydrogen atom from $R^4$ in Formula (1) is preferable.
A suitable aspect of $R^4$ is as described above.

<Repeating Unit a>

The repeating unit a is a repeating unit having the specific group.
The specific group may be directly bonded to a main chain of the repeating unit a, or the specific group may form a part of the main chain of the repeating unit a. Specifically, in a case where the main chain is an ethylene chain, a part of the ethylene chain may be composed of the specific group.

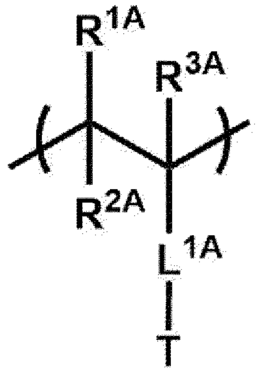

(a)

In Formula (a), $R^{1A}$ to $R^{3A}$ each independently represent a hydrogen atom or a substituent, $L^{1A}$ represents a single bond or a divalent linking group, and T represents the specific group.

$R^{1A}$ to $R^{3A}$ each independently represent a hydrogen atom or a substituent.

$R^{1A}$ to $R^{3A}$ are each independently preferably a hydrogen atom, an alkyl group, a cycloalkyl group, a halogen atom, a cyano group, or an alkoxycarbonyl group; more preferably a hydrogen atom, a halogen atom, or an alkyl group; and still more preferably a hydrogen atom or a methyl group.

$L^{1A}$ represents a single bond or a divalent linking group.

Examples of the above-described divalent linking group include -CO-, -O-, -S, -SO-, $-SO_2-$, $-NR^N-$, a hydrocarbon group (for example, an alkylene group, a cycloalkylene group, an alkenylene group, an arylene group, and the like), and a group formed by a combination of these groups. $R^N$ represents a substituent (for example, the substituent which can be included in $R^1$ to $R^4$ described above). The above-described hydrocarbon group may further have a substituent, and preferably has a halogen atom (preferably a fluorine atom) as the substituent.

The above-described alkylene group may be linear or branched.

The number of carbon atoms in the above-described alkylene group is preferably 1 to 4.

The above-described cycloalkylene group may be monocyclic or polycyclic.

The number of carbon atoms in the above-described cycloalkylene group is preferably 3 to 15.

One or more (for example, one or two) of $-CH_2-$'s constituting a ring structure of the above-described cycloalkylene group may be replaced with a heteroatom (for example, -O-, -S-, and the like), $-SO_2-$, $-SO_3-$, an alkoxycarbonyl group, or a carbonyl group.

T represents the specific group.

The specific group is as described above.

It is more preferable that the resin A has at least one selected from the group consisting of repeating units represented by Formulae (aa) to (ac).

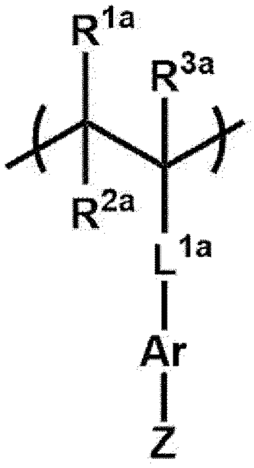
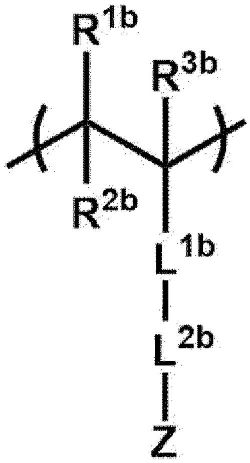
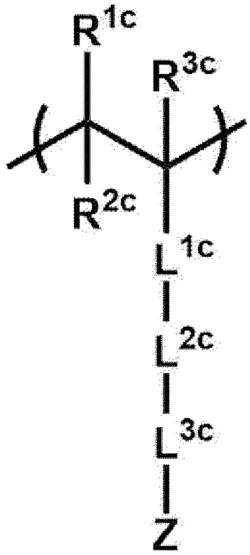

(aa) (ab) (ac)

In Formula (aa), $R^{1a}$ to $R^{3a}$ each independently represent a hydrogen atom or a substituent, $L^{1a}$ represents a single bond, an alkylene group, -COO-, an aromatic ring group, or a group formed by a combination of these groups, Ar represents an aromatic ring group, and Z represents a group represented by Formula (Za) or a group represented by Formula (Zb).

$R^{1a}$ to $R^{3a}$ each independently represent a hydrogen atom or a substituent.

$R^{1a}$ to $R^{3a}$ are each independently preferably a hydrogen atom, an alkyl group, a cycloalkyl group, a halogen atom, a cyano group, or an alkoxycarbonyl group; more preferably a hydrogen atom, a halogen atom, or an alkyl group; and still more preferably a hydrogen atom or a methyl group.

$L^{1a}$ represents a single bond, an alkylene group, -COO-, an aromatic ring group, or a group formed by a combination of these groups.

The above-described alkylene group may be linear or branched.

The number of carbon atoms in the above-described alkylene group is preferably 1 to 4.

The above-described aromatic ring group may be monocyclic or polycyclic.

The number of ring member atoms in the above-described aromatic ring group is preferably 5 to 15. The above-described aromatic ring group may have one or more (for example, one to five) heteroatoms (oxygen atom, sulfur atom, nitrogen atom, and/or the like) as the ring member atom. As the above-described aromatic ring group, a benzene ring group is preferable.

Examples of the combined group described above include -COO-alkylene group and -COO-aromatic ring group-.

The alkylene group and aromatic ring group described above may further have a substituent.

Examples of the above-described substituent include the substituents which can be adopted as $R^1$ and $R^4$ in Formula (1).

As $L^{1a}$, a single bond or an alkylene group is preferable, and a single bond is more preferable.

Ar represents an aromatic ring group.

The above-described aromatic ring group may be monocyclic or polycyclic.

The number of ring member atoms in the above-described aromatic ring group is preferably 5 to 15.

The above-described aromatic ring group may have one or more (for example, one to five) heteroatoms (oxygen atom, sulfur atom, nitrogen atom, and/or the like) as the ring member atom.

The above-described aromatic ring group may further have a substituent.

Examples of the above-described substituent include the substituents which can be adopted as $R^1$ and $R^4$ in Formula (1).

In addition, examples of the above-described substituent also include the group represented by Formula (Za) and the group represented by Formula (Zb).

As the above-described aromatic ring group, an arylene group such as a phenylene group and a naphthylene group is preferable, and a phenylene group is more preferable.

Z represents a group represented by Formula (Za) or a group represented by Formula (Zb).

As Z, a group represented by Formula (Za) is preferable.

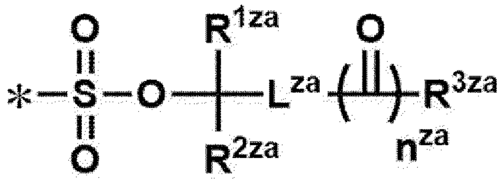

(Za)

In Formula (Za), $R^{1za}$ and $R^{2za}$ each independently represent a hydrogen atom or a substituent, $R^{3za}$ represents a substituent, $L^{za}$ represents a single bond or a divalent linking group, $n^{za}$ represents an integer of 1 or more, and * represents a bonding position.

$R^{1za}$, $R^{2za}$, $R^{3za}$, $L^{za}$, and $n^{za}$ have the same meanings as $R^2$, $R^3$, $R^4$, $L^1$, and n in (1) described above, and suitable aspects thereof are also the same.

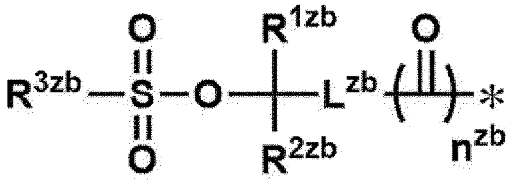

(Zb)

In Formula (Zb), $R^{1zb}$ and $R^{2zb}$ each independently represent a hydrogen atom or a substituent, $R^{3zb}$ represents a substituent, $L^{zb}$ represents a single bond or a divalent linking group, $n^{zb}$ represents an integer of 1 or more, and * represents a bonding position.

$R^{1zb}$, $R^{2zb}$, $R^{3zb}$, $L^{zb}$, and $n^{zb}$ have the same meanings as $R^2$, $R^3$, $R^1$, $L^1$, and n in (1) described above, and suitable aspects thereof are also the same.

In Formula (ab), $R^{1b}$ to $R^{3b}$ each independently represent a hydrogen atom or a substituent, $L^{1b}$ represents a single bond, an alkylene group, -COO-, or a group formed by a combination of these groups, $L^{2b}$ represents a single bond or an alkylene group, and Z represents a group represented by Formula (Za) or a group represented by Formula (Zb).

$R^{1b}$ to $R^{3b}$ each independently represent a hydrogen atom or a substituent.

$R^{1b}$ to $R^{3b}$ are each independently preferably a hydrogen atom, an alkyl group, a cycloalkyl group, a halogen atom, a cyano group, or an alkoxycarbonyl group; more preferably a hydrogen atom, a halogen atom, or an alkyl group; and still more preferably a hydrogen atom or a methyl group.

$L^{1b}$ represents a single bond, an alkylene group, -COO-, or a group formed by a combination of these groups.

The above-described alkylene group may be linear or branched.

The number of carbon atoms in the above-described alkylene group is preferably 1 to 4.

Examples of the combined group described above include -COO-alkylene group.

The above-described alkylene group may further have a substituent.

Examples of the above-described substituent include the substituents which can be adopted as $R^1$ and $R^4$ in Formula (1).

As $L^{1b}$, a single bond or an alkylene group is preferable, and a single bond is more preferable.

$L^{2b}$ represents a single bond or an alkylene group.

Examples of the above-described alkylene group include the alkylene group which can be adopted as $L^{1A}$ in Formula (a). In addition, examples of the above-described substituent also include the group represented by Formula (Za) and the group represented by Formula (Zb).

As $L^{2b}$, an alkylene group is preferable.

Z represents a group represented by Formula (Za) or a group represented by Formula (Zb).

The group represented by Formula (Za) and the group represented by Formula (Zb) are as described above.

In Formula (ac), $R^{1c}$ to $R^{3c}$ each independently represent a hydrogen atom or a substituent, $L^{1c}$ represents a single bond, an alkylene group, -COO-, an aromatic ring group, or a group formed by a combination of these groups, $L^{2c}$ represents a cycloalkylene group, $L^{3c}$ represents a single bond or a divalent linking group, and Z represents a group represented by Formula (Za) or a group represented by Formula (Zb).

$R^{1c}$ to $R^{3c}$ each independently represent a hydrogen atom or a substituent.

$R^{1c}$ to $R^{3c}$ are each independently preferably a hydrogen atom, an alkyl group, a cycloalkyl group, a halogen atom, a cyano group, or an alkoxycarbonyl group; more preferably a hydrogen atom, a halogen atom, or an alkyl group; and still more preferably a hydrogen atom or a methyl group.

$L^{1c}$ represents a single bond, an alkylene group, -COO-, an aromatic ring group, or a group formed by a combination of these groups.

Examples of $L^{1c}$ include $L^{1a}$ in Formula (aa).

As $L^{1c}$, a single bond, an alkylene group, -COO-, or a group formed by a combination of these groups is preferable, and -COO- is more preferable.

$L^{2c}$ represents a cycloalkylene group.

The above-described cycloalkylene group may be monocyclic or polycyclic.

The number of carbon atoms in the above-described cycloalkylene group is preferably 3 to 15.

One or more (for example, one or two) of $-CH_2-$'s constituting a ring structure of the above-described cycloalkylene group may be replaced with a heteroatom (for example, -O-, -S-, and the like), $-SO_2-$, $-SO_3-$, an alkoxycarbonyl group, or a carbonyl group.

The above-described cycloalkylene group may further have a substituent.

Examples of the above-described substituent include the substituents which can be adopted as $R^1$ and $R^4$ in Formula (1).

In addition, examples of the above-described substituent also include the group represented by Formula (Za) and the group represented by Formula (Zb).

$L^{3c}$ represents a single bond or a divalent linking group.

Examples of the above-described divalent linking group include -CO-, -O-, -S, -SO-, $-SO_2-$, $-NR^N-$, a hydrocarbon group (for example, an alkylene group, a cycloalkylene group, an alkenylene group, an arylene group, and the like), and a group formed by a combination of these groups. $R^N$ represents a substituent (for example, the substituent which can be included in $R^1$ to $R^4$ described above). The above-described hydrocarbon group may further have a substituent, and preferably has a halogen atom (preferably a fluorine atom) as the substituent. In addition, examples of the above-described substituent also include the group represented by Formula (Za) and the group represented by Formula (Zb).

As $L^{3c}$, an alkylene group which may have a fluorine atom or an alkoxycarbonyl group which may have a fluorine atom is preferable.

Z represents a group represented by Formula (Za) or a group represented by Formula (Zb).

The group represented by Formula (Za) and the group represented by Formula (Zb) are as described above.

Examples of the repeating unit a include the following repeating units.

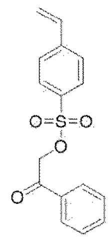
M-37

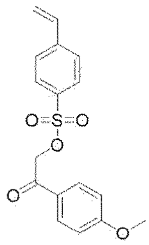
M-38

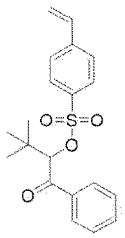
M-39

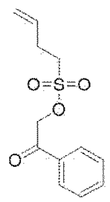
M-40

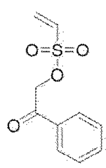
M-41

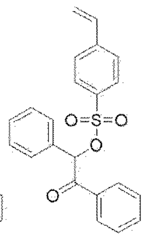
M-42

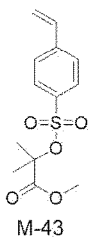
M-43

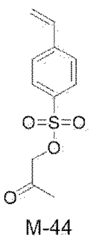
M-44

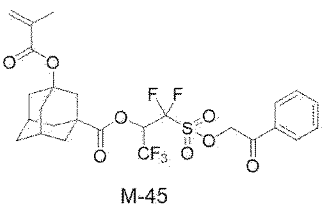
M-45

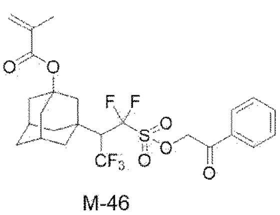
M-46

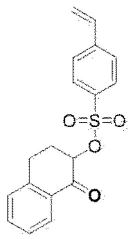 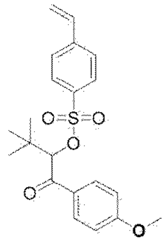 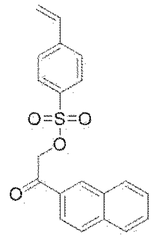 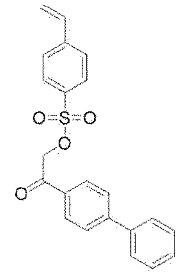 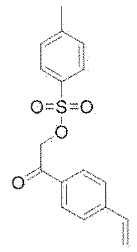

M-47 M-48 M-49 M-50 M-51

A content of the repeating unit a is preferably 1% by mole or more, more preferably 5% by mole or more, and still more preferably 10% by mole or more with respect to all repeating units in the acid-decomposable resin. The upper limit thereof is preferably less than 100% by mole, more preferably 90% by mole or less, and still more preferably 70% by mole or less with respect to all repeating units.

The repeating unit a may be used alone or in combination of two or more kinds thereof. In a case where two or more kinds of the repeating units a are used, a total content thereof is preferably within the content range.

In a case where two or more kinds of the resins A are present, it is preferable to contain one or more kinds of resins A in which the content of the repeating unit a is 40% by mole or more with respect to all repeating units. In this case, it is preferable to contain the resin A in which the content of the repeating unit a is 40% by mole or more with respect to all repeating units in an amount of 30% to 100% by mass with respect to the total content of the resin A, more preferably 60% to 100% by mass, and still more preferably 80% to 100% by mass.

<Repeating Unit having Acid-decomposable Group>

The acid-decomposable resin may have a repeating unit having an acid-decomposable group (hereinafter, also simply referred to as "repeating unit b").

The acid-decomposable group does not include the above-described specific group. In addition, the acid-decomposable group does not have the specific group as a part thereof. That is, the repeating unit b does not include the repeating unit a.

As the polar group, an alkali-soluble group is preferable.

Examples of the alkali-soluble group include an acidic group, such as a carboxy group, a phenolic hydroxyl group, a fluorinated alcohol group, a sulfonic acid group, a phosphoric acid group, a sulfonamide group, a sulfonylimide group, an (alkylsulfonyl)(alkylcarbonyl)methylene group, an (alkylsulfonyl)(alkylcarbonyl)imide group, a bis(alkylcarbonyl)methylene group, a bis(alkylcarbonyl)imide group, a bis(alkylsulfonyl)methylene group, a bis(alkylsulfonyl)imide group, a tris(alkylcarbonyl)methylene group, and a tris(alkylsulfonyl)methylene group; and an alcoholic hydroxyl group.

As described above, the acid-decomposable group preferably has a structure in which a polar group is protected by a leaving group which is eliminated by action of acid.

Examples of the leaving group which is eliminated by action of acid include groups represented by Formulae (Y1) to (Y4).

Formula (Y1): $-C(Rx_1)(Rx_2)(Rx_3)$

Formula (Y2): $-C(=O)OC(Rx_1)(Rx_2)(Rx_3)$

Formula (Y3): $-C(R_{36})(R_{37})(OR_{38})$

Formula (Y4): $-C(Rn)(H)(Ar)$

In Formula (Y1) and Formula (Y2), $Rx_1$ to $Rx_3$ each independently represent a (linear or branched) alkyl group (preferably having 1 to 6 carbon atoms), a (monocyclic or polycyclic) cycloalkyl group (preferably having 3 to 15 carbon atoms), a (linear or branched) alkenyl group (preferably having 2 to 6 carbon atoms), a (monocyclic or polycyclic) aryl group (preferably having 6 to 15 carbon atoms), and a (monocyclic or polycyclic) heteroaryl group (preferably having 5 to 15 ring member atoms).

Two of $Rx_1$ to $Rx_3$ may be bonded to each other to form a ring. The above-described ring may be a monocycle or a polycycle. Examples of the monocycle and the polycycle include a cycloalkane ring. As the above-described cycloalkane ring, monocyclic cycloalkane rings such as a cyclopentane ring and a cyclohexane ring, and polycyclic cycloalkane rings such as a norbornane ring, a tetracyclodecane ring, a tetracyclododecane ring, and an adamantane ring. In the above-described cycloalkane ring, for example, one or more (for example, one to three) of methylene groups constituting the ring may be replaced with a heteroatom (-O-, S-, or the like), $-SO_2-$, $-SO_3-$, -COO-, a carbonyl group, or a vinylidene group. In addition, in these cycloalkane ring, one or more (for example, one or two) of ethylene groups constituting the cycloalkane ring may be replaced with a vinylene group.

In Formula (Y1), in a case where two of $Rx_1$ to $Rx_3$ are bonded to each other to form a cycloalkane ring, and the cycloalkane ring forms a vinylene group with an $\alpha$-carbon and a $\gamma$-carbon relative to the carbon (C) atom in Formula (Y1), the remaining one of $Rx_1$ to $Rx_3$ may be a hydrogen atom.

In Formula (Y3), $R_{36}$ to $R_{38}$ each independently represent a hydrogen atom or an organic group. As the above-described organic group, a (linear or branched) alkyl group (preferably having 1 to 6 carbon atoms), a (monocyclic or polycyclic) cycloalkyl group (preferably having 3 to 15 carbon atoms), a (monocyclic or polycyclic) aryl group (preferably having 6 to 15 carbon atoms), an aralkyl group (preferably having 7 to 18 carbon atoms), or a (linear or branched) alkenyl group (preferably having 2 to 6 carbon atoms) is preferable.

$R_{37}$ and $R_{38}$ may be bonded to each other to form a ring. Examples of the above-described ring to be formed include the same rings as the monocycle and the polycycle, which can be formed by bonding two of $Rx_1$ to $Rx_3$.

In Formula (Y4), Ar represents an aromatic ring group. Rn represents a (linear or branched) alkyl group (preferably having 1 to 6 carbon atoms), a (monocyclic or polycyclic) cycloalkyl group (preferably having 3 to 15 carbon atoms), or a (monocyclic or polycyclic) aryl group (preferably having 6 to 15 carbon atoms). Rn and Ar may be bonded to each other to form a non-aromatic ring. As Rn, a (monocyclic or polycyclic) aryl group (preferably having 6 to 15 carbon atoms) is preferable.

The repeating unit b preferably includes at least one selected from the group consisting of repeating units represented by Formulae (M1) to (M5), and from the viewpoint that the LWR is more excellent, it is more preferable to include at least one selected from the group consisting of a repeating unit represented by Formula (M4) and a repeating unit represented by Formula (M5).

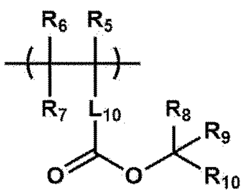
(M1)

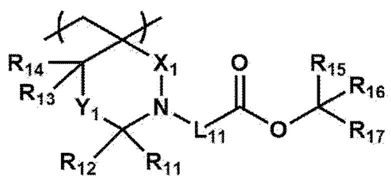
(M2)

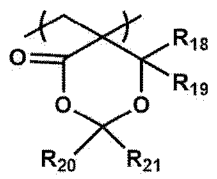
(M3)

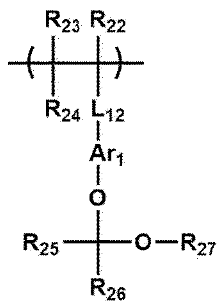
(M4)

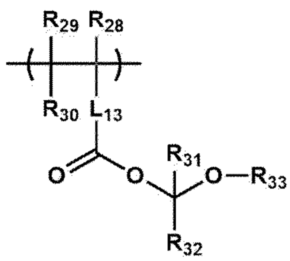
(M5)

In Formula (M1), $R_5$ to $R_7$ each independently represent a hydrogen atom, an alkyl group, a cycloalkyl group, a halogen atom, a cyano group, or an alkoxycarbonyl group, $L_{10}$ represents a single bond or a divalent linking group, and $R_8$ to $R_{10}$ each independently represent an alkyl group, a cycloalkyl group, an aryl group, an aralkyl group, or an alkenyl group.

$R_5$ to $R_7$ each independently represent a hydrogen atom, an alkyl group, a cycloalkyl group, a halogen atom, a cyano group, or an alkoxycarbonyl group.

The above-described alkyl group may be linear or branched. The number of carbon atoms in the above-described alkyl group is preferably 1 to 6.

The above-described cycloalkyl group may be monocyclic or polycyclic. The number of carbon atoms in the above-described cycloalkyl group is preferably 3 to 15.

Examples of the halogen atom include a fluorine atom, a chlorine atom, a bromine atom, and an iodine atom.

The number of carbon atoms in the above-described alkoxycarbonyl group is preferably 1 to 10. Examples of an alkyl group moiety in the above-described alkoxycarbonyl group include the same group as those in the above-described alkyl group.

As $R_5$, a hydrogen atom or an alkyl group is preferable, an alkyl group is more preferable, and a methyl group is still more preferable.

As $R_6$ and $R_7$, a hydrogen atom or an alkyl group is preferable, and a hydrogen atom is more preferable.

$L_{10}$ represents a single bond or a divalent linking group.

Examples of the above-described divalent linking group include -CO-, -O-, -S, -SO-, $-SO_2-$, $-NR^N-$, a hydrocarbon group (for example, an alkylene group, a cycloalkylene group, an alkenylene group, an arylene group, and the like), and a group formed by a combination of these groups. $R^N$ represents a substituent (for example, the substituent which can be included in $R^1$ to $R^4$ described above). The above-described hydrocarbon group may further have a substituent, and preferably has a halogen atom as the substituent.

As $L_{10}$, a single bond or an alkylene group is preferable.

$R_8$ to $R_{10}$ each independently represent an alkyl group, a cycloalkyl group, an aryl group, an aralkyl group, or an alkenyl group.

The above-described alkyl group may be linear or branched. The number of carbon atoms in the above-described alkyl group is preferably 1 to 6 and more preferably 1 to 3.

The above-described cycloalkyl group may be monocyclic or polycyclic. The number of carbon atoms in the above-described cycloalkyl group is preferably 3 to 15.

The above-described aryl group may be monocyclic or polycyclic. The number of carbon atoms in the above-described aryl group is preferably 6 to 15.

The number of carbon atoms in the above-described aralkyl group is preferably 7 to 18.

The above-described alkenyl group may be linear or branched. The number of carbon atoms in the above-described alkenyl group is preferably 2 to 6.

The alkyl group, cycloalkyl group, aryl group, aralkyl group, and alkenyl group described above may further have a substituent, and preferably have a halogen atom (preferably a fluorine atom) as the substituent.

As $R_8$ to $R_{10}$, an alkyl group or an aryl group is preferable, and an alkyl group is preferable.

At least two of $R_8$ to $R_{10}$ may be bonded to each other to form a ring.

In Formula (M2), $R_{11}$ to $R_{14}$ each independently represent a hydrogen atom or an organic group, where at least one of $R_{11}$ or $R_{12}$ represents an organic group, $X_1$ represents -CO-, -SO-, or $SO_2$-, $Y_1$ represents -O-, -S-, -SO-, $-SO_2-$, or $-NR_{34}-$, $R_{34}$ represents a hydrogen atom or an organic group, $L_{11}$ represents a single bond or a divalent linking group, and $R_{15}$ to $R_{17}$ each independently represent an alkyl group, a cycloalkyl group, an aryl group, an aralkyl group, or an alkenyl group.

$R_{11}$ to $R_{14}$ each independently represent a hydrogen atom or an organic group. However, at least one of $R_{11}$ or $R_{12}$ represents an organic group.

Examples of the above-described organic group include the organic group represented by $R^1$ and $R^4$ in Formula (1).

Among these, as the organic group, a (linear or branched) alkyl group (preferably having 1 to 6 carbon atoms), a (monocyclic or polycyclic) cycloalkyl group (preferably having 3 to 15 carbon atoms), a (monocyclic or polycyclic) aryl group (preferably having 6 to 15 carbon atoms), an aralkyl group (preferably having 7 to 18 carbon atoms), or a (linear or branched) alkenyl group (preferably having 2 to 6 carbon atoms) is preferable.

As $R_{11}$ and $R_{12}$, an alkyl group is preferable, and an alkyl group having a fluorine atom is more preferable.

As $R_{13}$ and $R_{14}$, a hydrogen atom or an alkyl group is preferable, and a hydrogen atom is more preferable.

$X_1$ represents -CO-, -SO-, or $-SO_2-$. As $X_1$, -CO- is preferable.

$Y_1$ represents -O-, -S-, -SO-, $-SO_2-$, or $-NR_{34}-$. As $Y_1$, -O- or -S- is preferable, and -O- is more preferable.

$R_{34}$ represents a hydrogen atom or an organic group. As $R_{34}$, an organic group is preferable.

$L_{11}$ represents a single bond or a divalent linking group.

Examples of the above-described divalent linking group include -CO-, -O-, -S, -SO-, $-SO_2-$, $-NR^N-$, a hydrocarbon group (for example, an alkylene group, a cycloalkylene group, an alkenylene group, an arylene group, and the like), and a group formed by a combination of these groups. $R^N$ represents a substituent (for example, the substituent which can be included in $R^1$ to $R^4$ described above). The above-described hydrocarbon group may further have a substituent, and preferably has a halogen atom as the substituent.

As $L_{11}$, a hydrocarbon group is preferable, and an alkylene group is more preferable.

$R_{15}$ to $R_{17}$ each independently represent an alkyl group, a cycloalkyl group, an aryl group, an aralkyl group, or an alkenyl group.

The above-described alkyl group may be linear or branched. The number of carbon atoms in the above-described alkyl group is preferably 1 to 6.

The above-described cycloalkyl group may be monocyclic or polycyclic. The number of carbon atoms in the above-described cycloalkyl group is preferably 3 to 15.

The above-described aryl group may be monocyclic or polycyclic. The number of carbon atoms in the above-described aryl group is preferably 6 to 15.

The number of carbon atoms in the above-described aralkyl group is preferably 7 to 18.

The above-described alkenyl group may be linear or branched. The number of carbon atoms in the above-described alkenyl group is preferably 2 to 6.

The alkyl group, cycloalkyl group, aryl group, aralkyl group, and alkenyl group described above may further have a substituent, and preferably have a halogen atom (preferably a fluorine atom) as the substituent.

As $R_{15}$ to $R_{17}$, an alkyl group or an aryl group is preferable.

At least two of $R_{15}$ to $R_{17}$ may be bonded to each other to form a ring.

In Formula (M3), $R_{18}$ and $R_{19}$ each independently represent a hydrogen atom or an organic group, and $R_{20}$ and $R_{21}$ each independently represent a hydrogen atom, an alkyl group, a cycloalkyl group, an aryl group, an aralkyl group, or an alkenyl group.

Examples of the above-described organic group include the organic group (for example, an alkyl group and the like) exemplified by the substituent represented by $R^1$ and $R^4$ in Formula (1).

As $R_{18}$ and $R_{19}$, a hydrogen atom or an alkyl group is preferable, and a hydrogen atom is more preferable.

Examples of $R_{20}$ and $R_{21}$ include $R_{15}$ to $R_{17}$ in Formula (M2), and a hydrogen atom or an alkyl group is preferable.

At least two of $R_{18}$ to $R_{21}$ may be bonded to each other to form a ring. Among these, it is preferable that $R_{18}$ and $R_{19}$, and $R_{20}$ and $R_{21}$ are bonded to each other to form a ring.

In Formula (M4), $R_{22}$ to $R_{24}$ each independently represent a hydrogen atom, an alkyl group, a cycloalkyl group, a halogen atom, a cyano group, or an alkoxycarbonyl group, $L_{12}$ represents a single bond or a divalent linking group, An represents an aromatic ring group, and $R_{25}$ to $R_{27}$ each independently represent a hydrogen atom, an alkyl group, a cycloalkyl group, an aryl group, an aralkyl group, or an alkenyl group.

$R_{22}$ to $R_{24}$ each independently represent a hydrogen atom, an alkyl group, a cycloalkyl group, a halogen atom, a cyano group, or an alkoxycarbonyl group.

The above-described alkyl group may be linear or branched. The number of carbon atoms in the above-described alkyl group is preferably 1 to 6 and more preferably 1 to 3.

The above-described cycloalkyl group may be monocyclic or polycyclic. The number of carbon atoms in the above-described cycloalkyl group is preferably 3 to 15.

Examples of the halogen atom include a fluorine atom, a chlorine atom, a bromine atom, and an iodine atom.

The number of carbon atoms in the above-described alkoxycarbonyl group is preferably 1 to 10. Examples of an alkyl group moiety in the above-described alkoxycarbonyl group include the same group as those in the above-described alkyl group.

As $R_{22}$ to $R_{24}$, a hydrogen atom or an alkyl group is preferable, and a hydrogen atom is more preferable.

$L_{12}$ represents a single bond or a divalent linking group.

Examples of $L_{12}$ include $L_{11}$ in Formula (M2), and a single bond is preferable.

An represents an aromatic ring group.

The above-described aromatic ring group may be monocyclic or polycyclic.

The number of ring member atoms in the above-described aromatic ring group is preferably 5 to 15. The above-described aromatic ring group may have one or more (for example, one to five) heteroatoms (oxygen atom, sulfur atom, nitrogen atom, and/or the like) as the ring member atom. As the above-described aromatic ring group, a benzene ring group is preferable.

$R_{25}$ to $R_{27}$ each independently represent a hydrogen atom, an alkyl group, a cycloalkyl group, an aryl group, an aralkyl group, or an alkenyl group.

Examples of $R_{25}$ to $R_{27}$ include $R_{15}$ to $R_{17}$ in Formula (M2).

As $R_{25}$ to $R_{27}$, a hydrogen atom or an alkyl group is preferable.

At least two of $R_{25}$ to $R_{27}$ may be bonded to each other to form a ring. Among these, it is preferable that $R_{26}$ and $R_{27}$ are bonded to each other to form a ring. In addition, An may be bonded to $R_{24}$ or $R_{25}$ to form a ring.

In Formula (M5), $R_{28}$ to $R_{30}$ each independently represent a hydrogen atom, an alkyl group, a cycloalkyl group, a halogen atom, a cyano group, or an alkoxycarbonyl group, $L_{13}$ represents a single bond or a divalent linking group, $R_{31}$ and $R_{32}$ each independently represent a hydrogen atom, an alkyl group, a cycloalkyl group, an aryl group, an aralkyl group, or an alkenyl group, and $R_{33}$ represents an alkyl group, a cycloalkyl group, an aryl group, an aralkyl group, or an alkenyl group.

$R_{28}$ to $R_{30}$ have the same meanings as $R_{22}$ to $R_{24}$ in Formula (M4), and suitable aspects thereof are also the same.

$L_{13}$ has the same meaning as $L_{12}$ in Formula (M4), and a suitable aspect thereof is also the same.

$R_{33}$ has the same meaning as $R_{15}$ to $R_{17}$ in Formula (M2), and a suitable aspect thereof is also the same.

At least two of $R_{31}$ to $R_{33}$ may be bonded to each other to form a ring. Among these, it is preferable that $R_{32}$ and $R_{33}$ are bonded to each other to form a ring.

With respect to all repeating units in the acid-decomposable resin, a content of the repeating unit b is preferably 1% by mole or more, more preferably 10% by mole or more, and from the viewpoint that the resolution and the LWR are more excellent, it is still more preferably 15% by mole or more. The upper limit thereof is preferably 80% by mole or less, more preferably 70% by mole or less, and still more preferably 60% by mole or less with respect to all repeating units.

The repeating unit b may be used alone or in combination of two or more kinds thereof. In a case where two or more kinds thereof are used, a total content thereof is preferably within the suitable content range.

<Repeating Unit having Lactone Group>

The acid-decomposable resin may have a repeating unit having a lactone group.

The repeating unit having a lactone group may or may not correspond to the above-described repeating units.

For example, as long as it has a lactone group, the repeating unit having a lactone group may or may not correspond to the repeating unit a, and may or may not correspond to the repeating unit b having an acid-decomposable group.

The lactone group may have a lactone structure or a sultone structure. The lactone structure is preferably a 5- to 7-membered ring lactone structure. Among these, those in which another ring structure is fused to the 5- to 7-membered ring lactone structure to form a bicyclo structure or a spiro structure are more preferable.

It is preferable that the acid-decomposable resin has a repeating unit having a lactone group, which is obtained by removing one or more (for example, one or two) hydrogen atoms from a lactone structure represented by any of Formulae (LC1-1) to (LC1-21).

In addition, the lactone group may be bonded directly to the main chain. For example, a ring member atom of the lactone group may constitute a main chain of the acid-decomposable resin.

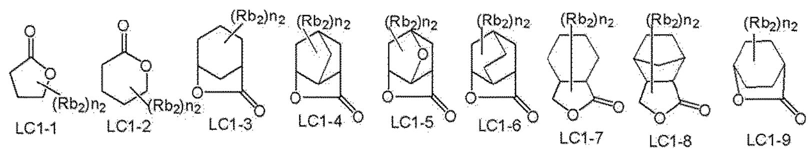

LC1-1 LC1-2 LC1-3 LC1-4 LC1-5 LC1-6 LC1-7 LC1-8 LC1-9

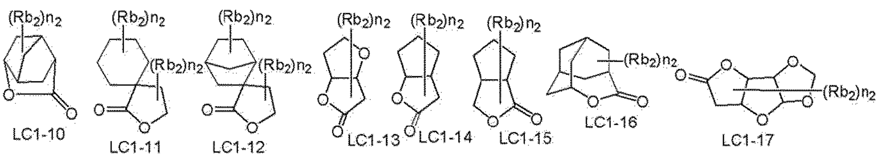

LC1-10 LC1-11 LC1-12 LC1-13 LC1-14 LC1-15 LC1-16 LC1-17

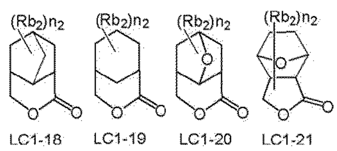

LC1-18 LC1-19 LC1-20 LC1-21

The above-described lactone structures may have a substituent ($Rb_2$). Examples of the substituent ($Rb_2$) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxy group, a halogen atom, a hydroxyl group, a cyano group, a group including an acid-decomposable group (acid-decomposable group itself may be used), and a group formed by a combination of these groups. n2 represents an integer of 0 to 4. In a case where $n_2$ is 2 or more, a plurality of $Rb_2$'s may be different from each other, and the plurality of $Rb_2$'s may be bonded to each other to form a ring.

One or more (for example, one or two) methylene groups not adjacent to -COO- or O-in the ring member atoms of the above-described lactone structure may be replaced with a heteroatom such as -O- and S-.

Examples of the repeating unit having a lactone group include a repeating unit represented by Formula (AI).

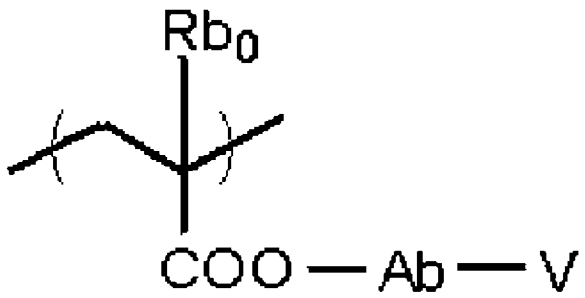

(AI)

In Formula (AI), $Rb_0$ represents a hydrogen atom, a halogen atom, or an alkyl group having 1 to 4 carbon atoms.
As the substituent which may be included in the above-described alkyl group, a hydroxyl group or a halogen atom is preferable.
Examples of the above-described halogen atom include a fluorine atom, a chlorine atom, a bromine atom, and an iodine atom. $Rb_0$ is preferably a hydrogen atom or a methyl group.
Ab represents a single bond, an alkylene group, a divalent linking group having a monocyclic or polycyclic alicyclic hydrocarbon structure, an ether group, -COO-, a carbonyl group, a carboxy group, or a group formed by a combination thereof. Among these, a single bond or a linking group represented by $Ab_1$-$CO_2$- is preferable. $Ab_1$ is a linear or branched alkylene group, or a monocyclic or polycyclic cycloalkylene group. Among these, a methylene group, an ethylene group, a cyclohexylene group, an adamantylene group, or a norbomylene group is preferable.
V represents a group formed by removing one hydrogen atom from ring member atoms of the lactone structure represented by any of Formulae (LC1-1) to (LC1-21).
The repeating unit having a lactone group may be, for example, a repeating unit represented by Formula (AII) or (AIII).

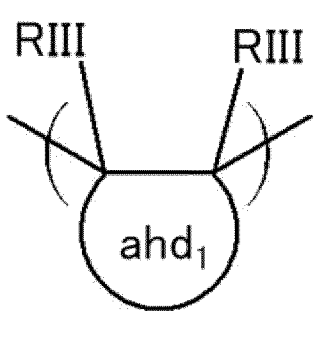

(AII)

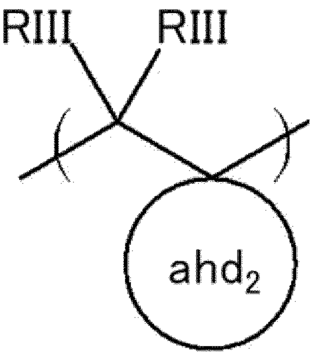

(AIII)

In Formulae (AII) and (AIII), RIII's each independently represent a hydrogen atom or a substituent.
RIII is preferably a hydrogen atom.
In Formula (AII), $ahd_1$ represents a group formed by removing one hydrogen atom from each of adjacent ring member atoms of the lactone structure represented by any of Formulae (LC1-1) to (LC1-21).
In Formula (AIII), $ahd_2$ represents a group formed by removing two hydrogen atoms from ring member atoms of the lactone structure represented by any of Formulae (LC1-1) to (LC1-21).
The repeating unit having a lactone group is exemplified below.

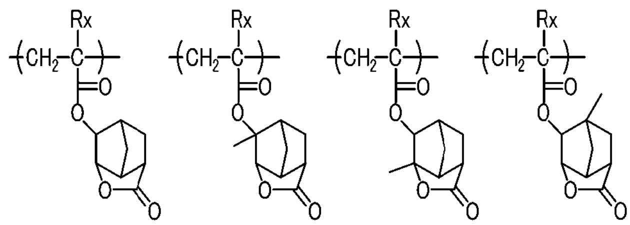

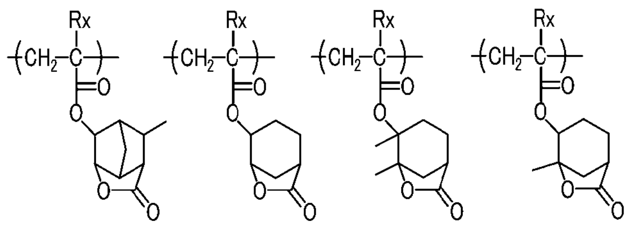

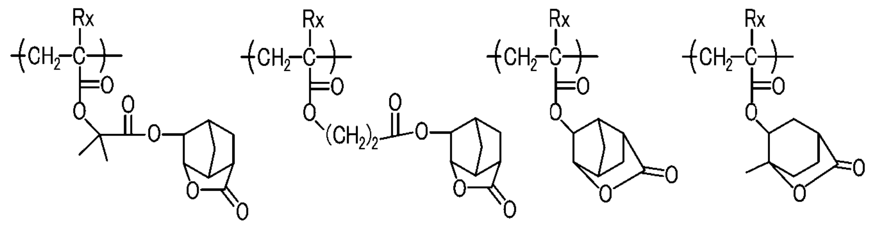

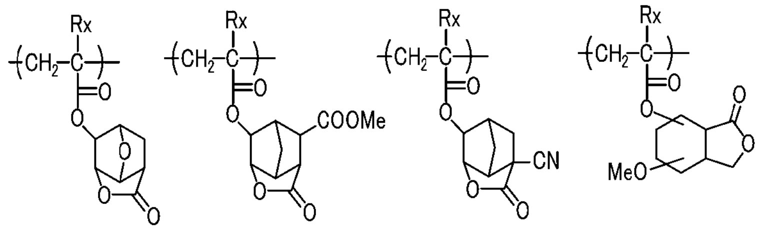

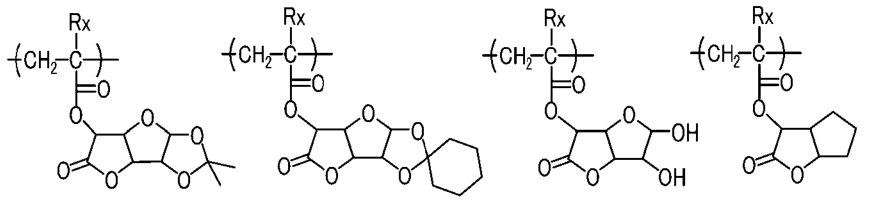

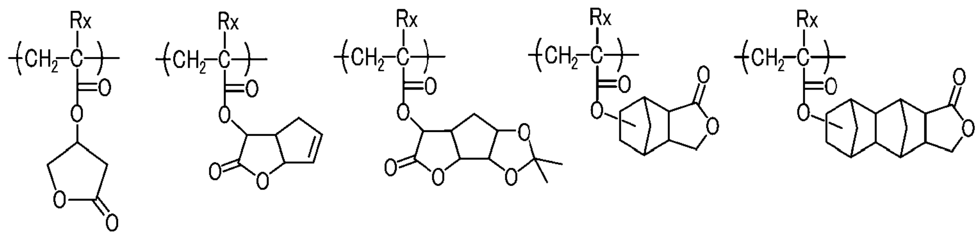

(in the formulae, Rx is H, $CH_3$, $CH_2OH$, or $CF_3$)

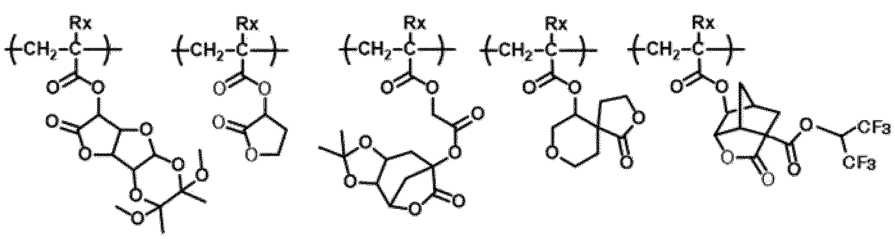

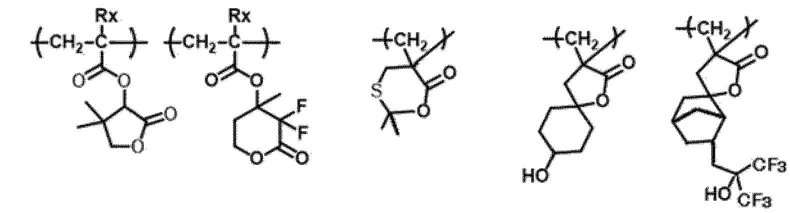

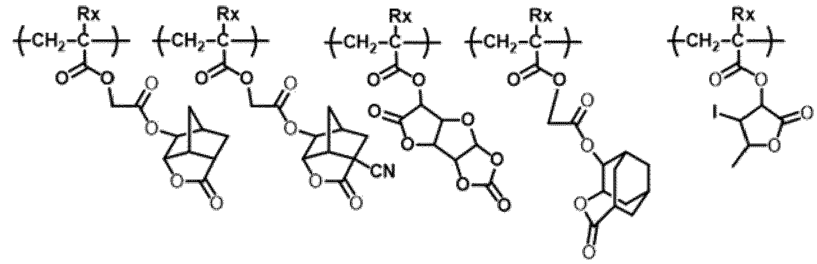

In a case where an optical isomer is present in the repeating unit having a lactone group, any of optical isomers may be used. In addition, one optical isomer may be used alone or a mixture of a plurality of the optical isomers may be used. In a case where one kind of optical isomers is mainly used, an optical purity (ee) thereof is preferably 90 or more, and more preferably 95 or more.

A content of the repeating unit having a lactone group is preferably 5% to 100% by mole, more preferably 10% to 80% by mole, and still more preferably 15% to 65% by mole with respect to all repeating units of the acid-decomposable resin.

Among the repeating units having a lactone group, a total of the repeating unit having a lactone group, which corresponds to the repeating unit a, and the repeating unit having a lactone group, which does not correspond to the repeating unit a, may satisfy the above-described suitable content; the repeating unit having a lactone group, which corresponds to the repeating unit a, alone may satisfy the above-described suitable content; or the repeating unit having a lactone group, which does not correspond to the repeating unit a, alone may satisfy the above-described suitable content.

The repeating unit having a lactone group may be used alone or in combination of two or more kinds thereof. In a case where two or more kinds thereof are used, a total content thereof is preferably within the suitable content range.

<Repeating Unit having Sultone Group or Carbonate Group>

The acid-decomposable resin may have a repeating unit having a sultone group.

It is sufficient that the sultone group has a sultone structure. The sultone structure is preferably a 5- to 7-membered ring sultone structure. Among these, those in which another ring structure is fused to the 5- to 7-membered ring sultone structure to form a bicyclo structure or a spiro structure are more preferable.

In addition, the sultone group may be bonded directly to the main chain. For example, a ring member atom of the sultone group may constitute the main chain of the acid-decomposable resin.

It is preferable that the acid-decomposable resin has a repeating unit having a sultone group, which is formed by removing one or more (for example, one or two) hydrogen atoms from ring member atoms of a sultone structure represented by any of Formulae (SL1-1) to (SL1-3).

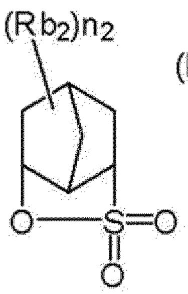

SL1-1

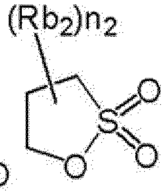

SL1-2

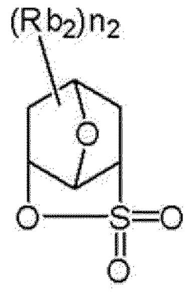

SL1-3

The above-described sultone structures may have a substituent ($Rb_2$). The substituent ($Rb_2$) in Formulae

(SL1-1) to (SL1-3) can be described in the same manner as the substituent ($Rb_2$) in the lactone structure represented by Formulae (LC1-1) to (LC1-21) described above.

One or more (for example, one or two) methylene groups not adjacent to -COO- or O-in the ring member atoms of the above-described sultone structure may be replaced with a heteroatom such as -O- and S-.

Examples of the repeating unit having a sultone group include a repeating unit in which V in the repeating unit represented by Formula (AI) described above is replaced with a group formed by removing one hydrogen atom from ring member atoms of the sultone structure represented by any of Formulae (SL1-1) to (SL1-3); a repeating unit in which $ahd_1$ in the repeating unit represented by Formula (AII) described above is replaced with a group formed by removing one hydrogen atom from each of adjacent ring member atoms of the sultone structure represented by any of Formulae (SL1-1) to (SL1-3); and a repeating unit in which $ahd_2$ in the repeating unit represented by General Formula (AIII) described above is replaced with a group formed by removing two hydrogen atoms from ring member atoms of the sultone structure represented by any of Formulae (SL1-1) to (SL1-3).

As the carbonate group, a cyclic carbonate ester group is preferable.

As the repeating unit having a cyclic carbonate ester group, a repeating unit represented by Formula (A-1) is preferable.

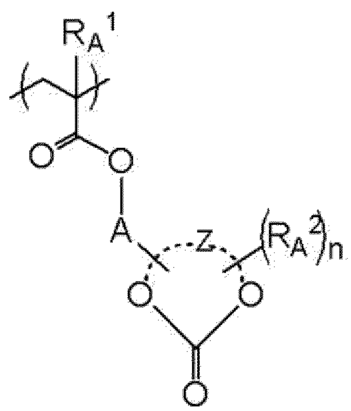

(A-1)

In Formula (A-1), $R_A^1$ represents a hydrogen atom, a halogen atom, or a monovalent organic group (preferably a methyl group).

n represents an integer of 0 or more.

$R_A^2$ represents a substituent. In a case where n is 2 or more, a plurality of $R_A^2$'s may be the same or different from each other.

A represents a single bond or a divalent linking group. As the above-described divalent linking group, an alkylene group, a divalent linking group having a monocyclic or polycyclic alicyclic hydrocarbon structure, -O-, -COO-, a carbonyl group, a carboxy group, or a divalent group formed by a combination thereof is preferable.

Z represents an atomic group which forms a monocycle or polycycle with a group represented by -O-CO-O- in the formula.

The repeating unit having a sultone group or a carbonate group is exemplified below.

(in the formulae, Rx is H, $CH_3$, $CH_2OH$, or $CF_3$)

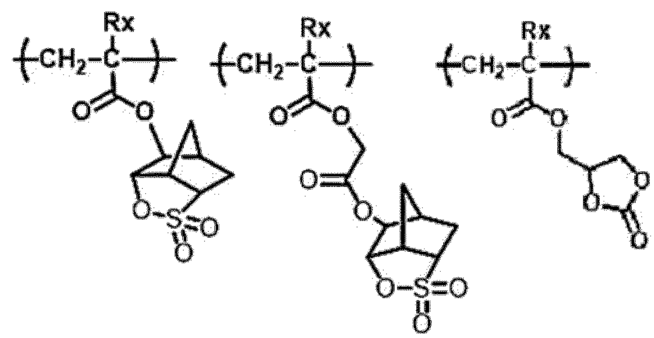
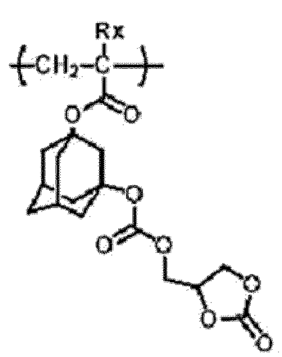

A content of the repeating unit having a sultone group or a carbonate group is preferably 1% by mole or more, and more preferably 10% by mole or more with respect to all the repeating units in the acid-decomposable resin. The

upper limit thereof is preferably 85% by mole or less, more preferably 80% by mole or less, still more preferably 70% by mole or less, and particularly preferably 60% by mole or less with respect to all repeating units in the acid-decomposable resin.

<Repeating Unit having Acid Group>

The acid-decomposable resin may have a repeating unit having an acid group.

It is preferable that the repeating unit having an acid group is different from the above-described repeating units.

As the acid group, an acid group having a pKa of 13 or less is preferable. An acid dissociation constant of the above-described acid group is preferably 13 or less, more preferably 3 to 13, and still more preferably 5 to 10.

In a case where the acid-decomposable resin has an acid group having a pKa of 13 or less, a content of the acid group in the acid-decomposable resin is 0.2 to 6.0 mmol/g in many cases, preferably 0.8 to 6.0 mmol/g, more preferably 1.2 to 5.0 mmol/g, and still more preferably 1.6 to 4.0 mmol/g. In a case where the content of the acid group is within the above-described range, the development proceeds satisfactorily, the formed pattern shape is excellent, and the resolution is also excellent.

As the acid group, for example, a carboxy group, a hydroxyl group, a phenolic hydroxyl group, a fluorinated alcohol group (preferably, a hexafluoroisopropanol group), a sulfonic acid group, a sulfonamide group, or an isopropanol group is preferable.

In addition, in the above-described hexafluoroisopropanol group, one or more (preferably one or two) fluorine atoms may be substituted with a group (an alkoxycarbonyl group and the like) other than a fluorine atom. $-C(CF_3)(OH)-CF_2-$ formed as above is also preferable as the acid group. In addition, one or more fluorine atoms may be substituted with a group other than a fluorine atom to form a ring including $-C(CF_3)(OH)-CF_2-$.

The repeating unit having an acid group may have a fluorine atom or an iodine atom.

As the repeating unit having an acid group, a repeating unit represented by Formula (B) is preferable.

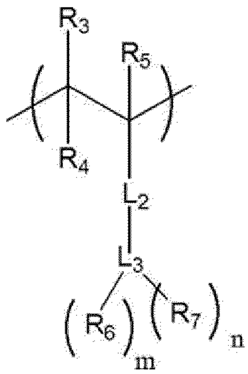

(B)

$R^3$ represents a hydrogen atom or a monovalent organic group which may have a fluorine atom or an iodine atom.

As the monovalent organic group which may have a fluorine atom or an iodine atom, a group represented by $-L_4-R_8$ is preferable. $L_4$ represents a single bond or -COO-. $R_8$ represents an alkyl group which may have a fluorine atom or an iodine atom, a cycloalkyl group which may have a fluorine atom or an iodine atom, an aryl group which may have a fluorine atom or an iodine atom, or a group formed by a combination thereof.

$R_4$ and $R_5$ each independently represent a hydrogen atom, a fluorine atom, an iodine atom, or an alkyl group which may have a fluorine atom or an iodine atom.

$L_2$ represents a single bond, -COO-, or a divalent group formed by combining -CO-, -O-, and an alkylene group (preferably having 1 to 6 carbon atoms; which may be linear or branched; in addition, $-CH_2-$ may be substituted with a halogen atom).

$L^3$ represents an (n+m+1)-valent aromatic hydrocarbon ring group or an (n+m+1)-valent alicyclic hydrocarbon ring group. Examples of the aromatic hydrocarbon ring group include a benzene ring group and a naphthalene ring group. The alicyclic hydrocarbon ring group may be monocyclic or polycyclic, and examples thereof include a cycloalkyl ring group, a norbornene ring group, and an adamantane ring group.

$R^6$ represents a hydroxyl group or a fluorinated alcohol group. The fluorinated alcohol group is preferably a monovalent group represented by Formula (3L).

$$*-L_{6X}-R_{6X} \qquad (3L)$$

$L_{6X}$ represents a single bond or a divalent linking group. Examples of the divalent linking group include -CO-,

-O-, -SO-, $-SO_2-$, -NRA-, an alkylene group (preferably having 1 to 6 carbon atoms; may be linear or branched), and a divalent linking group formed by a combination of a plurality of these groups. Examples of $R^A$ include a hydrogen atom and an alkyl group having 1 to 6 carbon atoms. In addition, the above-described alkylene group may have a substituent. Examples of the substituent include a halogen atom (preferably, a fluorine atom) and a hydroxyl group. $R_{6X}$ represents a hexafluoroisopropanol group. In a case where $R_6$ is a hydroxyl group, $L_3$ is also preferably the (n+m+1)-valent aromatic hydrocarbon ring group.

$R^7$ represents a halogen atom. Examples of the halogen atom include a fluorine atom, a chlorine atom, a bromine atom, and an iodine atom.

m represents an integer of 1 or more. m is preferably an integer of 1 to 3 and preferably an integer of 1 or 2.
n represents 0 or an integer of 1 or more. n is preferably an integer of 1 to 4.
(n+m+1) is preferably an integer of 1 to 5.

As the repeating unit having an acid group, a repeating unit represented by Formula (I) is also preferable.

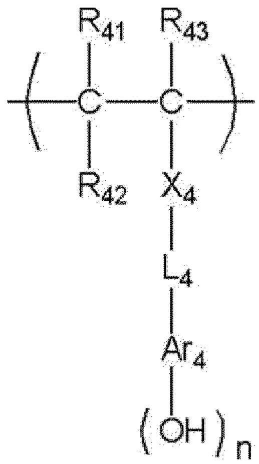

(I)

In Formula (I), $R_{41}$, $R_{42}$, and $R_{43}$ each independently represent a hydrogen atom, an alkyl group, a cycloalkyl group, a halogen atom, a cyano group, or an alkoxycarbonyl group, where $R_{42}$ may be bonded to $Ar_4$ to form a ring, and in this case, $R_{42}$ represents a single bond or an alkylene group,

$X_4$ represents a single bond, -COO-, or $CONR_{64}$-, and $R_{64}$ represents a hydrogen atom or an alkyl group,
$L_4$ represents a single bond or an alkylene group,
$Ar_4$ represents an (n+1)-valent aromatic ring group, and in a case where $Ar_4$ is bonded to $R_{42}$ to form a ring, $Ar_4$ represents an (n+2)-valent aromatic ring group, and
n represents an integer of 1 to 5.

As the alkyl group represented by each of $R_{41}$, $R_{42}$, and $R_{43}$ in Formula (I), an alkyl group having 20 or less carbon atoms, such as a methyl group, an ethyl group, a propyl group, an isopropyl group, an n-butyl group, a sec-butyl group, a hexyl group, a 2-ethylhexyl group, an octyl group, and a dodecyl group is preferable, an alkyl group having 8 or less carbon atoms is more preferable, and an alkyl group having 3 or less carbon atoms is still more preferable.

The cycloalkyl group of $R_{41}$, $R_{42}$, and $R_{43}$ in Formula (I) may be monocyclic or polycyclic. Among these, a monocyclic cycloalkyl group having 3 to 8 carbon atoms, such as a cyclopropyl group, a cyclopentyl group, and a cyclohexyl group, is preferable.

Examples of the halogen atom of $R_{41}$, $R_{42}$, and $R_{43}$ in Formula (I) include a fluorine atom, a chlorine atom, a bromine atom, and an iodine atom, and a fluorine atom is preferable.

As the alkyl group included in the alkoxycarbonyl group of $R_{41}$, $R_{42}$, and $R_{43}$ in Formula (I), the same group as the alkyl group in $R_{41}$, $R_{42}$, and $R_{43}$ described above is also preferable.

Preferred examples of the substituent in each of the groups include an alkyl group, a cycloalkyl group, an aryl group, an amino group, an amide group, a ureido group, a urethane group, a hydroxyl group, a carboxy group, a halogen atom, an alkoxy group, a thioether group, an acyl group, an acyloxy group, an alkoxycarbonyl group, a cyano group, and a nitro group. The number of carbon atoms in the substituent is preferably 8 or less.

$Ar_4$ represents an (n+1)-valent aromatic ring group. The divalent aromatic ring group in a case where n is 1 is preferably, for example, an arylene group having 6 to 18 carbon atoms, such as a phenylene group, a tolylene group,

a naphthylene group, and an anthracenylene group, or a divalent aromatic ring group including a heterocycle such as a thiophene ring, a furan ring, a pyrrole ring, a benzothiophene ring, a benzofuran ring, a benzopyrrole ring, a triazine ring, an imidazole ring, a benzimidazole ring, a triazole ring, a thiadiazole ring, and a thiazole ring. The above-described aromatic ring group may have a substituent.

Examples of the (n+1)-valent aromatic ring group in a case where n is an integer of 2 or more include groups formed by removing any (n-1) hydrogen atoms from the above-described specific examples of the divalent aromatic ring group.

The (n+1)-valent aromatic ring group may further have a substituent.

Examples of the substituent which may be included in the alkyl group, cycloalkyl group, alkoxycarbonyl group, alkylene group, and (n+1)-valent aromatic ring group described above include alkyl groups mentioned as $R_{41}$, $R_{42}$, and $R_{43}$ in General Formula (I); alkoxy groups such as a methoxy group, an ethoxy group, a hydroxyethoxy group, a propoxy group, a hydroxypropoxy group, and a butoxy group; and aryl groups such as a phenyl group.

Examples of the alkyl group of $R_{64}$ in $-CONR_{64}-$ represented by $X_4$ ($R_{64}$ represents a hydrogen atom or an alkyl group) include an alkyl group having 20 or less carbon atoms, such as a methyl group, an ethyl group, a propyl group, an isopropyl group, an n-butyl group, a sec-butyl group, a hexyl group, a 2-ethylhexyl group, an octyl group, and a dodecyl group, and an alkyl group having 8 or less carbon atoms, is preferable.

$X_4$ is preferably a single bond, -COO-, or -CONH-, and more preferably a single bond or -COO-.

As the alkylene group in $L_4$, an alkylene group having 1 to 8 carbon atoms, such as a methylene group, an ethylene group, a propylene group, a butylene group, a hexylene group, and an octylene group, is preferable.

As $Ar_4$, an aromatic ring group having 6 to 18 carbon atoms is preferable, and a benzene ring group, a naphthalene ring group, and a biphenylene ring group are more preferable.

The repeating unit represented by Formula (I) preferably includes a hydroxystyrene structure. That is, $Ar_4$ is preferably a benzene ring group.

As the repeating unit represented by Formula (I), a repeating unit represented by Formula (1) is preferable.

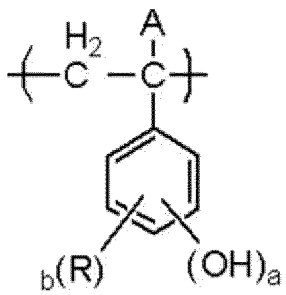

(1)

In Formula (1),

A represents a hydrogen atom, an alkyl group, a cycloalkyl group, a halogen atom, or a cyano group,
R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group, where in a case of a plurality of R's, the plurality of R's may be the same or different from each other, in a case where a plurality of R's are present, the plurality of R's may be combined with each other to form a ring, R is preferably a hydrogen atom.
a represents an integer of 1 to 3, and
b represents an integer of 0 to (5 - a).

The repeating unit having an acid group is exemplified below.

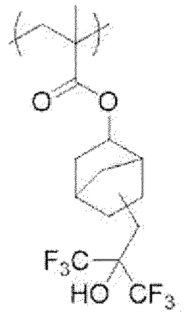
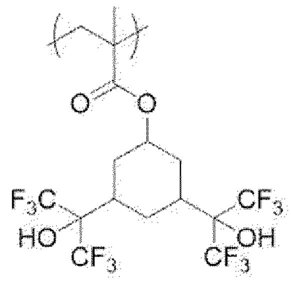
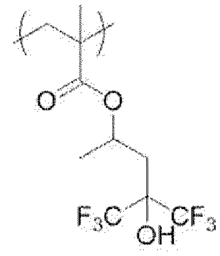
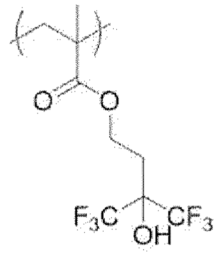

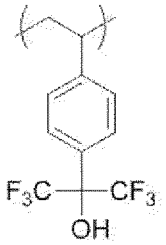
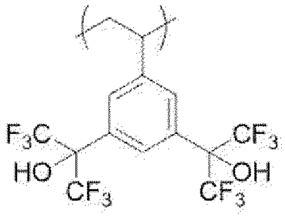
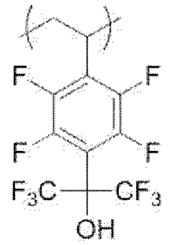
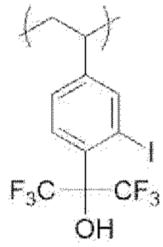

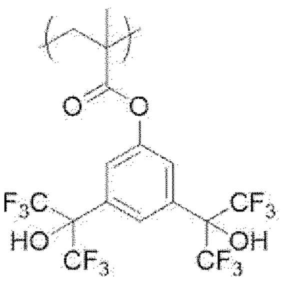
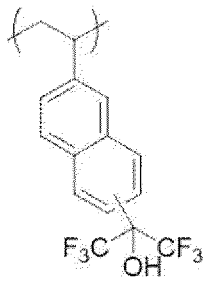

In the following examples, a represents 1 or 2 in the formulae.

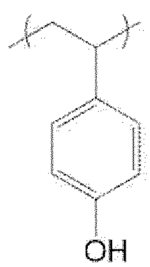
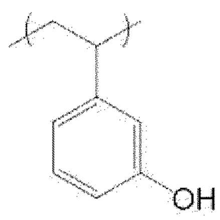
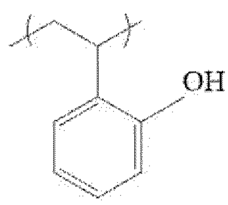

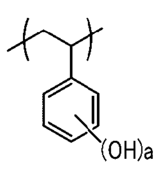
(B-1)

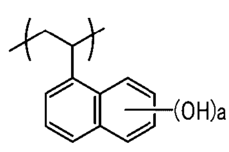
(B-2)

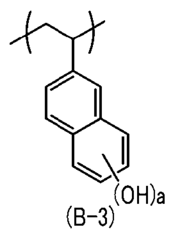
(B-3)

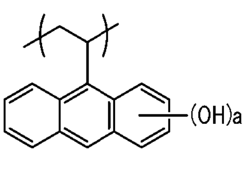

(B-4)

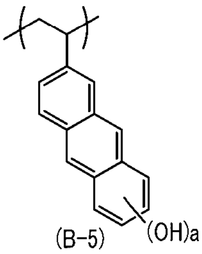

(B-5)

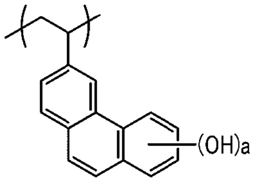

(B-6)

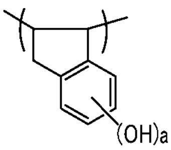

(B-7)

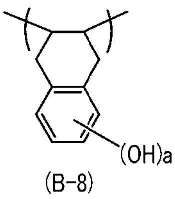

(B-8)

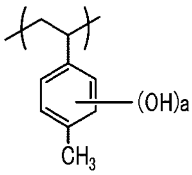

(B-9)

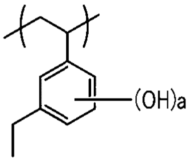

(B-10)

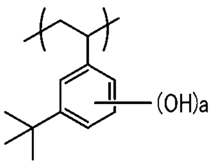

(B-11)

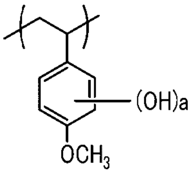

(B-12)

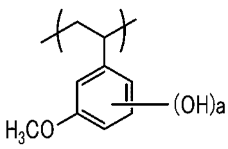

(B-13)

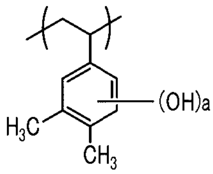

(B-14)

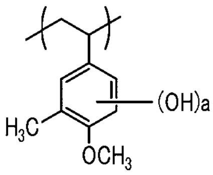

(B-15)

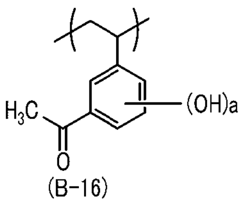

(B-16)

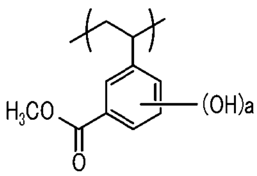

(B-17)

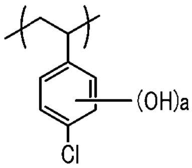

(B-18)

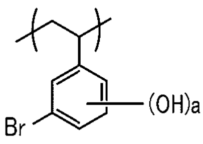

(B-19)

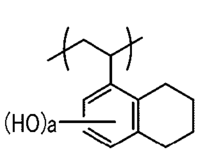

(B-20)

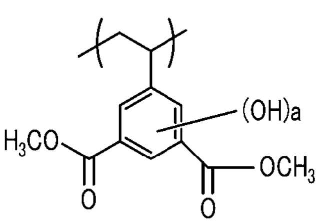

(B-21)

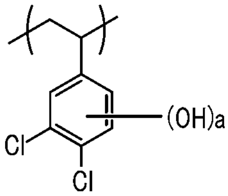

(B-22)

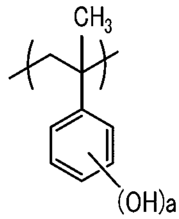

(B-23)

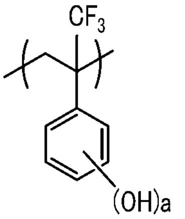

(B-24)

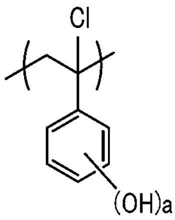

(B-25)

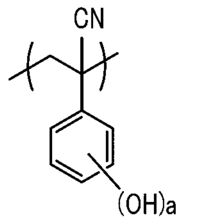

(B-26)

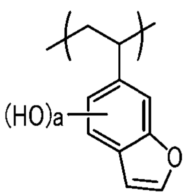

(B-27)

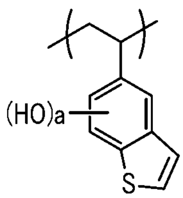

(B-28)

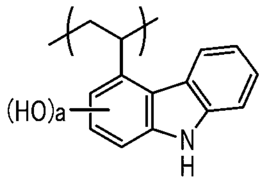

(B-29)

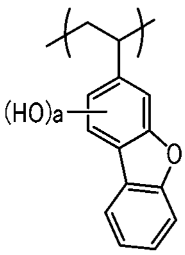

(B-30)

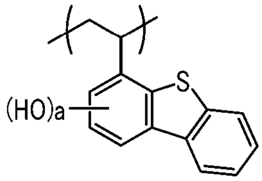

(B-31)

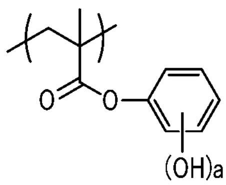

(B-32)

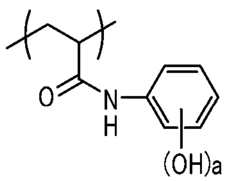

(B-33)

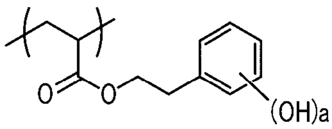

(B-34)

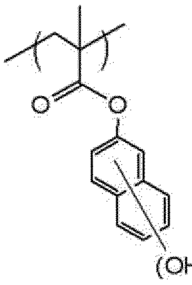

(B-35)

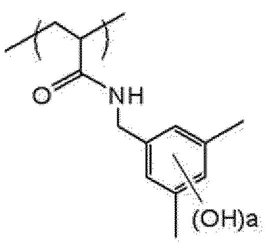

(B-36)

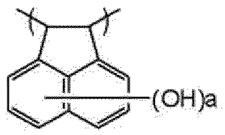

(B-37)

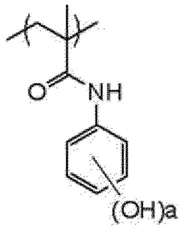

(B-38)

Among these, repeating units specifically described below are preferable. In the formulae, R represents a hydrogen atom or a methyl group, and a represents 2 or 3.

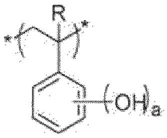

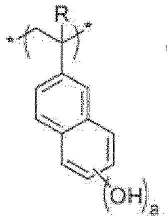

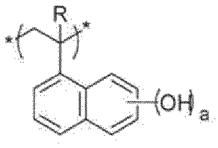

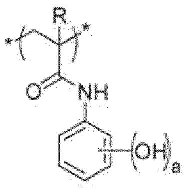

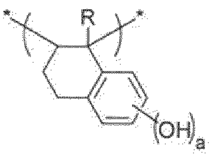

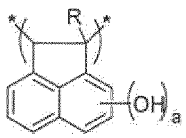

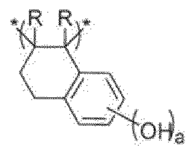

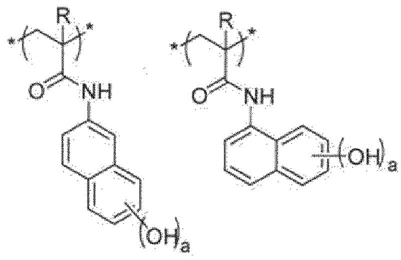

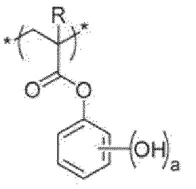

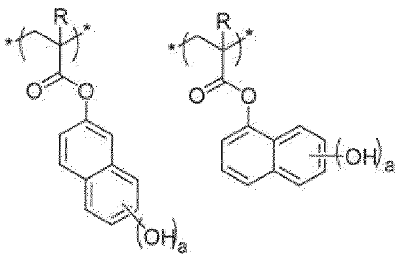

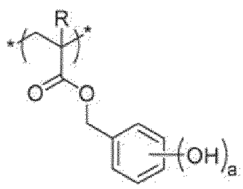

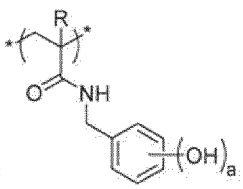

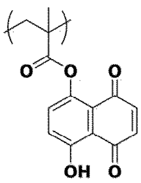

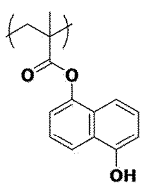

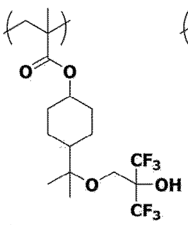

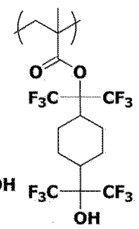

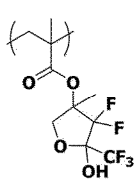

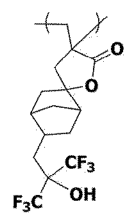

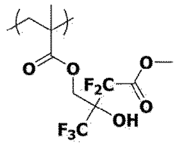

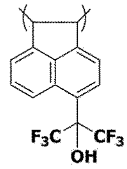

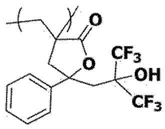

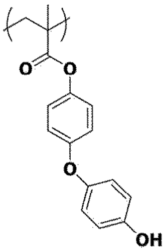

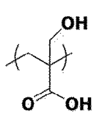

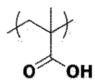

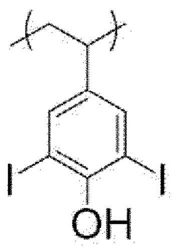
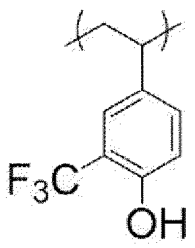
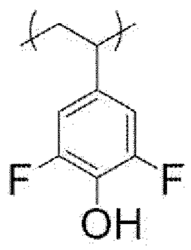
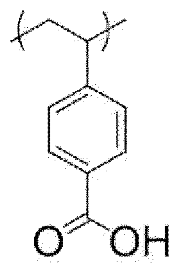
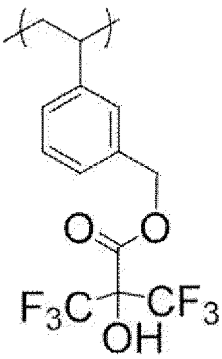
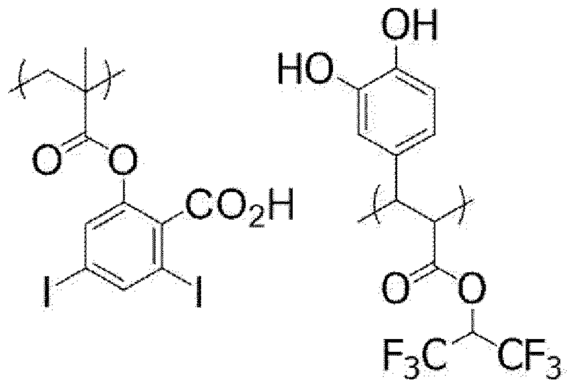

A content of the repeating unit having an acid group is preferably 5% by mole or more, and more preferably 10% by mole or more with respect to all repeating units in the acid-decomposable resin. The upper limit thereof is preferably 70% by mole or less, more preferably 65% by mole or less, and still more preferably 60% by mole or less with respect to all repeating units in the acid-decomposable resin.

<Repeating Unit having Fluorine Atom or Iodine Atom>

The acid-decomposable resin may have a repeating unit having a fluorine atom or an iodine atom.
It is preferable that the repeating unit having a fluorine atom or an iodine atom is different from the above-described repeating units.
As the repeating unit having a fluorine atom or an iodine atom, a repeating unit represented by Formula (C) is preferable.

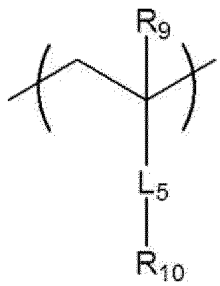
(C)

$L_5$ represents a single bond or -COO-.
$R_9$ represents a hydrogen atom or an alkyl group which may have a fluorine atom or an iodine atom.
$R_{10}$ represents a hydrogen atom, an alkyl group which may have a fluorine atom or an iodine atom, a cycloalkyl group which may have a fluorine atom or an iodine atom, an aryl group which may have a fluorine atom or an iodine atom, or a group formed by a combination thereof.
The repeating unit having a fluorine atom or an iodine atom is exemplified below.

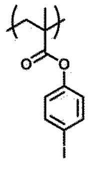 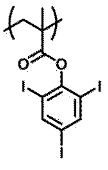 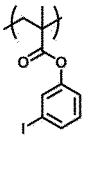 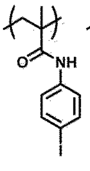 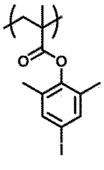 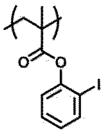 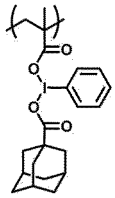 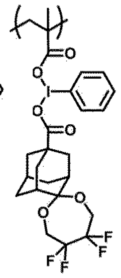 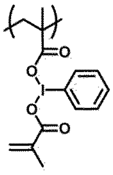

A content of the repeating unit having a fluorine atom or an iodine atom is preferably 0% by mole or more, more preferably 5% by mole or more, and still more preferably 10% by mole or more with respect to all repeating units in the acid-decomposable resin. The upper limit thereof is preferably 50% by mole or less, more preferably 45% by mole or less, and still more preferably 40% by mole or less with respect to all repeating units in the acid-decomposable resin.

<Repeating Unit represented by Formula (V-1) or Formula (V-2)>

The acid-decomposable resin may have a repeating unit represented by Formula (V-1) or Formula (V-2).
The repeating unit represented by Formula (V-1) and Formula (V-2) is preferably a repeating unit different from the above-described repeating units.

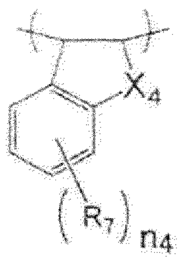

(V-1)

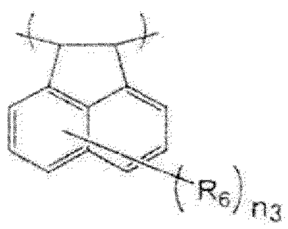

(V-2)

In the formula, $R^6$ and $R^7$ each independently represent a hydrogen atom, a hydroxyl group, an alkyl group, an alkoxy group, an acyloxy group, a cyano group, a nitro group, an amino group, a halogen atom, -COO- (-OCOR or -COOR; R is an alkyl group or fluorinated alkyl group having 1 to 6 carbon atoms), or a carboxy group. As the alkyl group, a linear, branched, or cyclic alkyl group having 1 to 10 carbon atoms is preferable.

$n_3$ represents an integer of 0 to 6.
$n_4$ represents an integer of 0 to 4.
$X^4$ is a methylene group, an oxygen atom, or a sulfur atom.

Examples of the repeating unit represented by Formula (V-1) or Formula (V-2) include repeating units described in paragraph [0100] of WO2018/193954A.
A content of the repeating unit represented by Formula (V-1) or Formula (V-2) is preferably 1% to 65% by mole, and more preferably 5% to 45% by mole with respect to all repeating units in the acid-decomposable resin.

<Repeating Unit for Reducing Mobility of Main Chain>

The acid-decomposable resin may have a repeating unit for reducing mobility of the main chain, as a repeating unit different from the repeating unit a.
From the viewpoint that excessive diffusion of a generated acid or pattern collapse during development can be suppressed, the acid-decomposable resin preferably has a high glass transition temperature (Tg). The Tg is preferably higher than 90°C, more preferably higher than 100°C, still more preferably higher than 110°C, and particularly preferably higher than 125°C. Since an excessively high Tg causes a decrease in dissolution rate in a developer, the Tg is preferably 400°C or lower and more preferably 350°C or lower.
In the present specification, the glass transition temperature (Tg) of a polymer such as the acid-decomposable resin is calculated by the following method. First, each Tg of homopolymers consisting of only the respective repeating

units included in the polymer is calculated by the Bicerano method. Hereinafter, the Tg calculated is referred to as a "Tg of the repeating unit". Next, the mass proportion (%) of each repeating unit to all repeating units in the polymer is calculated. Next, the Tg at each mass proportion is calculated using a Fox's equation (described in Materials Letters 62 (2008) 3152, and the like), and these are summed to obtain the Tg (°C) of the polymer.

The Bicerano method is described in Prediction of polymer properties, Marcel Dekker Inc., New York (1993), and the like. In addition, the calculation of Tg by the Bicerano method can be carried out using MDL Polymer (MDL Information Systems, Inc.), which is software for estimating physical properties of a polymer.

In order to raise the Tg of the acid-decomposable resin (preferably to raise the Tg to higher than 90°C), it is preferable to reduce the mobility of the main chain of the acid-decomposable resin. Examples of a method for lowering the mobility of the main chain of the acid-decomposable resin include the following (a) to (e) methods.

(a) introduction of a bulky substituent into the main chain
(b) introduction of a plurality of substituents into the main chain
(c) introduction of a substituent causing an interaction between the acid-decomposable resins into the vicinity of the main chain
(d) formation of the main chain in a cyclic structure
(e) linking of a cyclic structure to the main chain

The acid-decomposable resin preferably has a repeating unit in which the homopolymer exhibits a Tg of 130°C or higher.

The type of the repeating unit in which the homopolymer exhibits a Tg of 130°C or higher may be any of repeating units in which the homopolymer exhibits a Tg of 130°C or higher, as calculated by a Bicerano method. It corresponds to a repeating unit having a Tg of a homopolymer exhibiting 130°C or higher, depending on the type of a functional group in the repeating units represented by Formulae (A) to (E), which will be described later.

(Repeating Unit Represented by Formula (A))

Specific examples of the (a) include a method of introducing a repeating unit represented by Formula (A) into the acid-decomposable resin.

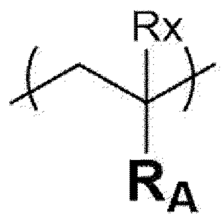

(A)

In Formula (A), $R_A$ represents a group having a polycyclic structure. $R_x$ represents a hydrogen atom, a methyl group, or an ethyl group. The group having a polycyclic structure is a group having a plurality of ring structures, and the plurality of ring structures may or may not be fused.

Examples of the repeating unit represented by Formula (A) include repeating units described in paragraphs [0107] to [0119] of WO2018/193954A.

A content of the repeating unit represented by Formula (A) is preferably 1% to 65% by mole, and more preferably 5% to 45% by mole with respect to all repeating units in the acid-decomposable resin.

(Repeating Unit Represented by Formula (B))

Specific examples of the (b) include a method of introducing a repeating unit represented by Formula (B) into the acid-decomposable resin.

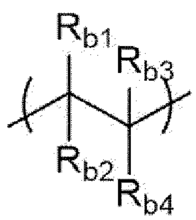

(B)

In Formula (B), $R_{b1}$ to $R_{b4}$ each independently represent a hydrogen atom or an organic group, and at least two or more of $R_{b1}$, ..., or $R_{b4}$ represent an organic group.

In addition, in a case where at least one of the organic groups is a group in which a ring structure is directly linked to the main chain in the repeating unit, the types of the other organic groups are not particularly limited.

In addition, in a case where none of the organic groups is a group in which a ring structure is directly linked to the main chain in the repeating unit, at least two or more of the organic groups are substituents having three or more constituent atoms excluding hydrogen atoms.

Examples of the repeating unit represented by Formula (B) include repeating units described in paragraphs [0113] to [0115] of WO2018/193954A.

A content of the repeating unit represented by Formula (B) is preferably 1% to 65% by mole, and more preferably 5% to 45% by mole with respect to all repeating units in the acid-decomposable resin.

(Repeating Unit Represented by Formula (C))

Specific examples of the (c) include a method of introducing a repeating unit represented by Formula (C) into the acid-decomposable resin.

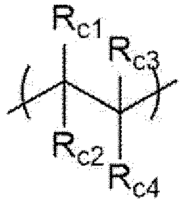 (C)

In Formula (C), $R_{c1}$ to $R_{c4}$ each independently represent a hydrogen atom or an organic group, and at least one of $R_{c1}$, ..., or $R_{c4}$ is a group having a hydrogen-bonding hydrogen atom with the number of atoms of 3 or less from the main chain carbon. Among these, it is preferable that the group has hydrogen-bonding hydrogen atoms with the number of atoms of 2 or less (on a side closer to the vicinity of the main chain) to cause an interaction between the main chains of the acid-decomposable resin.

Examples of the repeating unit represented by Formula (C) include repeating units described in paragraphs [0119] to [0121] of WO2018/193954A.

A content of the repeating unit represented by Formula (C) is preferably 1% to 65% by mole, and more preferably 5% to 45% by mole with respect to all repeating units in the acid-decomposable resin.

(Repeating Unit Represented by Formula (D))

Specific examples of the (d) include a method of introducing a repeating unit represented by Formula (D) into the acid-decomposable resin.

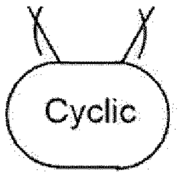 (D)

In Formula (D), "Cyclic" is a group which forms a main chain as a cyclic structure. The number of ring-constituting atoms is not particularly limited.

Examples of the repeating unit represented by Formula (D) include repeating units described in paragraphs [0126] to [0027] of WO2018/193954A.

A content of the repeating unit represented by Formula (D) is preferably 1% to 65% by mole, and more preferably 5% to 45% by mole with respect to all repeating units in the acid-decomposable resin.

(Repeating Unit Represented by Formula (E))

Specific examples of the (e) include a method of introducing a repeating unit represented by Formula (E) into the acid-decomposable resin.

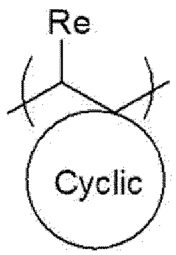

(E)

In Formula (E), Re's each independently represent a hydrogen atom or an organic group. Examples of the organic group include an alkyl group, a cycloalkyl group, an aryl group, an aralkyl group, and an alkenyl group, which may have a substituent.
"Cyclic" is a cyclic group including a carbon atom of a main chain. The number of atoms included in the cyclic group is not particularly limited.
Examples of the repeating unit represented by Formula (E) include repeating units described in paragraphs [0131] to [0133] of WO2018/193954A.
A content of the repeating unit represented by Formula (E) is preferably 1% to 65% by mole, and more preferably 5% to 45% by mole with respect to all repeating units in the acid-decomposable resin.

<Repeating Unit having Hydroxyl Group or Cyano Group>

The acid-decomposable resin may have a repeating unit having a hydroxyl group or a cyano group.
As a result, adhesiveness to the substrate and/or affinity for a developer is improved.
The repeating unit having a hydroxyl group or a cyano group is preferably a repeating unit having an alicyclic hydrocarbon structure substituted with a hydroxyl group or a cyano group.
It is preferable that the repeating unit having a hydroxyl group or a cyano group does not have the acid-decomposable group. Examples of the repeating unit having a hydroxyl group or a cyano group include repeating units described in paragraphs [0153] to [0158] of WO2020/004306A.
A content of the repeating unit having a hydroxyl group or a cyano group is preferably 1% to 65% by mole, and more preferably 5% to 45% by mole with respect to all repeating units in the acid-decomposable resin.

<Repeating Unit having Alicyclic Hydrocarbon Structure and Not Exhibiting Acid Decomposability>

The acid-decomposable resin may have a repeating unit having an alicyclic hydrocarbon structure and not exhibiting acid decomposability. As a result, it is possible to reduce elution of low-molecular-weight components from the resist film into the immersion liquid during liquid immersion exposure. Examples of such a repeating unit include a repeating unit derived from 1-adamantyl (meth)acrylate, diadamantyl (meth)acrylate, tricyclodecanyl (meth)acrylate, or cyclohexyl (meth)acrylate.
A content of the repeating unit having an alicyclic hydrocarbon structure and not exhibiting acid decomposability is preferably 1% to 65% by mole, and more preferably 5% to 45% by mole with respect to all repeating units in the acid-decomposable resin.

<Repeating Unit Represented by Formula (III) having Neither Hydroxyl Group Nor Cyano Group>

The acid-decomposable resin may have a repeating unit represented by Formula (III), which has neither a hydroxyl group nor a cyano group.

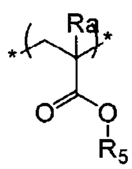

(III)

In Formula (III), $R_5$ represents a hydrocarbon group having at least one cyclic structure and having neither a hydroxyl group nor a cyano group.
Ra represents a hydrogen atom, an alkyl group, or a $-CH_2-O-Ra_2$ group. $Ra_2$ represents a hydrogen atom, an

alkyl group, or an acyl group.

The cyclic structure included in $R_5$ includes a monocyclic hydrocarbon group and a polycyclic hydrocarbon group. Examples of the monocyclic hydrocarbon group include a cycloalkyl group having 3 to 12 carbon atoms (more preferably having 3 to 7 carbon atoms) and a cycloalkenyl group having 3 to 12 carbon atoms.

Examples of detailed definitions of each group in Formula (III) and examples of the repeating unit include those described in paragraphs [0169] to [0173] of WO2020/004306A.

A content of the repeating unit represented by Formula (III), which has neither a hydroxyl group nor a cyano group, is preferably 1% to 65% by mole, and more preferably 5% to 45% by mole with respect to all repeating units in the acid-decomposable resin.

<Other Repeating Units>

The acid-decomposable resin may have other repeating units in addition to the above-described repeating units.

The other repeating units are not particularly limited as long as a repeating unit other than the above-described repeating units.

For example, the acid-decomposable resin may have, as the other repeating units, a repeating unit selected from the group consisting of a repeating unit having an oxathiane ring group, a repeating unit having an oxazolone ring group, a repeating unit having a dioxane ring group, a repeating unit having a hydantoin ring group, and a repeating unit having a sulfolane ring group.

A content of the other repeating units is preferably 1% to 65% by mole, and more preferably 5% to 45% by mole with respect to all repeating units in the acid-decomposable resin.

The other repeating units are shown below.

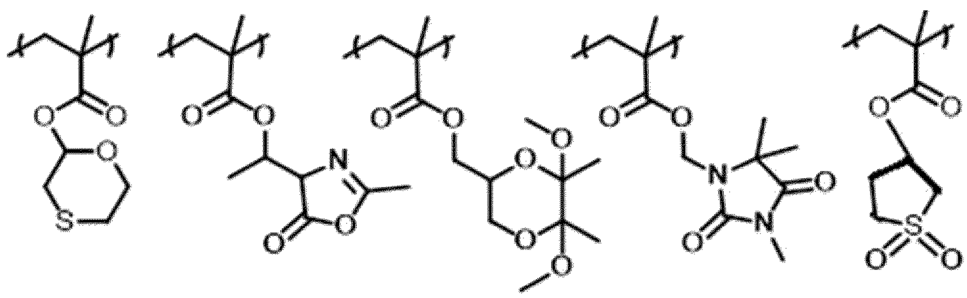

For the purpose of controlling dry etching resistance, suitability for a standard developer, substrate adhesiveness, resist profile, resolving power, heat resistance, sensitivity, and the like, the acid-decomposable resin may have various repeating units in addition to the repeating units described above.

The acid-decomposable resin can be synthesized in accordance with an ordinary method (for example, a radical polymerization).

A weight-average molecular weight of the acid-decomposable resin as a value expressed in terms of polystyrene by a GPC method is preferably 1,000 to 200,000, more preferably 3,000 to 20,000, and still more preferably 5,000 to 15,000. By setting the weight-average molecular weight of the acid-decomposable resin to 1,000 to 200,000, deterioration of heat resistance and dry etching resistance can be further suppressed. In addition, deterioration of developability and deterioration of film-forming properties due to high viscosity can also be further suppressed.

A dispersity (molecular weight distribution) of the acid-decomposable resin is usually 1 to 5, and preferably 1.00 to 3.00, more preferably 1.20 to 3.00, and still more preferably 1.20 to 2.00. As the dispersity is smaller, resolution and resist shape are more excellent, and a side wall of a resist pattern is smoother and roughness is also more excellent.

In the resist composition, a content of the acid-decomposable resin is preferably 10.0% to 99.0% by mass, more preferably 20.0% to 98.0% by mass, and still more preferably 25.0% to 95.0% by mass with respect to the total solid content of the resist composition.

In addition, the acid-decomposable resin may be used alone or in combination of two or more kinds thereof. In a case where two or more kinds thereof are used, a total content thereof is preferably within the suitable content range.

[Photoacid Generator]

The resist composition may contain one or more kinds (hereinafter, also referred to as "specific photoacid generator") selected from the group consisting of compounds (I) and (II), as a compound (photoacid generator) which generates an acid by irradiation with actinic ray or radiation.

As will be described later, the resist composition may further contain a photoacid generator other than the specific photoacid generator (hereinafter, also simply referred to as "other photoacid generators"). In addition, the resist composition may contain a compound (III).

The photoacid generator does not include the above-described compound (1).

Hereinafter, first, the specific photoacid generator (compounds (I) and (II)) will be described.

<Compound (I)>

The compound (I) is a compound having one or more of the following structural moieties X and one or more of the following structural moieties Y, in which the compound generates an acid including the following first acidic moiety derived from the following structural moiety X and the following second acidic moiety derived from the following structural moiety Y by irradiation with actinic ray or radiation.

> Structural moiety X: a structural moiety which consists of an anionic moiety $A_1^-$ and a cationic moiety $M_1^+$, and forms a first acidic moiety represented by $HA_1$ by irradiation with actinic ray or radiation
> Structural moiety Y: a structural moiety which consists of an anionic moiety $A_2^-$ and a cationic moiety $M_2^+$, and forms a second acidic moiety represented by $HA_2$ by irradiation with an actinic ray or a radiation

However, the compound (I) satisfies the following condition I.

Condition I: a compound PI, which is formed by, in the compound (I), replacing the cationic moiety $M_1^+$ in the structural moiety X and the cationic moiety $M_2^+$ in the structural moiety Y with $H^+$, has an acid dissociation constant a1 derived from the acidic moiety represented by $HA_1$, formed by replacing the cationic moiety $M_1^+$ in the structural moiety X with $H^+$, and has an acid dissociation constant a2 derived from the acidic moiety represented by $HA_2$, formed by replacing the cationic moiety $M_2^+$ in the structural moiety Y with $H^+$, in which the acid dissociation constant a2 is larger than the acid dissociation constant a1

Hereinafter, the condition I will be described in more detail.

In a case where the compound (I) is, for example, a compound that generates an acid having one first acidic moiety derived from the above-described structural moiety X and one second acidic moiety derived from the above-described structural moiety Y, the compound PI corresponds to "compound having $HA_1$ and $HA_2$".

As the acid dissociation constant a1 and the acid dissociation constant a2 of such a compound PI, more specifically, in a case of obtaining acid dissociation constants of the compound PI, a pKa in a case where the compound PI is to be "compound having $A_1^-$ and $HA_2$" is defined as the acid dissociation constant a1, and a pKa in a case where the "compound having $A_1^-$ and $HA_2$" is to be "compound having $A_1^-$ and $A_2^-$" is defined as the acid dissociation constant a2.

In addition, in a case where the compound (I) is, for example, a compound that generates an acid having two first acidic moieties derived from the above-described structural moiety X and one second acidic moiety derived from the above-described structural moiety Y, the compound PI corresponds to "compound having two $HA_1$'s and one $HA_2$".

In a case of obtaining acid dissociation constants of such a compound PI, an acid dissociation constant in a case where the compound PI is to be "compound having one $A_1^-$, one $HA_1$, and one $HA_2$" and an acid dissociation constant in a case where the "compound having one $A_1^-$, one $HA_1$, and one $HA_2$" is to be "compound having two $A_1^-$ and one $HA_2$" correspond to the above-described acid dissociation constant a1. In addition, an acid dissociation constant in a case where the "compound having two $A_1^-$ and one $HA_2$" is to be "compound having two $A_1^-$ and one $A_2^-$" corresponds to the acid dissociation constant a2. That is, in the compound PI, in a case of a plurality of acid dissociation constants derived from the acidic moiety represented by $HA_1$, which is formed by replacing the above-described cationic moiety $M_1^+$ in the above-described structural moiety X with $H^+$, a value of the acid dissociation constant a2 is larger than the largest value of the plurality of acid dissociation constants a1. In a case where the acid dissociation constant in a case where the compound PI is to be the "compound having one $A_1^-$, one $HA_1$, and one $HA_2$" is defined as aa, and the acid dissociation constant in a case where the "compound having one $A_1^-$, one $HA_1$, and one $HA_2$" is to be the "compound having two $A_1^-$ and one $HA_2$" is defined as ab, a relationship between aa and ab satisfies $aa < ab$.

The acid dissociation constant a1 and the acid dissociation constant a2 can be obtained by the above-described method for measuring an acid dissociation constant.

The above-described compound PI corresponds to an acid generated in a case where the compound (I) is irradiated with actinic ray or radiation.

In a case where the compound (I) has two or more of the structural moieties X, the structural moieties X may be the same or different from each other. In addition, two or more of $A_1^-$'s and two or more of $M_1^+$ may be the same or different from each other.

In addition, in the compound (I), $A_1^-$ and $A_2^-$, and $M_1^+$ and $M_2^+$ may be the same or different from each other, but it is preferable that $A_1^-$ and $A_2^-$ are different from each other.

From the viewpoint that LWR performance of the pattern to be formed is more excellent, in the above-described

compound PI, a difference between the acid dissociation constant a1 (in a case of a preferably of acid dissociation constants a1, the maximum value thereof) and the acid dissociation constant a2 is preferably 0.1 or more, more preferably 0.5 or more, and still more preferably 1.0 or more. The upper limit of the difference between the acid dissociation constant a1 (in a case of a preferably of acid dissociation constants a1, the maximum value thereof) and the acid dissociation constant a2 is preferably 16 or less.

In addition, from the viewpoint that LWR performance of the pattern to be formed is more excellent, the acid dissociation constant a2 in the above-described compound PI is, for example, 20 or less, preferably 15 or less. The lower limit of the acid dissociation constant a2 is preferably -4.0 or more.

In addition, from the viewpoint that LWR performance of the pattern to be formed is more excellent, the acid dissociation constant a1 in the above-described compound PI is preferably 2.0 or less, and more preferably 0 or less. The lower limit of the acid dissociation constant a1 is preferably -20.0 or more.

The anionic moiety $A_1^-$ and the anionic moiety $A_2^-$ are structural moieties including a negatively charged atom or atomic group, and examples thereof include structural moieties selected from the group consisting of Formulae (AA-1) to (AA-3), and Formulae (BB-1) to (BB-6). The anionic moiety $A_1^-$ is preferably an acidic moiety capable of forming an acidic moiety having a small acid dissociation constant, and among these, any one of Formulae (AA-1) to (AA-3) is more preferable. In addition, the anionic moiety $A_2^-$ is preferably an acidic moiety capable of forming an acidic moiety having a larger acid dissociation constant than the anionic moiety $A_1^-$, and it is more preferably selected from any one of Formulae (BB-1) to (BB-6). In Formulae (AA-1) to (AA-3) and Formulae (BB-1) to (BB-6), * represents a bonding position.

In Formula (AA-2), $R^A$ represents a monovalent organic group. Examples of the monovalent organic group represented by $R^A$ include a cyano group, a trifluoromethyl group, and a methanesulfonyl group.

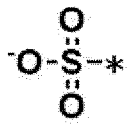
AA-1

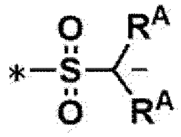
AA-2

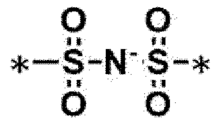
AA-3

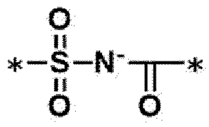
BB-1

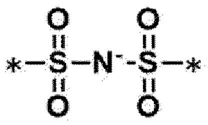
BB-2

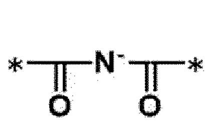
BB-3

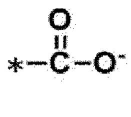
BB-4

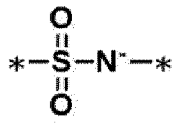
BB-5

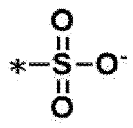
BB-6

In addition, the cationic moiety $M_1^+$ and the cationic moiety $M_2^+$ are a structural moiety including a positively charged atom or atomic group, and examples thereof include an organic cation having a charge of 1. Examples of the organic cation include the same organic cation represented by $M_{11}^+$ and $M_{12}^+$ in Formula (Ia-1) described below.

Examples of a specific structure of the compound (I) include compounds represented by Formulae (Ia-1) to (Ia-5) described below.

Hereinafter, first, the compound represented by Formula (Ia-1) will be described. The compound represented by Formula (Ia-1) is as follows.

$$M_{11}^+ \ A_{11}^- \text{-} L_1 \text{-} A_{12}^- \text{-} M_{12}^+ \qquad \text{(Ia-1)}$$

The compound (Ia-1) generates an acid represented by $HA_{11}$-$L_1$-$A_{12}H$ by irradiation with actinic ray or radiation.

In Formula (Ia-1), $M_{11}^+$ and $M_{12}^+$ each independently represent an organic cation.

$A_{11}^-$ and $A_{12}^-$ each independently represent a monovalent anionic functional group.

$L_1$ represents a divalent linking group.

$M_{11}^+$ and $M_{12}^+$ may be the same or different from each other.

$A_{11}^-$ and $A_{12}^-$ may be the same or different from each other, but it is preferable to be different from each other.

However, in the compound PIa ($HA_{11}$-$L_1$-$A_{12}H$) formed by replacing organic cations represented by $M_{11}^+$ and $M_{12}^+$ with $H^+$ in Formula (Ia-1), the acid dissociation constant a2 derived from the acidic moiety represented by $A_{12}H$ is larger than the acid dissociation constant a1 derived from the acidic moiety represented by $HA_{11}$. Suitable values of the acid dissociation constant a1 and the acid dissociation constant a2 are as described above. In addition, the acid generated from the compound PIa and the acid generated from the compound represented by Formula (Ia-1) by irradiation with actinic ray or radiation are the same.

In addition, at least one of $M_{11}^+$, $M_{12}^+$, $A_{11}^-$, $A_{12}^-$, or $L_1$ may have an acid-decomposable group as a substituent.

The organic cations represented by $M_1^+$ and $M_2^+$ in Formula (Ia-1) are as described later.

The monovalent anionic functional group represented by $A_{11}^-$ means a monovalent group including the above-described anionic moiety $A_1^-$. In addition, the monovalent anionic functional group represented by $A_{12}^-$ means a monovalent group including the above-described anionic moiety $A_2^-$.

As the monovalent anionic functional group represented by $A_{11}^-$ and $A_{12}^-$, a monovalent anionic functional group including the anionic moiety of any of Formulae (AA-1) to (AA-3) and Formulae (BB-1) to (BB-6) described above is preferable, and a monovalent anionic functional group selected from the group consisting of Formulae (AX-1) to (AX-3) and Formulae (BX-1) to (BX-7) is more preferable.

As the monovalent anionic functional group represented by $A_{11}^-$, a monovalent anionic functional group represented by any of Formulae (AX-1) to (AX-3) is preferable. In addition, as the monovalent anionic functional group represented by $A_{12}^-$, a monovalent anionic functional group represented by any of Formulae (BX-1) to (BX-7) is preferable, and a monovalent anionic functional group represented by any of Formulae (BX-1) to (BX-6) is more preferable.

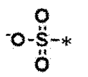
AX-1

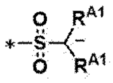
AX-2

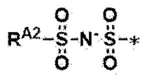
AX-3

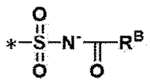
BX-1

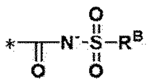
BX-2

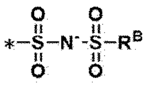
BX-3

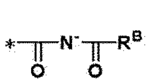
BX-4

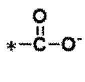
BX-5

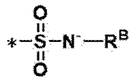
BX-6

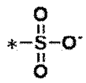
BX-7

In Formulae (AX-1) to (AX-3), $R^{A1}$ and $R^{A2}$ each independently represent a monovalent organic group. * represents a bonding position.

Examples of the monovalent organic group represented by $R^{A1}$ include a cyano group, a trifluoromethyl group, and a methanesulfonyl group.

As the monovalent organic group represented by $R^{A2}$, a linear, branched, or cyclic alkyl group, or an aryl group is preferable.

The number of carbon atoms in the above-described alkyl group is preferably 1 to 15, more preferably 1 to 10, and still more preferably 1 to 6.

The above-described alkyl group may have a substituent. As the substituent, a fluorine atom or a cyano group is preferable, and a fluorine atom is more preferable. In a case where the above-described alkyl group has a fluorine atom as the substituent, the substituent may be a perfluoroalkyl group.

The above-described aryl group is preferably a phenyl group or a naphthyl group, and more preferably a phenyl group.

The above-described aryl group may have a substituent. As the substituent, a fluorine atom, an iodine atom, a perfluoroalkyl group (for example, preferably having 1 to 10 carbon atoms and more preferably having 1 to 6 carbon atoms), or a cyano group is preferable, and a fluorine atom, an iodine atom, or a perfluoroalkyl group is more preferable.

In Formulae (BX-1) to (BX-4) and (BX-6), $R^B$ represents a monovalent organic group. * represents a bonding position.

As the monovalent organic group represented by $R^B$, a linear, branched, or cyclic alkyl group, or an aryl group is preferable.

The number of carbon atoms in the above-described alkyl group is preferably 1 to 15, more preferably 1 to 10, and still more preferably 1 to 6.

The above-described alkyl group may have a substituent. As the substituent, a fluorine atom or a cyano group is preferable, and a fluorine atom is more preferable. In a case where the above-described alkyl group has a fluorine atom as the substituent, the substituent may be a perfluoroalkyl group.

In a case where the carbon atom to be the bonding position in the alkyl group (for example, in the case of Formulae (BX-1) and (BX-4), a carbon atom directly bonded to -CO-specified in the alkyl group of the formulae; in the case of Formulae (BX-2) and (BX-3), a carbon atom directly bonded to $-SO_2-$ specified in the alkyl group of the formulae; and in the case of Formula (BX-6), a carbon atom directly bonded to $N^-$ specified in the alkyl group of the formula) has a substituent, the substituent is also preferably a fluorine atom or a substituent other than a cyano group.

In addition, the above-described alkyl group may have a carbon atom substituted with a carbonyl carbon.

The above-described aryl group is preferably a phenyl group or a naphthyl group, and more preferably a phenyl group.

The above-described aryl group may have a substituent. As the substituent, a fluorine atom, an iodine atom,

a perfluoroalkyl group (for example, preferably having 1 to 10 carbon atoms and more preferably having 1 to 6 carbon atoms), a cyano group, an alkyl group (for example, preferably having 1 to 10 carbon atoms and more preferably having 1 to 6 carbon atoms), an alkoxy group (for example, preferably having 1 to 10 carbon atoms and more preferably having 1 to 6 carbon atoms), or an alkoxycarbonyl group (for example, preferably having 2 to 10 carbon atoms and more preferably having 2 to 6 carbon atoms) is preferable; and a fluorine atom, an iodine atom, a perfluoroalkyl group, an alkyl group, an alkoxy group, or an alkoxycarbonyl group is more preferable.

In Formula (Ia-1), examples of the divalent linking group represented by $L_1$ include -CO-, -NR-, -CO-, -O-, -S-, -SO-, $-SO_2-$, an alkylene group (preferably having 1 to 6 carbon atoms; may be linear or branched), a cycloalkylene group (preferably having 3 to 15 carbon atoms), an alkenylene group (preferably having 2 to 6 carbon atoms), a divalent aliphatic heterocyclic group (preferably a 5- to 10-membered ring, more preferably a 5- to 7-membered ring, and still more preferably a 5- or 6-membered ring; each having at least one of a nitrogen atom, an oxygen atom, a sulfur atom, or an Se atom in the ring structure), and a divalent aromatic heterocyclic group (preferably a 5- to 10-membered ring, more preferably a 5- to 7-membered ring, and still more preferably a 5- or 6-membered ring; each having at least one of a nitrogen atom, an oxygen atom, a sulfur atom, or an Se atom in the ring structure), a divalent aromatic hydrocarbon ring group (preferably a 6- to 10-membered ring, and more preferably a 6-membered ring), and a divalent linking group formed by a combination of a plurality of these groups. Examples of R include a hydrogen atom and a monovalent organic group. As the monovalent organic group, for example, an alkyl group (preferably having 1 to 6 carbon atoms) is preferable.

The above-described alkylene group, the above-described cycloalkylene group, the above-described alkenylene group, and the above-described divalent aliphatic heterocyclic group, divalent aromatic heterocyclic group, and divalent aromatic hydrocarbon ring group may have a substituent. Examples of the substituent include a halogen atom (preferably, a fluorine atom).

As the divalent linking group represented by $L_1$, a divalent linking group represented by Formula (L1) is preferable.

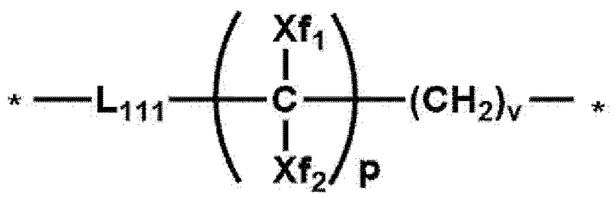

(L1)

In Formula (L1), $L_{111}$ represents a single bond or a divalent linking group.

Examples of the divalent linking group represented by $L_{111}$ include -CO-, -NH-, -O-, -SO-, $-SO_2-$, an alkylene group which may have a substituent (preferably having 1 to 6 carbon atoms; may be linear or branched), a cycloalkylene group which may have a substituent (preferably having 3 to 15 carbon atoms), an aryl which may have a substituent (preferably having 6 to 10 carbon atoms), and a divalent linking group formed by a combination of a plurality of these groups. Examples of the substituent include a halogen atom.

p represents an integer of 0 to 3, and preferably represents an integer of 1 to 3.
v represents an integer of 0 or 1.

$Xf_1$'s each independently represent a fluorine atom or an alkyl group substituted with at least one fluorine atom. The number of carbon atoms in the alkyl group is preferably 1 to 10 and more preferably 1 to 4. In addition, the alkyl group substituted with at least one fluorine atom is preferably a perfluoroalkyl group.

$Xf_2$'s each independently represent a hydrogen atom, an alkyl group which may have a fluorine atom as a substituent, or a fluorine atom. The number of carbon atoms in the alkyl group is preferably 1 to 10 and more preferably 1 to 4. Among these, $Xf_2$ preferably represents a fluorine atom or an alkyl group substituted with at least one fluorine atom, and more preferably represents a fluorine atom or a perfluoroalkyl group.

Among these, $Xf_1$ and $Xf_2$ are each independently preferably a fluorine atom or a perfluoroalkyl group having 1 to 4 carbon atoms and more preferably a fluorine atom or $CF_3$, and it is still more preferable that both $Xf_1$ and $Xf_2$ are fluorine atoms.

* represents a bonding position.

In a case where $L_{11}$ in Formula (Ia-1) represents the divalent linking group represented by Formula (L1), it is preferable that the bonding site (*) on the Lm side of Formula (L1) is bonded to $A_{12}^-$ of Formula (Ia-1).

In (Ia-1), preferred forms of the organic cations represented by $M_{11}^+$ and $M_{12}^+$ will be described in detail.

The organic cations represented by $M_{11}^+$ and $M_{12}^+$ each independently preferably an organic cation represented by Formula (ZaI) (cation (ZaI)) or an organic cation represented by Formula (ZaII) (cation (ZaII)).

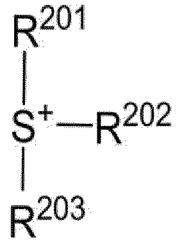

(ZaI)

$R^{204}$-$I^+$-$R^{205}$ (ZaII)

In Formula (ZaI), $R^{201}$, $R^{202}$, and $R^{203}$ each independently represent an organic group.

The number of carbon atoms in the organic group of $R^{201}$, $R^{202}$, and $R^{203}$ is usually 1 to 30, and preferably 1 to 20. In addition, two of $R^{201}$ to $R^{203}$ may be bonded to each other to form a ring structure, and the ring structure may include an oxygen atom, a sulfur atom, -COO-, an amide group, or a carbonyl group in the ring. Examples of the group formed by the bonding of two of $R^{203}$ to $R^{203}$ include an alkylene group (for example, a butylene group and a pentylene group) and $-CH_2-CH_2-O-CH_2-CH_2-$.

Examples of suitable aspects of the organic cation in Formula (ZaI) include a cation (ZaI-1), a cation (ZaI-2), an organic cation (cation (ZaI-3b)) represented by Formula (ZaI-3b), and an organic cation (cation (ZaI-4b)) represented by Formula (ZaI-4b), each of which will be described later.

First, the cation (ZaI-1) will be described.

The cation (ZaI-1) is an arylsulfonium cation in which at least one of $R^{201}$, $R^{202}$, or $R^{203}$ of Formula (ZaI) described above is an aryl group.

In the arylsulfonium cation, all of $R^{203}$ to $R^{203}$ may be aryl groups, or some of $R^{203}$ to $R^{203}$ may be an aryl group and the rest may be an alkyl group or a cycloalkyl group.

In addition, one of $R^{203}$ to $R^{203}$ may be an aryl group, the remaining two of $R^{203}$ to $R^{203}$ may be bonded to each other to form a ring structure, and an oxygen atom, a sulfur atom, -COO-, an amide group, or a carbonyl group may be included in the ring. Examples of the group formed by the bonding of two of $R^{203}$ to $R^{203}$ include an alkylene group (for example, a butylene group, a pentylene group, or $-CH_2-CH_2-O-CH_2-CH_2-$) in which one or more methylene groups may be substituted with an oxygen atom, a sulfur atom, -COO-, an amide group, and/or a carbonyl group.

Examples of the arylsulfonium cation include a triarylsulfonium cation, a diarylalkylsulfonium cation, an aryldialkylsulfonium cation, a diarylcycloalkylsulfonium cation, and an aryldicycloalkylsulfonium cation.

The aryl group included in the arylsulfonium cation is preferably a phenyl group or a naphthyl group, and more preferably a phenyl group. The aryl group may be an aryl group which has a heterocyclic structure having an oxygen atom, a nitrogen atom, a sulfur atom, or the like. Examples of the heterocyclic structure include a pyrrole residue, a furan residue, a thiophene residue, an indole residue, a benzofuran residue, and a benzothiophene residue. In a case where the arylsulfonium cation has two or more aryl groups, the two or more aryl groups may be the same or different from each other.

The alkyl group or the cycloalkyl group included in the arylsulfonium cation as necessary is preferably a linear alkyl group having 1 to 15 carbon atoms, a branched alkyl group having 3 to 15 carbon atoms, or a cycloalkyl group having 3 to 15 carbon atoms, and for example, a methyl group, an ethyl group, a propyl group, an n-butyl group, a sec-butyl group, a t-butyl group, a cyclopropyl group, a cyclobutyl group, a cyclohexyl group, or the like is more preferable.

The substituents which may be included in the aryl group, alkyl group, and cycloalkyl group of $R^{203}$ to $R^{203}$ are each independently preferably an alkyl group (for example, having 1 to 15 carbon atoms), a cycloalkyl group (for example, having 3 to 15 carbon atoms), an aryl group (for example, having 6 to 14 carbon atoms), an alkoxy group (for example, having 1 to 15 carbon atoms), a cycloalkyl alkoxy group (for example, having 1 to 15 carbon atoms), a halogen atom (for example, fluorine and iodine), a hydroxyl group, a carboxy group, -COO-, a sulfinyl group, a sulfonyl group, an alkylthio group, or a phenylthio group.

The substituent may further have a substituent if possible, and for example, it is also preferable that the above-described alkyl group has a halogen atom as the substituent to form an alkyl halide group such as a trifluoromethyl group.

In addition, it is also preferable to form an acid-decomposable group by any combination of the above-described substituents.

The acid-decomposable group is intended to a group which is decomposed by action of acid to form an acid group, and preferably has a structure in which an acid group is protected by a leaving group which is eliminated by action

of acid. The above-described acid group and acid-leaving group are as described above.

Next, the cation (ZaI-2) will be described.

The cation (ZaI-2) is a cation in which $R^{203}$ to $R^{203}$ in Formula (ZaI) are each independently a cation representing an organic group having no aromatic ring. The aromatic ring also encompasses an aromatic ring including a heteroatom.

The number of carbon atoms in the organic group as $R^{203}$ to $R^{203}$, which has no aromatic ring, is generally 1 to 30, and preferably 1 to 20.

$R^{203}$ to $R^{203}$ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

Examples of the alkyl group and cycloalkyl group of $R^{201}$ to $R^{203}$ include a linear alkyl group having 1 to 10 carbon atoms or a branched alkyl group having 3 to 10 carbon atoms (for example, a methyl group, an ethyl group, a propyl group, a butyl group, and a pentyl group), and a cycloalkyl group having 3 to 10 carbon atoms (for example, a cyclopentyl group, a cyclohexyl group, and a norbornyl group).

$R^{203}$ to $R^{203}$ may further be substituted with a halogen atom, an alkoxy group (for example, having 1 to 5 carbon atoms), a hydroxyl group, a cyano group, or a nitro group.

In addition, it is also preferable that the substituents of $R^{203}$ to $R^{203}$ each independently form an acid-decomposable group by any combination of the substituents.

Next, the cation (ZaI-3b) will be described.

The cation (ZaI-3b) is a cation represented by Formula (ZaI-3b).

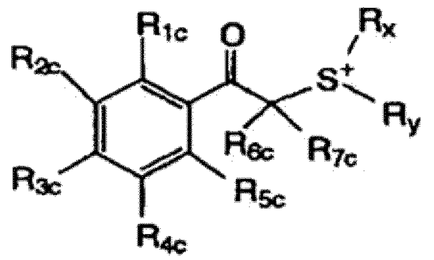

(ZaI-3b)

In Formula (ZaI-3b), $R_{1c}$ to $R_{5c}$ each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxyl group, a nitro group, an alkylthio group, or an arylthio group.

$R_{6c}$ and $R_{7c}$ each independently represent a hydrogen atom, an alkyl group (a t-butyl group and the like), a cycloalkyl group, a halogen atom, a cyano group, or an aryl group.

$R_x$ and $R_y$ each independently represent an alkyl group, a cycloalkyl group, a 2-oxoalkyl group, a 2-oxocycloalkyl group, an alkoxycarbonylalkyl group, an allyl group, or a vinyl group.

In addition, it is also preferable that the substituents of $R_{1c}$ to $R_{7c}$, $R_x$, and $R_y$ each independently form an acid-decomposable group by any combination of the substituents.

Any two or more of $R_{1c}$, ... , or $R_{5c}$, $R_{5c}$ and $R_{6c}$, $R_{6c}$ and $R_{7c}$, $R_{5c}$ and $R_x$, and $R_x$ and $R_y$ may each be bonded to each other to form a ring, and the rings may each independently include an oxygen atom, a sulfur atom, a ketone group, an ester bond, or an amide bond.

Examples of the above-described ring include an aromatic or non-aromatic hydrocarbon ring, an aromatic or non-aromatic heterocyclic ring, and a polycyclic fused ring formed by a combination of two or more of these rings. Examples of the ring include a 3- to 10-membered ring, and the ring is preferably a 4- to 8-membered ring and more preferably a 5- or 6-membered ring.

Examples of the group formed by the bonding of any two or more of $R_{1c}$, ... , or $R_{5c}$, $R_{6c}$ and $R_{7c}$, and $R_x$ and $R_y$ include an alkylene group such as a butylene group and a pentylene group. A methylene group in this alkylene group may be substituted with a heteroatom such as an oxygen atom.

As the group formed by the bonding of $R_{5c}$ and $R_{6c}$, and $R_{5c}$ and $R_x$, a single bond or an alkylene group is preferable. Examples of the alkylene group include a methylene group and an ethylene group.

The ring formed by bonding $R_{1c}$ to $R_{5c}$, $R_{6c}$, $R_{7c}$, $R_x$, $R_y$, any two or more of $R_{1c}$, ... , or $R_{5c}$, $R_{5c}$ and $R_{6c}$, $R_{6c}$ and $R_{7c}$, $R_{5c}$ and $R_x$, and $R_x$ and $R_y$ to each other may have a substituent.

Next, the cation (ZaI-4b) will be described.

The cation (ZaI-4b) is a cation represented by Formula (ZaI-4b).

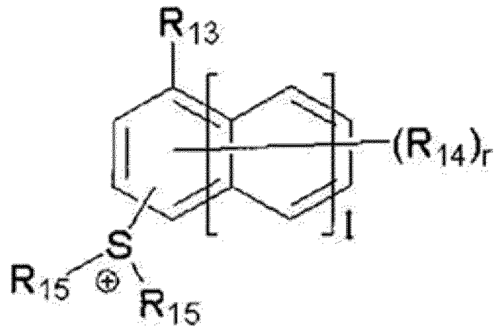

(ZaI-4b)

In Formula (ZaI-4b),

1 represents an integer of 0 to 2.
r represents an integer of 0 to 8.

$R_{13}$ represents a hydrogen atom, a halogen atom (for example, a fluorine atom, an iodine atom, or the like), a hydroxyl group, an alkyl group, an alkyl halide group, an alkoxy group, a carboxy group, an alkoxycarbonyl group, or a group having a cycloalkyl group (which may be the cycloalkyl group itself or a group including the cycloalkyl group in a part thereof). These groups may further have a substituent.

$R_{14}$ represents a hydroxyl group, a halogen atom (for example, a fluorine atom, an iodine atom, or the like), an alkyl group, an alkyl halide group, an alkoxy group, an alkoxycarbonyl group, an alkylcarbonyl group, an alkylsulfonyl group, a cycloalkyl sulfonyl group, or a group having a cycloalkyl group (which may be the cycloalkyl group itself or a group including the cycloalkyl group in a part thereof). These groups may have a substituent. In a case of a plurality of $R_{14}$'s, $R_{14}$'s each independently represent the above-described group such as a hydroxyl group.

$R_{15}$'s each independently represent an alkyl group, a cycloalkyl group, or a naphthyl group. Two $R_{15}$'s may be bonded to each other to form a ring. In a case where two $R_{15}$'s are bonded to each other to form a ring, the ring skeleton may include a heteroatom such as an oxygen atom and a nitrogen atom.

In one aspect, it is preferable that two $R_{15}$'s are alkylene groups and are bonded to each other to form a ring structure. The above-described alkyl group, the above-described cycloalkyl group, the above-descried naphthyl group, and the ring formed by bonding two $R_{15}$'s to each other may have a substituent.

The alkyl group of $R_{13}$, $R_{14}$, and $R_{15}$ may be linear or branched. The number of carbon atoms in the alkyl group is preferably 1 to 10. The alkyl group is more preferably a methyl group, an ethyl group, an n-butyl group, a t-butyl group, or the like.

In addition, it is also preferable that the substituents of $R_{13}$ to $R_{15}$, $R_x$, and $R_y$ each independently form an acid-decomposable group by any combination of the substituents.

Next, Formula (ZaII) will be described.

In Formula (ZaII), $R^{204}$ and $R^{205}$ each independently represent an aryl group, an alkyl group, or a cycloalkyl group.

The aryl group of $R^{204}$ and $R^{205}$ is preferably a phenyl group or a naphthyl group, and more preferably a phenyl group. The aryl group of $R^{204}$ and $R^{205}$ may be an aryl group which has a heterocyclic ring having an oxygen atom, a nitrogen atom, a sulfur atom, or the like. Examples of a skeleton of the aryl group having a heterocyclic ring include pyrrole, furan, thiophene, indole, benzofuran, and benzothiophene.

The alkyl group and cycloalkyl group of $R^{204}$ and $R^{205}$ are preferably a linear alkyl group having 1 to 10 carbon atoms or a branched alkyl group having 3 to 10 carbon atoms (for example, a methyl group, an ethyl group, a propyl group, a butyl group, and a pentyl group), or a cycloalkyl group having 3 to 10 carbon atoms (for example, a cyclopentyl group, a cyclohexyl group, and a norbornyl group).

The aryl group, the alkyl group, and the cycloalkyl group of $R^{204}$ and $R^{205}$ may each independently have a substituent. Examples of the substituent which may be included in each of the aryl group, the alkyl group, and the cycloalkyl group of $R^{204}$ and $R^{205}$ include an alkyl group (for example, having 1 to 15 carbon atoms), a cycloalkyl group (for example, having 3 to 15 carbon atoms), an aryl group (for example, having 6 to 15 carbon atoms), an alkoxy group (for example, having 1 to 15 carbon atoms), a halogen atom, a hydroxyl group, and a phenylthio group. In addition, it is also preferable that the substituents of $R^{204}$ and $R^{205}$ each independently form an acid-decomposable group by any combination of the substituents.

Next, Formulae (Ia-2) to (Ia-4) will be described.

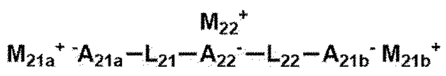

(Ia-2)

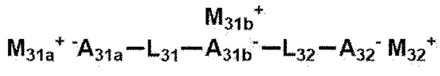

(Ia-3)

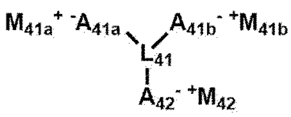

(Ia-4)

In Formula (Ia-2), $A_{21a}^-$ and $A_{21b}^-$ each independently represent a monovalent anionic functional group. Here, the monovalent anionic functional group represented by $A_{21a}^-$ and $A_{21b}^-$ means a monovalent group including the above-described anionic moiety $A_1^-$. Examples of the monovalent anionic functional group represented by $A_{21a}^-$ and $A_{21b}^-$ include the monovalent anionic functional group selected from the group consisting of Formulae (AX-1) to (AX-3) described above.

$A_{22}^-$ represents a divalent anionic functional group. Here, the divalent anionic functional group represented by $A_{22}^-$ means a divalent group including the above-described anionic moiety $A_2^-$. Examples of the divalent anionic functional group represented by $A_{22}^-$ include a divalent anionic functional group represented by Formulae (BX-8) to (BX-11).

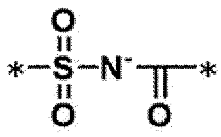

BX-8

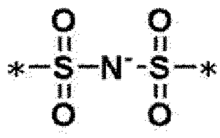

BX-9

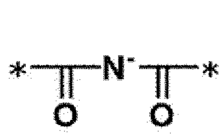

BX-10

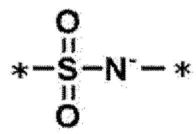

BX-11

$M_{21a}^+$, $M_{21b}^+$, and $M_{22}^+$ each independently represent an organic cation. The organic cation represented by $M_{21a}^+$, $M_{21b}^+$, and $M_{22}^+$ has the same meaning as $M_1^+$ described above, and a suitable aspect thereof is also the same.

$L_{21}$ and $L_{22}$ each independently represent a divalent organic group.

In addition, in a compound PIa-2 of Formula (Ia-2), in which the organic cation represented by $M_{21a}^+$, $M_{21b}^+$, and $M_{22}^+$ is replaced with $H^+$, an acid dissociation constant a2 derived from an acidic moiety represented by $A_{22}H$ is larger than an acid dissociation constant a1-1 derived from $A_{21a}H$ and an acid dissociation constant a1-2 derived from an acidic moiety represented by $A_{21b}H$. The acid dissociation constant a1-1 and the acid dissociation constant a1-2 correspond to the above-described acid dissociation constant a1.

$A_{21a}^-$ and $A_{21b}^-$ may be the same or different from each other. In addition, $M_{21a}^+$, $M_{21b}^+$, and $M_{22}^+$ may be the same or different from each other.

In addition, at least one of $M_{21a}^+$, $M_{21b}^+$, $M_{22}^+$, $A_{21a}^-$, $A_{21b}^-$, $L_{21}$, or $L_{22}$ may have an acid-decomposable group as a substituent.

In Formula (Ia-3), $A_{31a}^-$ and $A_{32}^-$ each independently represent a monovalent anionic functional group. The definition of the monovalent anionic functional group represented by $A_{31a}^-$ is the same as $A_{21a}^-$ and $A_{21b}^-$ in Formula (Ia-2), and a suitable aspect thereof is also the same.

The monovalent anionic functional group represented by $A_{32}^-$ means a monovalent group including the above-described anionic moiety $A_2^-$. Examples of the monovalent anionic functional group represented by $As_2^-$ include the monovalent anionic functional group selected from the group consisting of Formulae (BX-1) to (BX-7) described above.

$A_{31b}^-$ represents a divalent anionic functional group. Here, the divalent anionic functional group represented by $A_{31b}^-$ means a divalent group including the above-described anionic moiety $A_1^-$. Examples of the divalent anionic functional group represented by $A_{31b}^-$ include a divalent anionic functional group represented by Formula (AX-4).

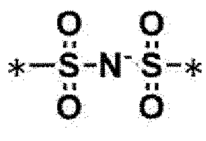

AX-4

$M_{31a}^+$, $M_{31b}^+$, and $M_{32}^+$ each independently represent a monovalent organic cation. The organic cation of $M_{31a}^+$, $M_{31b}^+$, and $M_{32}^+$ has the same meaning as $M_1^+$ described above, and a suitable aspect thereof is also the same.

$L_{31}$ and $L_{32}$ each independently represent a divalent organic group.

In addition, in a compound PIa-3 of Formula (Ia-3), in which the organic cation represented by $M_{31a}^+$, $M_{31b}^+$, and $M_{32}^+$ is replaced with $H^+$, an acid dissociation constant a2 derived from an acidic moiety represented by $A_{32}H$ is larger than an acid dissociation constant a1-3 derived from an acidic moiety represented by $A_{31a}H$ and an acid dissociation constant a1-4 derived from an acidic moiety represented by $A_{31b}H$. The acid dissociation constant a1-3 and the acid dissociation constant a1-4 correspond to the above-described acid dissociation constant a1.

$A_{31a}^-$ and $A_{32}^-$ may be the same or different from each other. In addition, $M_{31a}^+$, $M_{31b}^+$, and $M_{32}^+$ may be the same or different from each other.

In addition, at least one of $M_{31a}^+$, $M_{31b}^+$, $M_{32}^+$, $A_{31a}^-$, $A_{32}^-$, $L_{31}$, or $L_{32}$ may have an acid-decomposable group as a substituent.

In Formula (Ia-4), $A_{41a}^-$, $A_{41b}^-$, and $A_{42}^-$ each independently represent a monovalent anionic functional group. The definition of the monovalent anionic functional group represented by $A_{41a}^-$ and $A_{41b}^-$ is the same as $A_{21a}^-$ and $A_{21b}^-$ in Formula (Ia-2). In addition, the definition of the monovalent anionic functional group represented by $A_{42}^-$ is the same as $A_{32}^-$ in Formula (Ia-3), and a suitable aspect thereof is also the same.

$M_{41a}^+$, $M_{41b}^+$, and $M_{42}^+$ each independently represent an organic cation.

$L_{41}$ represents a trivalent organic group.

In addition, in a compound PIa-4 of Formula (Ia-4), in which the organic cation represented by $M_{41a}^+$, $M_{41b}^+$, and $M_{42}^+$ is replaced with $H^+$, an acid dissociation constant a2 derived from an acidic moiety represented by $A_{42}H$ is larger than an acid dissociation constant a1-5 derived from an acidic moiety represented by $A_{41a}H$ and an acid dissociation constant a1-6 derived from an acidic moiety represented by $A_{41b}H$. The acid dissociation constant a1-5 and the acid dissociation constant a1-6 correspond to the above-described acid dissociation constant a1.

$A_{41a}^-$, $A_{41b}^-$, and $A_{42}^-$ may be the same or different from each other. In addition, $M_{41a}^+$, $M_{41b}^+$, and $M_{42}^+$ may be the same or different from each other.

In addition, at least one of $M_{41a}^+$, $M_{41b}^+$, $M_{42}^+$, $A_{41a}^-$, $A_{41b}^-$, $A_{42}^-$, or $L_{41}$ may have an acid-decomposable group as a substituent.

Examples of the divalent organic group represented by $L_{21}$ and $L_{22}$ in Formula (Ia-2) and $L_{31}$ and $L_{32}$ in Formula (Ia-3) include -CO-, -NR-, -O-, -S-, -SO-, $-SO_2-$, an alkylene group (preferably having 1 to 6 carbon atoms; may be linear or branched), a cycloalkylene group (preferably having 3 to 15 carbon atoms), an alkenylene group (preferably having 2 to 6 carbon atoms), a divalent aliphatic heterocyclic group (preferably a 5- to 10-membered ring, more preferably a 5- to 7-membered ring, and still more preferably a 5- or 6-membered ring; each having at least one of a nitrogen atom, an oxygen atom, a sulfur atom, or an Se atom in the ring structure), and a divalent aromatic heterocyclic group (preferably a 5- to 10-membered ring, more preferably a 5- to 7-membered ring, and still more preferably a 5- or 6-membered ring; each having at least one of a nitrogen atom, an oxygen atom, a sulfur atom, or an Se atom in the ring structure), a divalent aromatic hydrocarbon ring group (preferably a 6- to 10-membered ring, and more preferably a 6-membered ring), and a divalent organic group formed by a combination of a plurality of these groups. Examples of R include a hydrogen atom and a monovalent organic group. As the monovalent organic group, for example, an alkyl group (preferably having 1 to 6 carbon atoms) is preferable.

The above-described alkylene group, the above-described cycloalkylene group, the above-described alkenylene group, and the above-described divalent aliphatic heterocyclic group, divalent aromatic heterocyclic group, and divalent aromatic hydrocarbon ring group may have a substituent. Examples of the substituent include a halogen atom (preferably, a fluorine atom).

As the divalent organic group represented by $L_{21}$ and $L_{22}$ in Formula (Ia-2) and $L_{31}$ and $L_{32}$ in Formula (Ia-3), for example, a divalent organic group represented by Formula (L2) is also preferable.

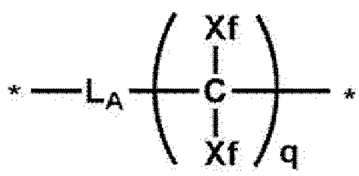

(L2)

In Formula (L2), q represents an integer of 1 to 3. * represents a bonding position.

Xf's each independently represent a fluorine atom or an alkyl group substituted with at least one fluorine atom. The number of carbon atoms in the alkyl group is preferably 1 to 10 and more preferably 1 to 4. In addition, the alkyl group substituted with at least one fluorine atom is preferably a perfluoroalkyl group.

Xf is preferably a fluorine atom or a perfluoroalkyl group having 1 to 4 carbon atoms, and more preferably a

fluorine atom or $CF_3$. It is still more preferable that both Xf's are fluorine atoms.

$L_A$ represents a single bond or a divalent linking group.

Examples of the divalent linking group represented by $L_A$ include -CO-, -O-, -SO-, $-SO_2-$, an alkylene group (preferably having 1 to 6 carbon atoms; may be linear or branched), a cycloalkylene group (preferably having 3 to 15 carbon atoms), a divalent aromatic hydrocarbon ring group (preferably a 6- to 10-membered ring, and more preferably a 6-membered ring), and a divalent linking group formed by a combination of a plurality of these groups.

In addition, the above-described alkylene group, the above-described cycloalkylene group, and the divalent aromatic hydrocarbon ring group may have a substituent. Examples of the substituent include a halogen atom (preferably, a fluorine atom).

Examples of the divalent organic group represented by Formula (L2) include $*-CF_2-*$, $*-CF_2-CF_2-*$, $*-CF_2-CF_2-CF_2-*$, $*-Ph-O-SO_2-CF_2-*$, $*-Ph-O-SO_2-CF_2-CF_2-*$, $*-Ph-O-SO_2-CF_2-CF_2-CF_2-*$, and $*-Ph-OCO-CF_2-*$. Ph is a phenylene group which may have a substituent, and is preferably a 1,4-phenylene group. As the substituent, an alkyl group (preferably having 1 to 10 carbon atoms and more preferably having 1 to 6 carbon atoms) or an alkoxy group (preferably having 1 to 10 carbon atoms and more preferably having 1 to 6 carbon atoms), or an alkoxycarbonyl group (preferably having 2 to 10 carbon atoms and more preferably having 2 to 6 carbon atoms) is preferable.

In a case where $L_{21}$ and $L_{22}$ in Formula (Ia-2) represents the divalent organic group represented by Formula (L2), it is preferable that the bonding site (*) on the $L_A$ side of Formula (L2) is bonded to $A_{21}^-$ and $A_{21b}^-$ of Formula (Ia-2).

In addition, in a case where $L_{31}$ and $L_{32}$ in Formula (Ia-3) represents the divalent organic group represented by Formula (L2), it is preferable that the bonding site (*) on the $L_A$ side of Formula (L2) is bonded to $A_{31a}^-$ and $A_{32}^-$ of Formula (Ia-3).

Examples of the trivalent organic group represented by $L_{41}$ in Formula (Ia-4) include a trivalent organic group represented by Formula (L3).

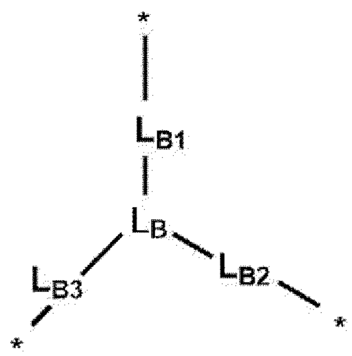

(L3)

In Formula (L3), $L_B$ represents a trivalent hydrocarbon ring group or a trivalent heterocyclic group. * represents a bonding position.

The above-described hydrocarbon ring group may be an aromatic hydrocarbon ring group or an aliphatic hydrocarbon ring group. The number of carbon atoms included in the above-described hydrocarbon ring group is preferably 6 to 18, and more preferably 6 to 14. The above-described heterocyclic group may be an aromatic hydrocarbon ring group or an aliphatic hydrocarbon ring group. The above-described heterocycle is preferably a 5- to 10-membered ring, more preferably a 5- to 7-membered ring, and still more preferably a 5- or 6-membered ring, each of which has at least one nitrogen atom, oxygen atom, sulfur atom, or Se atom in the ring structure.

As $L_B$, a trivalent hydrocarbon ring group is preferable, and a benzene ring group or an adamantane ring group is more preferable. The benzene ring group or the adamantane ring group may have a substituent. Examples of the substituent include a halogen atom (preferably, a fluorine atom).

In addition, in Formula (L3), $L_{B1}$ to $L_{B3}$ each independently represent a single bond or a divalent linking group. Examples of the divalent linking group represented by $L_{B1}$ to $L_{B3}$ include -CO-, -NR-, -O-, -S-, -SO-, $-SO_2-$, an alkylene group (preferably having 1 to 6 carbon atoms; may be linear or branched), a cycloalkylene group (preferably having 3 to 15 carbon atoms), an alkenylene group (preferably having 2 to 6 carbon atoms), a divalent aliphatic heterocyclic group (preferably a 5- to 10-membered ring, more preferably a 5- to 7-membered ring, and still more preferably a 5- or 6-membered ring; each having at least one of a nitrogen atom, an oxygen atom, a sulfur atom, or an Se atom in the ring structure), and a divalent aromatic heterocyclic group (preferably a 5- to 10-membered ring, more preferably a 5- to 7-membered ring, and still more preferably a 5- or 6-membered ring; each having at least one of a nitrogen atom, an

oxygen atom, a sulfur atom, or an Se atom in the ring structure), a divalent aromatic hydrocarbon ring group (preferably a 6- to 10-membered ring, and more preferably a 6-membered ring), and a divalent linking group formed by a combination of these groups. Examples of R include a hydrogen atom and a monovalent organic group. As the monovalent organic group, for example, an alkyl group (preferably having 1 to 6 carbon atoms) is preferable.

The above-described alkylene group, the above-described cycloalkylene group, the above-described alkenylene group, and the above-described divalent aliphatic heterocyclic group, divalent aromatic heterocyclic group, and divalent aromatic hydrocarbon ring group may have a substituent. Examples of the substituent include a halogen atom (preferably, a fluorine atom).

As the divalent linking group represented by $L_{B1}$ to $L_{B3}$ include -CO-, -NR-, -O-, -S-, -SO-, $-SO_2-$, an alkylene group which may have a substituent, or a divalent linking group formed by a combination of these groups is preferable.

As the divalent linking group represented by $L_{B1}$ to $L_{B3}$, a divalent linking group represented by Formula (L3-1) is more preferable.

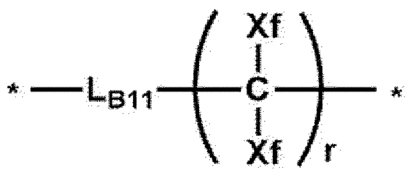

(L3-1)

In Formula (L3-1), $L_{B11}$ represents a single bond or a divalent linking group.

Examples of the divalent linking group represented by $L_{B11}$ include -CO-, -O-, -SO-, $-SO_2-$, an alkylene group which may have a substituent (preferably having 1 to 6 carbon atoms; may be linear or branched), and a divalent linking group formed by a combination of these groups. Examples of the substituent include a halogen atom.

r represents an integer of 1 to 3.
Xf has the same meaning as Xf in Formula (L2) described above, and a suitable aspect thereof is also the same.
* represents a bonding position.

Examples of the divalent linking group represented by $L_{B1}$ to $L_{B3}$ include *-O-*, *-O-$SO_2$-$CF_2$-*, *-O-$SO_2$-$CF_2$-$CF_2$-*, *-O-$SO_2$-$CF_2$-$CF_2$-$CF_2$-*, and *-COO-$CH_2$-$CH_2$-*.

In a case where $L_{41}$ in Formula (Ia-4) includes the divalent organic group represented by Formula (L3-1) and the divalent organic group represented by Formula (L3-1) and $A_{42}^-$ are bonded to each other, it is preferable that the bonding site (*) on the carbon atom side specified in Formula (L3-1) is bonded to $A_{42}^-$ in Formula (Ia-4).

Next, Formula (Ia-5) will be described.

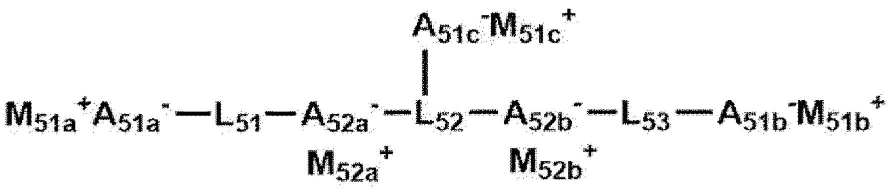

(Ia-5)

In Formula (Ia-5), $A_{51a}^-$, $A_{51b}^-$, and $A_{51c}^-$ each independently represent a monovalent anionic functional group. Here, the monovalent anionic functional group represented by $A_{51a}^-$, $A_{51b}^-$, and $A_{51c}^-$ means a monovalent group including the above-described anionic moiety $A_1^-$. Examples of the monovalent anionic functional group represented by $A_{51a}^-$, $A_{51b}^-$, and $A_{51c}^-$ include the monovalent anionic functional group selected from the group consisting of Formulae (AX-1) to (AX-3) described above.

$A_{52a}^-$ and $A_{52b}^-$ represents a divalent anionic functional group. Here, the divalent anionic functional group represented by $A_{52a}^-$ and $A_{52b}^-$ means a divalent group including the above-described anionic moiety $A_2^-$. Examples of the divalent anionic functional group represented by $A_{22}^-$ include the divalent anionic functional group selected from the group consisting of Formulae (BX-8) to (BX-11) described above.

$M_{51a}^+$, $M_{51b}^+$, $M_{51c}^+$, $M_{52a}^+$, and $M_{52b}^+$ each independently represent an organic cation. The organic cation represented by $M_{51a}^+$, $M_{51b}^+$, $M_{51c}^+$, $M_{52a}^+$, and $M_{52b}^+$ has the same meaning as $M_1^+$ described above, and a suitable aspect thereof is also the same.

$L_{51}$ and $L_{53}$ each independently represent a divalent organic group. The divalent organic group represented by $L_{51}$ and $L_{53}$ has the same meaning as $L_{21}$ and $L_{22}$ in Formula (Ia-2) described above, and a suitable aspect thereof is also the same.

$L_{52}$ represents a trivalent organic group. The trivalent organic group represented by $L_{52}$ has the same meaning as $L_{41}$ in Formula (Ia-4) described above, and a suitable aspect thereof is also the same.

In addition, in a compound PIa-5 of Formula (Ia-5), in which the organic cation represented by $M_{51a}^+$, $M_{51b}^+$, $M_{51c}^+$, $M_{52a}^+$, and $M_{52b}^+$ is replaced with $H^+$, an acid dissociation constant a2-1 derived from an acidic moiety represented by $A_{52a}H$ and an acid dissociation constant a2-2 derived from an acidic moiety represented by $A_{52b}H$ are larger than an acid dissociation constant a1-1 derived from an acidic moiety represented by $A_{51a}H$, an acid dissociation constant a1-2 derived from an acidic moiety represented by $A_{51b}H$, and an acid dissociation constant a1-3 derived from an acidic moiety represented by $A_{51c}H$. The acid dissociation constants a1-1 to a1-3 correspond to the above-described acid dissociation constant a1, and the acid dissociation constants a2-1 and a2-2 correspond to the above-described acid dissociation constant a2.

$A_{51a}^-$, $A_{51b}^-$, and $A_{51c}^-$ may be the same or different from each other. In addition, $A_{52a}^-$ and $A_{52b}^-$ may be the same or different from each other. In addition, $M_{51a}^+$, $M_{51b}^+$, $M_{51c}^+$, $M_{52a}^+$, and $M_{52b}^+$ may be the same or different from each other.

In addition, at least one of $M_{51b}^+$, $M_{51c}^+$, $M_{52a}^+$, $M_{52b}^+$, $A_{51a}^-$, $A_{51b}^-$, $A_{51c}^-$, $L_{51}$, $L_{52}$, or $L_{53}$ may have an acid-decomposable group as a substituent.

<Compound (II)>

The compound (II) is a compound having two or more of the structural moieties X and one or more of the following structural moieties Z, in which the compound generates an acid including two or more of the above-described first acidic moieties derived from the above-described structural moiety X and a structural moiety Z by irradiation with actinic ray or radiation.

Structural moiety Z: a non-ionic moiety capable of neutralizing an acid

In the compound (II), the definition of the structural moiety X and the definitions of $A_1^-$ and $M_1^+$ are the same as the definition of the structural moiety X and the definitions of $A_1^-$ and $M_1^+$ in the above-described compound (I), and suitable aspects thereof are also the same.

In a compound PII formed by replacing the above-described cationic moiety $M_1^+$ in the above-described structural moiety X with $H^+$ in the above-described compound (II), a suitable range of an acid dissociation constant a1 derived from the acidic moiety represented by $HA_1$, formed by replacing the above-described cationic moiety $M_1^+$ in the above-described structural moiety X with $H^+$, is the same as in the acid dissociation constant a1 of the above-described compound PI.

In a case where the compound (II) is, for example, a compound which generates an acid having two of the above-described first acidic moieties derived from the above-described structural moiety X and the above-described structural moiety Z, the compound PII corresponds to "compound having two $HA_1$". In a case of obtaining acid dissociation constants of the compound PII, an acid dissociation constant in a case where the compound PII is to be "compound having one $A_1^-$ and one $HA_1$" and an acid dissociation constant in a case where the "compound having one $A_1^-$ and one $HA_1$" is to be "compound having two $A_1^-$" correspond to the acid dissociation constant a1.

The acid dissociation constant a1 can be obtained by the above-described method for measuring an acid dissociation constant.

The above-described compound PII corresponds to an acid generated in a case where the compound (II) is irradiated with actinic ray or radiation.

The above-described two or more of the structural moieties X may be the same or different from each other. In addition, two or more of $A_1^-$'s and two or more of $M_1^+$ may be the same or different from each other.

The non-ionic moiety capable of neutralizing an acid in the structural moiety Z is preferably, for example, a moiety including a functional group having a group or an electron which is capable of electrostatically interacting with a proton.

Examples of the functional group having a group or electron capable of electrostatically interacting with a proton include a functional group with a macrocyclic structure, such as a cyclic polyether, or a functional group having a nitrogen atom having an unshared electron pair not contributing to π-conjugation. For example, the nitrogen atom having the unshared electron pair, which does not contribute to the π-conjugation, is a nitrogen atom having a partial structure represented by the following formula.

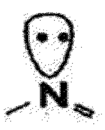 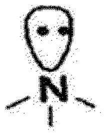 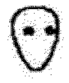

unshared electron pair

Examples of the partial structure of the functional group having a group or electron which is capable of electrostatically interacting with a proton include a crown ether structure, an azacrown ether structure, primary to tertiary amine structures, a pyridine structure, an imidazole structure, and a pyrazine structure, and primary to tertiary amine structures are preferable.

Examples of the compound (II) include compounds represented by Formula (IIa-1) and Formula (IIa-2).

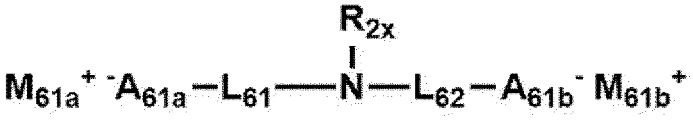

(IIa-1)

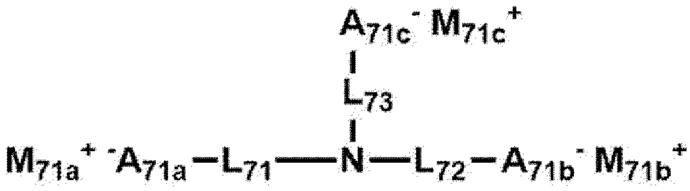

(IIa-2)

In Formula (IIa-1), $A_{61a}^-$ and $A_{61b}^-$ have the same meaning as $A_{11}^-$ in Formula (Ia-1) described above, and suitable aspects thereof are also the same. In addition, $M_{61a}^+$ and $M_{61b}^+$ have the same meaning as $M_{11}^+$ in Formula (Ia-1) described above, and suitable aspects thereof are also the same.

In Formula (IIa-1), $L_{61}$ and $L_{62}$ have the same meaning as $L_1$ in Formula (Ia-1) described above, and suitable aspects thereof are also the same.

In Formula (IIa-1), $R_{2X}$ represents a monovalent organic group. Examples of the monovalent organic group represented by $R_{2X}$ include an alkyl group (which preferably has 1 to 10 carbon atoms, and may be linear or branched), a cycloalkyl group (preferably having 3 to 15 carbon atoms), and an alkenyl group (preferably having 2 to 6 carbon atoms), in which $-CH_2-$ may be substituted with one or a combination of two or more selected from the group consisting of -CO-, -NH-, -O-, -S-, -SO-, and $-SO_2-$.

In addition, the above-described alkylene group, the above-described cycloalkylene group, and the above-described alkenylene group may have a substituent. Examples of the substituent include a halogen atom (preferably, a fluorine atom).

In addition, in a compound PIIa-1 of Formula (IIa-1), in which the organic cation represented by $M_{61a}^+$ and $M_{61b}^+$ is replaced with $H^+$, an acid dissociation constant a1-7 derived from an acidic moiety represented by $A_{61a}H$ and an acid dissociation constant a1-8 derived from an acidic moiety represented by $A_{61b}H$ correspond to the above-described acid dissociation constant a1.

The compound PIIa-1 formed by replacing the above-described cationic moieties $M_{61a}^+$ and $M_{61b}^+$ in the above-described structural moiety X with $H^+$ in the above-described compound (IIa-1) corresponds to $HA_{61a}-L_{61}-N(R_{2X})-L_{62}-A_{61b}H$. In addition, the acid generated from the compound PIIa-1 and the acid generated from the compound represented by Formula (IIa-1) by irradiation with actinic ray or radiation are the same.

In addition, at least one of $M_{61a}^+$, $M_{61b}^+$, $A_{61a}^-$, $A_{61b}^-$, $L_{61}$, $L_{62}$, or $R_{2X}$ may have an acid-decomposable group as a substituent.

In Formula (IIa-2), $A_{71a}^-$, $A_{71b}^-$, and $A_{71c}^-$ have the same meaning as $A_{11}^-$ in Formula (Ia-1) described above, and suitable aspects thereof are also the same. In addition, $M_{71a}^+$, $M_{71b}^+$, and $M_{71c}^+$ have the same meaning as $M_{11}^+$ in Formula (Ia-1) described above, and suitable aspects thereof are also the same.

In Formula (IIa-2), $L_{71}$, $L_{72}$, and $L_{73}$ have the same meaning as $L_1$ in Formula (Ia-1) described above, and

suitable aspects thereof are also the same.

In addition, in a compound PIIa-2 of Formula (IIa-2), in which the organic cation represented by $M_{71a}^+$, $M_{71b}^+$, and $M_{71c}^+$ is replaced with $H^+$, an acid dissociation constant a1-9 derived from an acidic moiety represented by $A_{71a}H$, an acid dissociation constant a1-10 derived from an acidic moiety represented by $A_{71b}H$, and an acid dissociation constant a1-11 derived from an acidic moiety represented by $A_{71c}H$ correspond to the above-described acid dissociation constant a1.

The compound PIIa-2 formed by replacing the above-described cationic moieties $M_{71a}^+$,$M_{71b}^+$, and $M_{71c}^+$ in the above-described structural moiety X in the above-described compound (IIa-1) corresponds to $HA_{71a}$-$L_{71}$-N($L_{73}$-$A_{71c}H$)-$L_{72}$-$A_{71b}H$. In addition, the acid generated from the compound PIIa-2 and the acid generated from the compound represented by Formula (IIa-2) by irradiation with actinic ray or radiation are the same.

In addition, at least one of $M_{71a}^+$, $M_{71b}^+$, $M_{71c}^+$, $A_{71a}^-$, $A_{71b}^-$, $A_{71c}^-$, $L_{71}$, $L_{72}$, or $L_{73}$ may have an acid-decomposable group as a substituent.

Hereinafter, organic cations and other moieties, which can be included in the specific photoacid generator, are exemplified.

The above-described organic cation can be used, for example, as $M_{11}^+$, $M_{12}^+$, $M_{21a}^+$, $M_{21b}^+$, $M_{22}^+$, $M_{31a}^+$, $M_{31b}^+$, $M_{32}^+$, $M_{41a}^+$, $M_{41b}^+$, $M_{42}^+$, $M_{51a}^+$, $M_{51b}^+$, $M_{51c}^+$, $M_{52a}^+$, or $M_{52b}^+$ in the compounds represented by Formulae (Ia-1) to (Ia-5).

The above-described other moieties can be used, for example, as a part other than $M_{11}^+$, $M_{12}^+$, $M_{21a}^+$, $M_{21b}^+$, $M_{22}^+$, $M_{31a}^+$, $M_{31b}^+$, $M_{32}^+$, $M_{41a}^+$, $M_{41b}^+$, $M_{42}^+$, $M_{51a}^+$, $M_{51b}^+$, $M_{51c}^+$, $M_{52a}^+$, and $M_{52b}^+$ in the compounds represented by Formulae (Ia-1) to (Ia-5).

The following organic cation and other moieties may be appropriately combined and used as the specific photoacid generator.

First, examples of the organic cation which can be included in the specific photoacid generator are shown below.

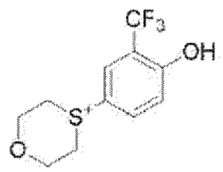
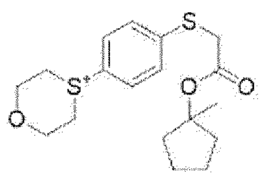
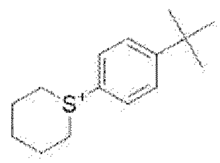
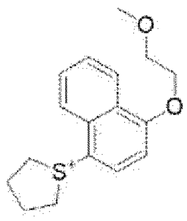

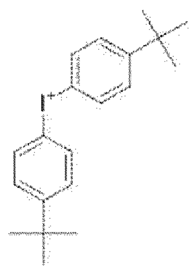
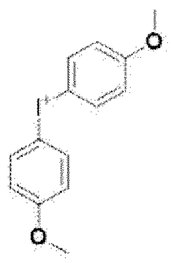
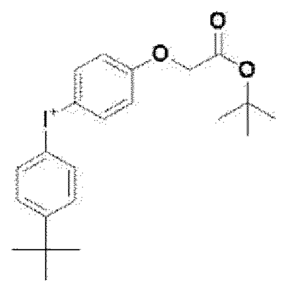

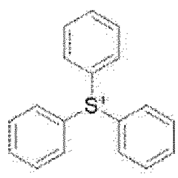
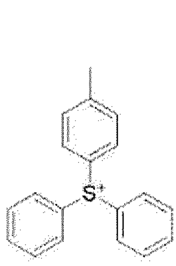
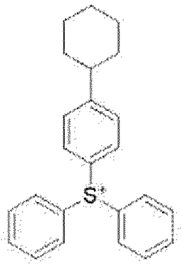
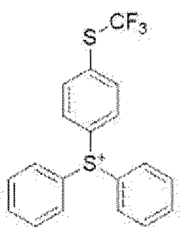
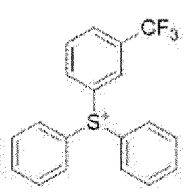
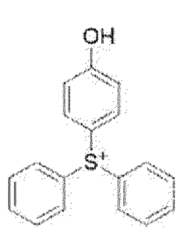

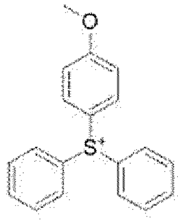
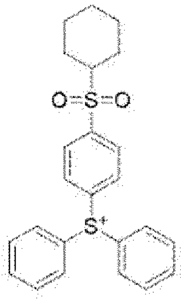
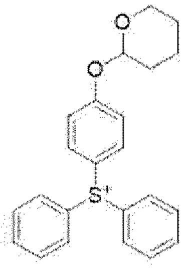
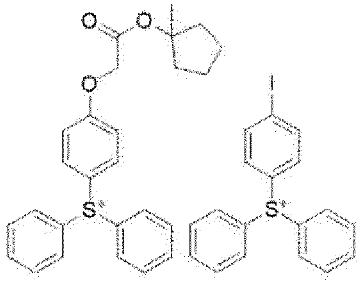

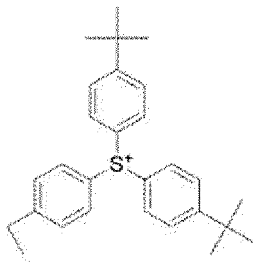
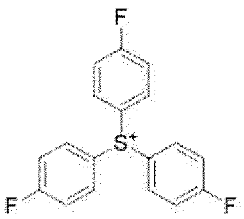
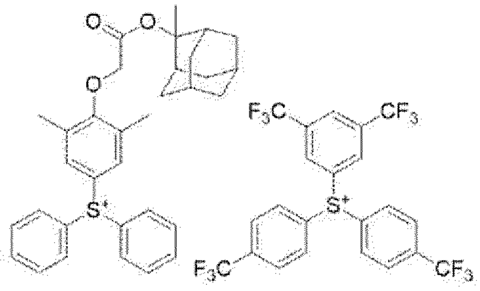

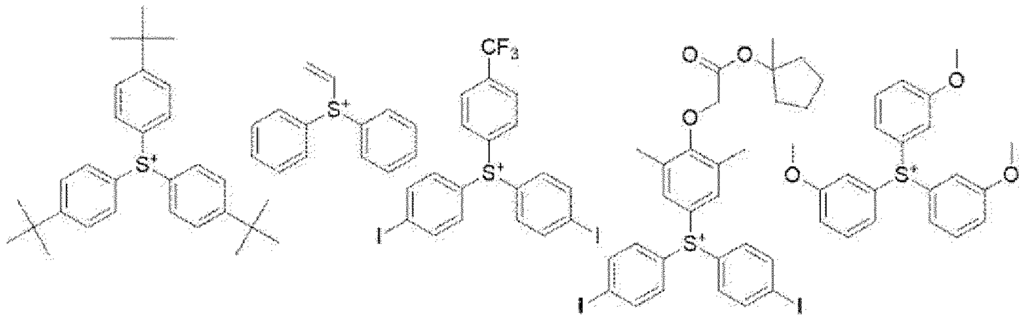

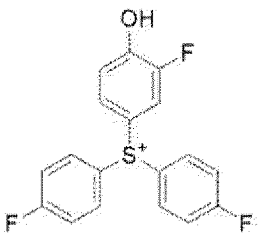
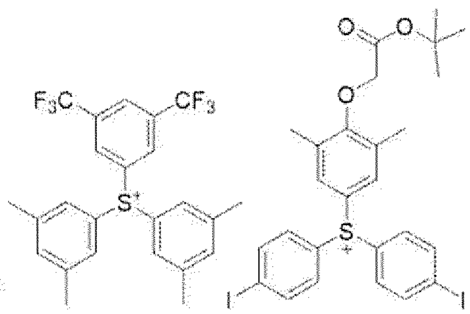
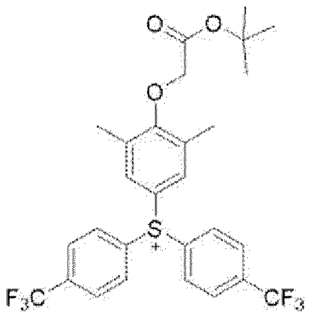

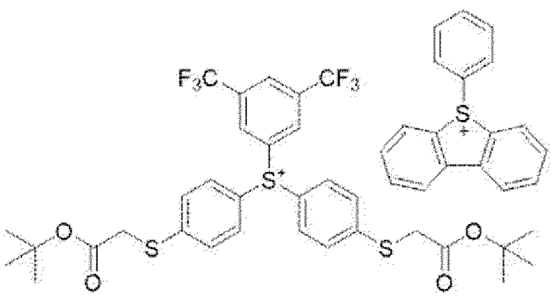
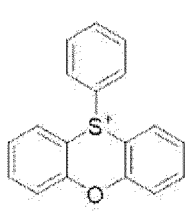
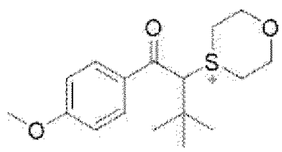

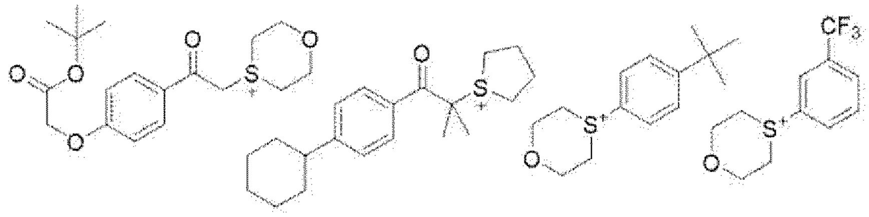

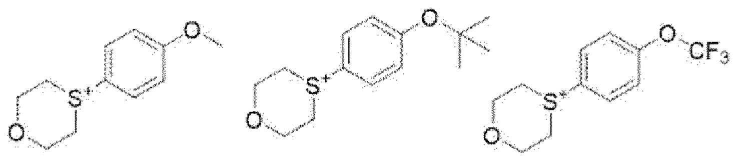

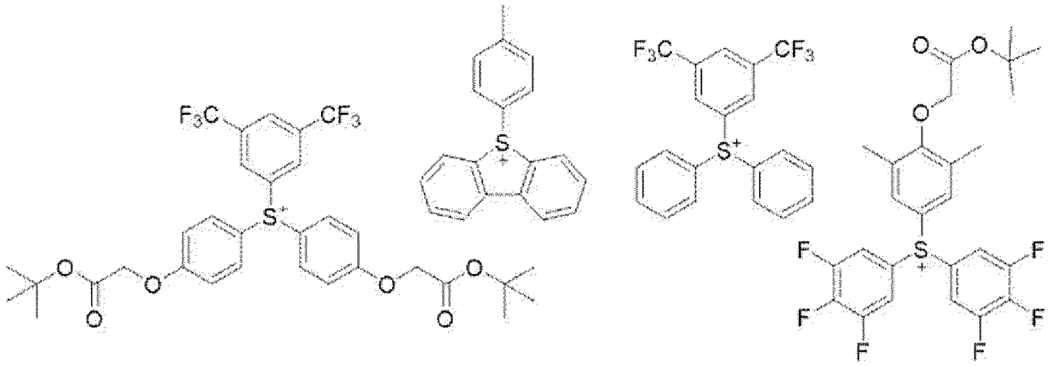

Next, examples of the moiety other than the organic cation, which can be included in the specific photoacid generator, are shown below.

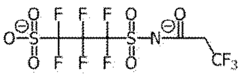
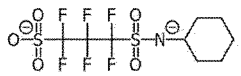
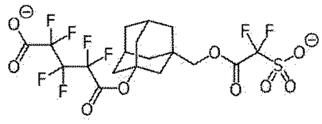

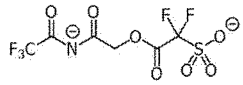
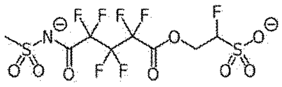
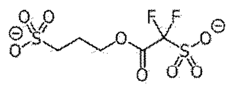

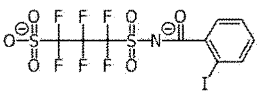
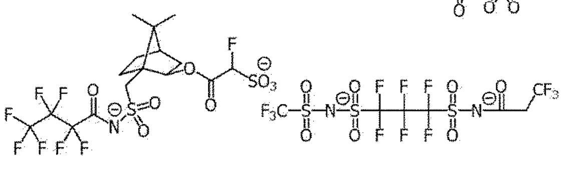

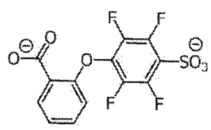
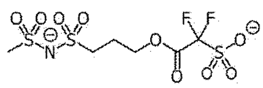
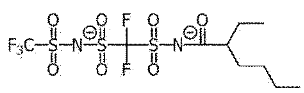

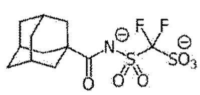
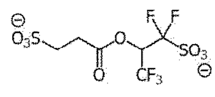
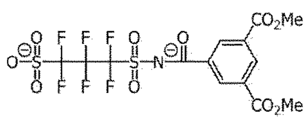

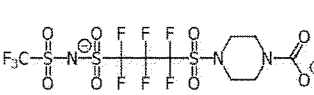
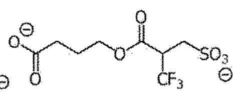
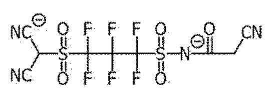

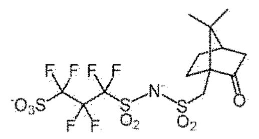
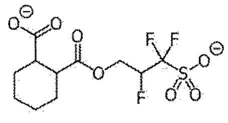
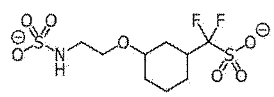

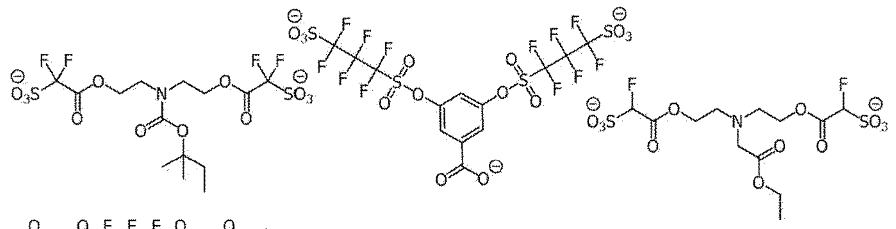

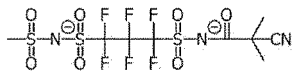

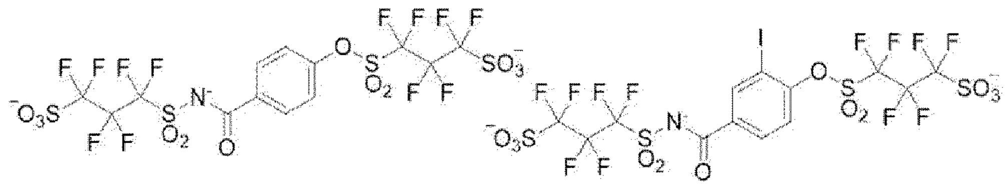

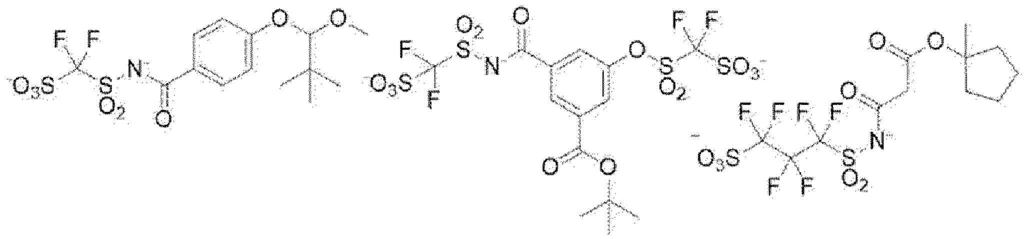

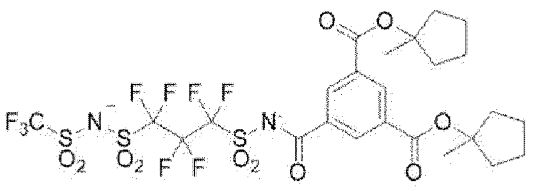
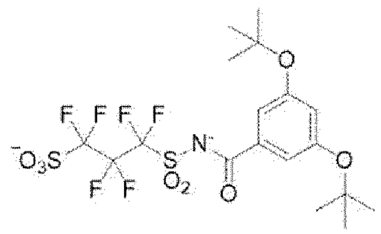

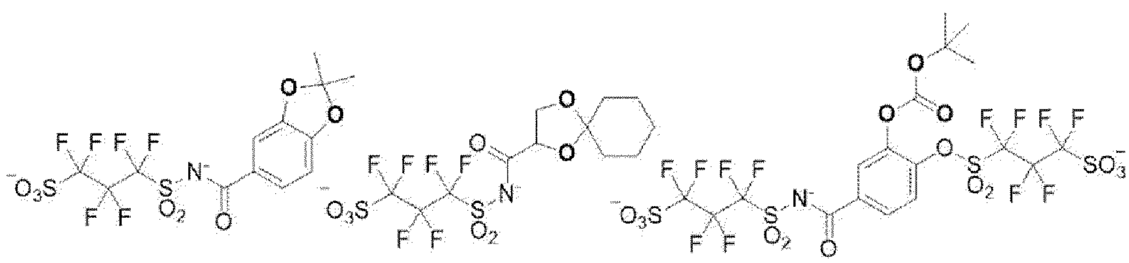

A molecular weight of the specific photoacid generator is preferably 100 to 10,000, more preferably 100 to 2,500, and still more preferably 100 to 1,500.

A content of the specific photoacid generator (total content of the compounds (I) and (II)) is preferably 10% by mass or more, more preferably 15% by mass or more, more preferably 20% by mass or more, and still more preferably 40% by mass or more with respect to the total solid content of the resist composition. The upper limit thereof is preferably 80% by mass or less, more preferably 70% by mass or less, and still more preferably 60% by mass or less with respect

to the total solid content of the resist composition.

The specific photoacid generator may be used alone or in combination of two or more kinds thereof. In a case where two or more kinds thereof are used, a total content thereof is preferably within the suitable content range.

<Compound (III)>

The compound (III) is a compound (onium salt) represented by "$M^+ X^-$", and is preferably a compound which generates an organic acid by exposure.

Examples of the above-described organic acid include sulfonic acid (aliphatic sulfonic acid such as fluoroaliphatic sulfonic acid, aromatic sulfonic acid, camphor sulfonic acid, and the like), bis(alkylsulfonyl)imide acid, and tris(alkylsulfonyl)methide acid.

$M^+$ represents an organic cation.

The above-described organic cations is preferably an organic cation (cation (ZaI)) represented by Formula (ZaI) or an organic cation (cation (ZaII)) represented by Formula (ZaII).

In the compound represented by "$M^+ X^-$", $X^-$ represents an organic anion.

The above-described organic anion is preferably a non-nucleophilic anion (an anion having a significantly low ability to cause a nucleophilic reaction).

Examples of the non-nucleophilic anion include a sulfonate anion (an aliphatic sulfonate anion, an aromatic sulfonate anion, a camphor sulfonate anion, and the like), a sulfonylimide anion, a bis(alkylsulfonyl)imide anion, and a tris(alkylsulfonyl)methide anion.

An aliphatic moiety in the aliphatic sulfonate anion may be an alkyl group or a cycloalkyl group, and a linear or branched alkyl group having 1 to 30 carbon atoms or a cycloalkyl group having 3 to 30 carbon atoms is preferable.

The above-described alkyl group may be, for example, a fluoroalkyl group (which may or may not have a substituent other than a fluorine atom, and may be a perfluoroalkyl group).

An aryl group in the aromatic sulfonate anion and the aromatic carboxylate anion is preferably an aryl group having 6 to 14 carbon atoms, and examples thereof include a phenyl group, a tolyl group, and a naphthyl group.

The alkyl group, cycloalkyl group, and aryl group mentioned above may have a substituent. Specific examples of the substituent include a nitro group, a halogen atom such as a fluorine atom and a chlorine atom, a carboxy group, a hydroxyl group, an amino group, a cyano group, an alkoxy group (preferably having 1 to 15 carbon atoms), an alkyl group (preferably having 1 to 10 carbon atoms), a cycloalkyl group (preferably having 3 to 15 carbon atoms), an aryl group (preferably having 6 to 14 carbon atoms), an alkoxycarbonyl group (preferably having 2 to 12 carbon atoms), an acyl group (preferably having 2 to 12 carbon atoms), an alkoxycarbonyloxy group (preferably having 2 to 18 carbon atoms), an alkylthio group (preferably having 1 to 15 carbon atoms), an alkylsulfonyl group (preferably having 1 to 15 carbon atoms), an alkyliminosulfonyl group (preferably having 1 to 15 carbon atoms), an alkylaminosulfonyl group (preferably having 1 to 15 carbon atoms), and an aryloxysulfonyl group (preferably having 6 to 20 carbon atoms).

As the alkyl group in the bis(alkylsulfonyl)imide anion and the tris(alkylsulfonyl)methide anion, an alkyl group having 1 to 5 carbon atoms is preferable. Examples of a substituent of these alkyl group include a halogen atom, an alkyl group substituted with a halogen atom, an alkoxy group, an alkylthio group, an alkyloxysulfonyl group, an aryloxysulfonyl group, and a cycloalkylaryloxysulfonyl group, and a fluorine atom or an alkyl group substituted with a fluorine atom is preferable.

In addition, the alkyl groups in the bis(alkylsulfonyl)imide anion may be bonded to each other to form a ring structure. As a result, acid strength is increased.

As the non-nucleophilic anion, an aliphatic sulfonate anion in which at least an $\alpha$-position of the sulfonic acid is substituted with a fluorine atom, an aromatic sulfonate anion substituted with a fluorine atom or a group having a fluorine atom, a bis(alkylsulfonyl)imide anion in which an alkyl group is substituted with a fluorine atom, or a tris(alkylsulfonyl)methide anion in which an alkyl group is substituted with a fluorine atom is preferable.

As the non-nucleophilic anion, an anion represented by Formula (AN1) is also preferable.

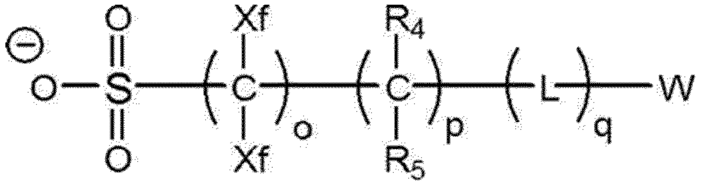
(AN1)

In Formula (AN1), o represents an integer of 1 to 3. p represents an integer of 0 to 10. q represents an integer of 0 to 10.

Xf represents a fluorine atom or an alkyl group substituted with at least one fluorine atom. The number of carbon

atoms in the above-described alkyl group is preferably 1 to 10 and more preferably 1 to 4. In addition, the alkyl group substituted with at least one fluorine atom is preferably a perfluoroalkyl group.

Xf is preferably a fluorine atom or a perfluoroalkyl group having 1 to 4 carbon atoms and more preferably a fluorine atom or $CF_3$, and it is still more preferable that both Xf's are fluorine atoms.

$R_4$ and $R_5$ each independently represent a hydrogen atom, a fluorine atom, an alkyl group, or an alkyl group substituted with at least one fluorine atom. In a case of a plurality of $R_4$'s and Rs's, $R_4$'s and $R_5$'s may be the same or different from each other.

The number of carbon atoms in the alkyl group represented by $R_4$ and $R_5$ is preferably 1 to 4. The above-described alkyl group may further have a substituent. $R_4$ and $R_5$ are preferably a hydrogen atom.

Specific examples and suitable aspects of the alkyl group substituted with at least one fluorine atom are the same as the specific examples and suitable aspects of Xf in Formula (AN1).

L represents a divalent linking group.

In a case of a plurality of L's, L's may be the same or different from each other.

Examples of the divalent linking group include -O-CO-O-, -COO-, -CONH-, -CO-, -O-, -S-, -SO-, $-SO_2-$, an alkylene group (preferably having 1 to 6 carbon atoms), a cycloalkylene group (preferably having 3 to 15 carbon atoms), an alkenylene group (preferably having 2 to 6 carbon atoms), and a divalent linking group formed by a combination of these groups. Among these, as the divalent linking group, -O-CO-O-, -COO-, -CONH-, -CO-, -O-, $-SO_2-$, -O-CO-O-alkylene group-, -COO-alkylene group-, or -CONH-alkylene group- is preferable, and -O-CO-O-, -O-CO-O-alkylene group-, -COO-, -CONH-, $-SO_2-$, or -COO-alkylene group- is more preferable.

W represents an organic group including a cyclic structure. Among these, a cyclic organic group is preferable.

Examples of the cyclic organic group include an alicyclic group, an aryl group, and a heterocyclic group.

The alicyclic group may be monocyclic or polycyclic. Examples of the monocyclic alicyclic group include a monocyclic cycloalkyl group such as a cyclopentyl group, a cyclohexyl group, and a cyclooctyl group. Examples of the polycyclic alicyclic group include a polycyclic cycloalkyl group such as a norbornyl group, a tricyclodecanyl group, a tetracyclodecanyl group, a tetracyclododecanyl group, and an adamantyl group. Among these, an alicyclic group having a bulky structure with 7 or more carbon atoms, such as a norbornyl group, a tricyclodecanyl group, a tetracyclodecanyl group, a tetracyclododecanyl group, and an adamantyl group, is preferable.

The aryl group may be monocyclic or polycyclic. Examples of the above-described aryl group include a phenyl group, a naphthyl group, a phenanthryl group, and an anthryl group.

The heterocyclic group may be monocyclic or polycyclic. Among these, in a case of a polycyclic heterocyclic group, the diffusion of the acid can be further suppressed. In addition, the heterocyclic group may or may not have aromaticity. Examples of a heterocycle having aromaticity include a furan ring, a thiophene ring, a benzofuran ring, a benzothiophene ring, a dibenzofuran ring, a dibenzothiophene ring, and a pyridine ring. Examples of a heterocycle not having aromaticity include a tetrahydropyran ring, a lactone ring, a sultone ring, and a decahydroisoquinoline ring. As the heterocycle in the heterocyclic group, a furan ring, a thiophene ring, a pyridine ring, or a decahydroisoquinoline ring is preferable.

The above-described cyclic organic group may have a substituent. Examples of the above-described substituent include an alkyl group (may be linear or branched; preferably having 1 to 12 carbon atoms), a cycloalkyl group (may be monocyclic, polycyclic, or spirocyclic; preferably having 3 to 20 carbon atoms), an aryl group (preferably having 6 to 14 carbon atoms), a hydroxyl group, an alkoxy group, an ester group, an amide group, a urethane group, a ureido group, a thioether group, a sulfonamide group, and a sulfonic acid ester group. A carbon constituting the cyclic organic group (carbon contributing to ring formation) may be a carbonyl carbon.

As the anion represented by Formula (AN1), $SO_3^-$-$CF_2$-$CH_2$-OCO-$(L)_{q'}$-W, $SO_3^-$-$CF_2$-CHF-$CH_2$-OCO-$(L)_{q'}$-W, $SO_3^-$-$CF_2$-COO-$(L)_{q'}$-W, $SO_3^-$-$CF_2$-$CF_2$-$CH_2$-$CH_2$-$(L)_q$-W, or $SO_3^-$-$CF_2$-CH($CF_3$)-OCO-$(L)_{q'}$-W is preferable. Here, L, q, and W are the same as in Formula (AN1). q' represents an integer of 0 to 10.

As the non-nucleophilic anion, an anion represented by Formula (AN2) is also preferable.

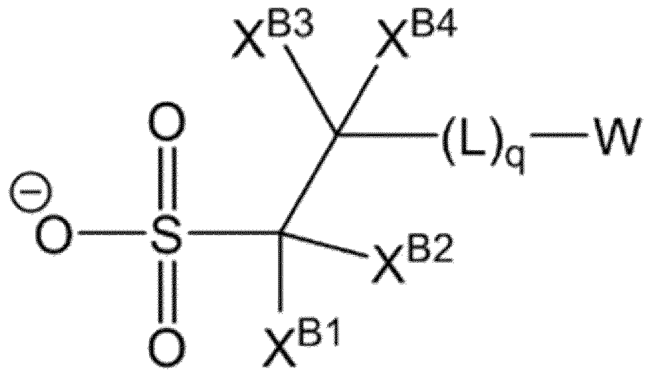

(AN2)

In Formula (AN2), $X^{B1}$ and $X^{B2}$ each independently represent a hydrogen atom or a monovalent organic group not having a fluorine atom.

It is preferable that $X^{B1}$ and $X^{B2}$ are hydrogen atoms.

$X^{B3}$ and $X^{B4}$ each independently represent a hydrogen atom or a monovalent organic group. It is preferable that at least one of $X^{B3}$ or $X^{B4}$ is a fluorine atom or a monovalent organic group having a fluorine atom, and it is more preferable that both $X^{B3}$ and $X^{B4}$ are a fluorine atom or a monovalent organic group having a fluorine atom. It is still more preferable that both $X^{B3}$ and $X^{B4}$ are an alkyl group substituted with fluorine.

L, q, and W are the same as in Formula (AN1).

As the non-nucleophilic anion, an anion represented by Formula (AN3) is preferable.

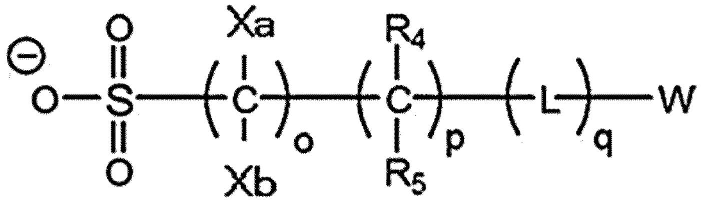

(AN3)

In Formula (AN3), Xa's each independently represent a fluorine atom or an alkyl group substituted with at least one fluorine atom. Xb's each independently represent a hydrogen atom or an organic group not having a fluorine atom. Definitions and suitable aspects of o, p, q, $R_4$, $R_5$, L, and W are the same as in Formula (AN1).

As the non-nucleophilic anion, an anion represented by Formula (AN4) is also preferable.

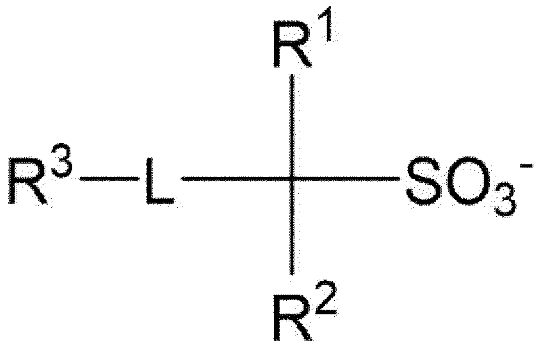

(AN4)

In Formula (AN4), $R^1$ and $R^2$ each independently represent a substituent which is not an electron withdrawing group, or a hydrogen atom.

Examples of the substituent which is not an electron withdrawing group include a hydrocarbon group, a hydroxyl group, an oxyhydrocarbon group, an oxycarbonyl hydrocarbon group, an amino group, a hydrocarbon-substituted amino group, and a hydrocarbon-substituted amide group.

In addition, the substituents which are not an electron withdrawing group are each independently preferably -R', -OH, -OR', -OCOR', $-NH_2$, $-NR'_2$, -NHR', or -NHCOR'. R' is a monovalent hydrocarbon group.

Examples of the above-described monovalent hydrocarbon group represented by R' include an alkyl group such as a methyl group, an ethyl group, a propyl group, and a butyl group; an alkenyl group such as an ethenyl group,

a propenyl group, and a butenyl group; a monovalent linear or branched hydrocarbon group of an alkynyl group or the like, such as an ethynyl group, a propynyl group, and a butynyl group; a cycloalkyl group such as a cyclopropyl group, a cyclobutyl group, a cyclopentyl group, a cyclohexyl group, a norbornyl group, and an adamantyl group; a monovalent alicyclic hydrocarbon group of a cycloalkenyl group or the like, such as a cyclopropenyl group, a cyclobutenyl group, a cyclopentenyl group, and a norbornenyl group; an aryl group such as a phenyl group, a tolyl group, a xylyl group, a mesityl group, a naphthyl group, a methylnaphthyl group, an anthryl group, and a methylanthryl group; and a monovalent aromatic hydrocarbon group of an aralkyl group or the like, such as a benzyl group, a phenethyl group, a phenylpropyl group, a naphthylmethyl group, and an anthrylmethyl group.

Among these, $R^1$ and $R^2$ are each independently a hydrocarbon group (preferably, a cycloalkyl group) or a hydrogen atom.

In Formula (AN4), L represents a divalent linking group including a combination of one or more linking groups S and an alkylene group which may have one or more substituents, or a divalent linking group including one or more linking groups S.

The linking group S is a group selected from the group consisting of $*^A$-O-CO-O-$*^B$, $*^A$-CO-$*^B$ , $*^A$-CO-O-$*^B$ , $*^A$-O-CO-$*^B$, $*^A$-O-$*^B$ , $*^A$-S-$*^B$ , and $*^A$-$SO_2$-$*^B$ .

However, in a case where L is "divalent linking group including a combination of one or more linking groups S and an alkylene group which does not have one or more substituents" which is an aspect of the "divalent linking group including a combination of one or more linking groups S and an alkylene group which may have one or more substituents", the linking group S is preferably a group selected from the group consisting of $*^A$-O-CO-O-$*^B$, $*^A$-CO-$*^B$, $*^A$-O-CO-$*^B$, $*^A$-O-$*^B$, $*^A$-S-$*^B$, and $*^A$-$SO_2$-$*^B$. In other words, in a case where all alkylene groups in the "divalent linking group including a combination of one or more linking groups S and an alkylene group which may have one or more substituents" are unsubstituted alkylene groups, the linking group S is preferably a group selected from the group consisting of $*^A$-O-CO-O-$*^B$, $*^A$-CO-$*^B$, $*^A$-O-CO-$*^B$, $*^A$-O-$*^B$, $*^A$-S-$*^B$, and $*^A$-$SO_2$-$*^B$.

$*^A$ represents a bonding position on the $R^3$ side in Formula (AN4), and *B represents a bonding position on the -$SO_3^-$ side in Formula (AN4).

In the divalent linking group including a combination of one or more linking groups S and an alkylene group which may have one or more substituents, only one linking group S may be present, or two or more thereof may be present. Similarly, only one alkylene group which may have a substituent may be present, or two or more thereof may be present. In a case where the above-described linking groups S are present in a plural number, the above-described linking groups S which are present in a plural number may be the same or different from each other. In a case where the above-described alkylene groups are present in a plural number, the above-described alkylene groups which are present in a plural number may be the same or different from each other.

The linking groups S may be continuously bonded to each other. However, it is preferable that groups selected from the group consisting of $*^A$-CO-$*^B$, $*^A$-O-CO-$*^B$, and $*^A$-O-$*^B$ are not continuously bonded to form "$*^A$-O-CO-O-$*^B$". In addition, it is preferable that groups selected from the group consisting of $*^A$-CO-$*^B$ and $*^A$-O-$*^B$ are not continuously bonded to form "$*^A$-O-CO-$*^B$" or "$*^A$-CO-O-$*^B$".

In the divalent linking group including one or more linking groups S, only one linking group S may be present, or two or more thereof may be present. In a case where the linking groups S are present in a plural number, the linking groups S which are present in a plural number may be the same or different from each other.

In this case, it is preferable that groups selected from the group consisting of $*^A$-CO-$*^B$, $*^A$-O-CO-$*^B$, and $*^A$-O-$*^B$ are not continuously bonded to form "$*^A$-O-CO-O-$*^B$". In addition, it is preferable that groups selected from the group consisting of $*^A$-CO-$*^B$ and $*^A$-O-$*^B$ are not continuously bonded to form "$*^A$-O-CO-$*^B$" or "$*^A$-CO-O-$*^B$".

However, in any case, a $\beta$-position atom relative to -$SO_3^-$ in L is not a carbon atom having a fluorine atom as a substituent.

In a case where the above-described $\beta$-position atom is a carbon atom, it is sufficient that the carbon atom is not directly substituted with a fluorine atom, and the carbon atom may have a substituent having a fluorine atom (for example, a fluoroalkyl group such as a trifluoromethyl group).

In other words, the above-described $\beta$-position atom is an atom in L, which is directly bonded to -C($R^1$)($R^2$)- in Formula (AN4).

Among these, it is preferable that L has only one linking group S.

That is, it is preferable that L represents a divalent linking group including a combination of one linking group S and an alkylene group which may have one or more substituents, or a divalent linking group including one linking group S.

For example, L is preferably a group represented by Formula (AN4-2).

$$*^a\text{-}(CR^{2a}_2)x\text{-}Q\text{-}(CR^{2b}_2)^{y}\text{-}*^b \qquad (AN4\text{-}2)$$

In Formula (AN4-2), $*^a$ represents a bonding position with $R^3$ in Formula (AN4).

$*^b$ represents a bonding position $-C(R^1)(R^2)-$ in Formula (AN4).
X and Y each independently represent an integer of 0 to 10, preferably an integer of 0 to 3.
$R^{2a}$ and $R^{2b}$ each independently represent a hydrogen atom or a substituent.

In a case where $R^{2a}$'s and $R^{2b}$'s are present in a plural number, the $R^{2a}$'s and $R^{2b}$'s which are present in a plural number may be the same or different from each other.
However, in a case where Y is 1 or more, $R^{2b}$ in $CR^{2b}_2$ which is directly bonded to $-C(R^1)(R^2)-$ in Formula (AN4) is not a fluorine atom.
Q represents $*^A$-O-CO-O-$*^B$, $*^A$-CO-$*^B$, $*^A$-CO-O-$*^B$, $*^A$-O-CO-$*^B$, $*^A$-O-$*^B$, $*^A$-S-$*^B$, or $*^A$-$SO_2$-$*^B$.
However, in a case where X + Y in Formula (AN4-2) is 1 or more and $R^{2a}$ and $R^{2b}$ in Formula (AN4-2) are all hydrogen atoms, Q represents $*^A$-O-CO-O-$*^B$, $*^A$-CO-$*^B$, $*^A$-O-CO-$*^B$ , $*^A$-O-$*^B$, $*^A$-S-$*^B$ , or $*^A$-$SO_2$-$*^B$.

$*^A$ represents a bonding position on the $R^3$ side in Formula (AN4), and *B represents a bonding position on the $-SO_3^-$ side in Formula (AN4).

In Formula (AN4), $R^3$ represents an organic group.
The above-described organic group is not limited as long as it has one or more carbon atoms, and may be a linear group (for example, a linear alkyl group) or a branched group (for example, a branched alkyl group such as a t-butyl group), and may be a cyclic group. The above-described organic group may or may not have a substituent. The above-described organic group may or may not have a heteroatom (oxygen atom, sulfur atom, nitrogen atom, and/or the like).
Among these, $R^3$ is preferably an organic group having a cyclic structure. The above-described cyclic structure may be monocyclic or polycyclic, and may have a substituent. The ring of the organic group including a cyclic structure is preferably directly bonded to L in Formula (AN4).
For example, the above-described organic group having a cyclic structure may or may not have a heteroatom (oxygen atom, sulfur atom, nitrogen atom, and/or the like). The heteroatom may be substituted on one or more carbon atoms forming the cyclic structure.
As the above-described organic group having a cyclic structure, for example, a hydrocarbon group having a cyclic structure, a lactone ring group, or a sultone ring group is preferable. Among these, the above-described organic group having a cyclic structure is preferably a hydrocarbon group having a cyclic structure.
The above-described hydrocarbon group having a cyclic structure is preferably a monocyclic or polycyclic cycloalkyl group. These groups may have a substituent.
The above-described cycloalkyl group may be a monocycle (cyclohexyl group or the like) or a polycycle (adamantyl group or the like), and the number of carbon atoms is preferably 5 to 12.
As the above-described lactone group and sultone group, for example, a group obtained by removing one hydrogen atom from ring member atoms constituting the lactone structure or the sultone structure in any of the structures represented by Formulae (LC1-1) to (LC1-21) described above and the structures represented by Formulae (SL1-1) to (SL1-3) described above is preferable.
The non-nucleophilic anion may be a benzenesulfonate anion, and is preferably a benzenesulfonate anion substituted with a branched alkyl group or a cycloalkyl group.
As the non-nucleophilic anion, an aromatic sulfonate anion represented by Formula (AN5) is also preferable.

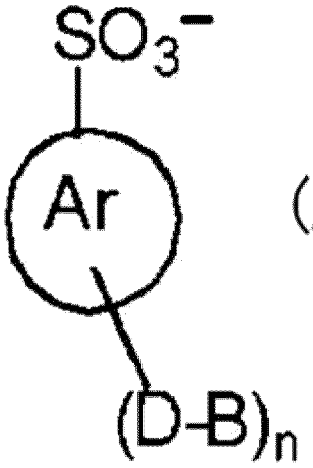

(AN5)

In Formula (AN5), Ar represents an aryl group (phenyl group or the like), and may further have a substituent other than a sulfonate anion and a -(D-B) group. Examples of the substituent which may be further included include a fluorine atom and a hydroxyl group.
n represents an integer of 0 or more. n is preferably 1 to 4, more preferably 2 or 3, and still more preferably 3.
D represents a single bond or a divalent linking group. Examples of the divalent linking group include -O-, a thioether group, a carbonyl group, a sulfoxide group, a sulfone group, sulfonic acid-COO-, -COO-, and a group consisting of a combination of two or more of these groups.
B represents a hydrocarbon group.
B preferably has an aliphatic hydrocarbon structure. B is more preferably an isopropyl group, a cyclohexyl group, or an aryl group which may further have a substituent (such as a tricyclohexylphenyl group).
As the non-nucleophilic anion, a disulfonamide anion is also preferable.
The disulfonamide anion is, for example, an anion represented by $N^-(SO_2-R^q)_2$.
Here, $R^q$ represents an alkyl group which may have a substituent, and is preferably a fluoroalkyl group and more preferably a perfluoroalkyl group. Two $R^q$'s may be bonded to each other to form a ring. The group formed by bonding two $R^q$'s to each other is preferably an alkylene group which may have a substituent, preferably a fluoroalkylene group, and still more preferably a perfluoroalkylene group. The number of carbon atoms in the above-described alkylene group is preferably 2 to 4.
The compound (III) may be in a form of a low-molecular-weight compound or a form incorporated into a part of a polymer. In addition, a combination of the form of a low-molecular-weight compound and the form incorporated into a part of a polymer may also be used.
In a case where the compound (III) is in the form of a low-molecular-weight compound, a molecular weight thereof is preferably 3,000 or less, more preferably 2,000 or less, and still more preferably 1,000 or less.
In a case where the compound (III) is in the form incorporated into a part of a polymer, it may be incorporated into the part of the acid-decomposable resin or into a resin which is different from the acid-decomposable resin.
The compound (III) is preferably in a form of a low-molecular-weight compound.
As the compound (III), for example, it is also preferable to use photoacid generators described in paragraphs [0135] to [0171] of WO2018/193954A, paragraphs [0077] to [0116] of WO2020/066824A, and paragraphs [0018] to [0075] and [0334] and [0335] of WO2017/154345A.
In a case where the resist composition contains the compound (III), a content thereof is preferably 0.5% by mass or more and more preferably 1% by mass or more with respect to the total solid content of the resist composition. The upper limit thereof is preferably 40% by mass or less and more preferably 30% by mass or less with respect to the total solid content of the resist composition.
The other photoacid generators may be used alone or in combination of two or more kinds thereof. In a case where two or more kinds thereof are used, a total content thereof is preferably within the suitable content range.

[Acid Diffusion Control Agent]

The resist composition may contain an acid diffusion control agent.
The acid diffusion control agent acts as a quencher which suppresses a reaction of an acid-decomposable resin in a non-exposed portion by excessive generated acids by trapping the acids generated from the photoacid generator and the like during exposure.
As the acid diffusion control agent, from the viewpoint that the LWR after aging storage is more excellent, a

basic compound having a nitrogen atom (a nitrogen-containing basic compound) is preferable.

Examples of the nitrogen-containing basic compound include a basic compound (DA), a basic compound (DB) (hereinafter, also simply referred to as "compound (DB)") in which basicity decreases or disappears by irradiation with actinic ray or radiation, a low-molecular-weight compound (DC) (hereinafter, also simply referred to as "compound DC") which has a nitrogen atom and has a group eliminated by action of acid, and an onium salt compound (DD) (hereinafter, also simply referred to as "compound DD") which has a nitrogen atom in a cationic moiety.

<Basic Compound (DA)>

As the basic compound (DA), a compound having a structure represented by Formulae (A) to (E) is preferable.

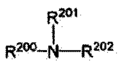 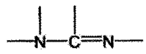 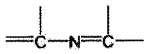 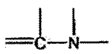 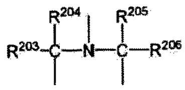

(A) (B) (C) (D) (E)

In Formula (A) and Formula (E), $R^{200}$, $R^{201}$, and $R^{202}$ may be the same or different from each other, and each independently represent a hydrogen atom, an alkyl group (preferably having 1 to 20 carbon atoms), a cycloalkyl group (preferably having 3 to 20 carbon atoms), or an aryl group (preferably having 6 to 20 carbon atoms). $R^{203}$ and $R^{202}$ may be bonded to each other to form a ring.

$R^{203}$, $R^{204}$, $R^{205}$, and $R^{206}$ may be the same or different from each other, and each independently represent an alkyl group having 1 to 20 carbon atoms.

The alkyl groups in Formulae (A) and (E) may have a substituent or may be unsubstituted.

The alkyl group having a substituent is preferably an aminoalkyl group having 1 to 20 carbon atoms, a hydroxyalkyl group having 1 to 20 carbon atoms, or a cyanoalkyl group having 1 to 20 carbon atoms.

The alkyl groups in Formulae (A) and (E) are more preferably unsubstituted.

As the basic compound (DA), guanidine, aminopyrrolidine, pyrazole, pyrazoline, piperazine, aminomorpholine, aminoalkylmorpholine, or piperidine is preferable; and a compound having an imidazole structure, a diazabicyclo structure, an onium hydroxide structure, an onium carboxylate structure, a trialkylamine structure, an aniline structure, or a pyridine structure, an alkylamine derivative having a hydroxyl group and/or an ether group, or an aniline derivative having a hydroxyl group and/or an ether group is more preferable.

<Compound (DB)>

The compound (DB) is a compound that has a proton-accepting functional group and is decomposed by irradiation with actinic ray or radiation to exhibit deterioration in proton-accepting properties, exhibit no proton-accepting properties, or change from proton-accepting properties to acidic properties.

The proton-accepting functional group refers to a functional group having a group or electron capable of electrostatically interacting with a proton, and for example, means a functional group with a macrocyclic structure, such as a cyclic polyether, and/or a functional group having a nitrogen atom having an unshared electron pair not contributing to $\pi$-conjugation. The nitrogen atom having the unshared electron pair, which does not contribute to the $\pi$-conjugation, is as described above.

As the partial structure of the proton-accepting functional group, a crown ether, azacrown ether, primary to tertiary amine, pyridine, imidazole, or pyrazine structure is preferable.

The compound (DB) is decomposed by irradiation with actinic ray or radiation to generate a compound exhibiting deterioration in proton-accepting properties, no proton-accepting properties, or a change from the proton-accepting properties to acidic properties. Here, the decrease or disappearance of proton-accepting properties, or the change from proton-accepting properties to acid property is a change in proton-accepting properties due to the proton being added to the proton-accepting functional group, and specifically, in a case where a proton adduct is generated from the compound (DB) having the proton-accepting functional group and the proton, the equilibrium constant in chemical equilibrium thereof decreases.

The proton-accepting properties can be confirmed by measuring pH.

A pKa of a compound generated by decomposition of the compound (DB) by the irradiation with actinic ray or radiation is preferably pKa < -1, more preferably -13 < pKa < -1, and still more preferably -13 < pKa < -3. The compound thus generated may be subjected to intramolecular neutralization to have a pKa of -1 or more.

The compound (DB) is preferably a compound represented by Formula (b-1).

$$R-B-X-A-W_1-N^{-}-W_2-R_f[C^+] \qquad (b-1)$$

In Formula (b-1),

$W_1$ and $W_2$ each independently represent $-SO_2-$ or -CO-,
$R_f$ represents an alkyl group which may have a substituent, a cycloalkyl group which may have a substituent, or an aryl group which may have a substituent,
A represents a single bond or a divalent linking group,
X represents a single bond, $-SO_2-$, or -CO-,
B represents a single bond, an oxygen atom, or $-N(R_x)R_y-$,
$R_x$ represents a hydrogen atom or an organic group,
$R_y$ represents a single bond or a divalent organic group,
R represents a monovalent organic group having a proton-accepting functional group,
$R_x$ may be bonded to $R_y$ to form a ring, or may be bonded to R to form a ring, and $[C^+]$ represents a counter cation.

It is preferable that least one of $W_1$ or $W_2$ is $-SO_2-$, and it is preferable that both are $-SO_2-$.
Rf is preferably an alkyl group having 1 to 6 carbon atoms, which may have a fluorine atom, more preferably a perfluoroalkyl group having 1 to 6 carbon atoms, and still more preferably a perfluoroalkyl group having 1 to 3 carbon atoms.
As the divalent linking group in A, a divalent linking group having 2 to 12 carbon atoms is preferable, and examples thereof include an alkylene group and a phenylene group. Among these, an alkylene group having at least one fluorine atom is preferable. The number of carbon atoms in the above-described alkylene group is preferably 2 to 6 and more preferably 2 to 4. A linking group such as an oxygen atom and a sulfur atom may be included in alkylene groups. It is preferable that the alkylene group is an alkylene group in which 30% to 100% of the number of hydrogen atoms is substituted with a fluorine atom, and it is more preferable that the carbon atom bonded to the Q site has a fluorine atom. As the divalent linking group in A, a perfluoroalkylene group is preferable, and a perfluoroethylene group, a perfluoropropylene group, or a perfluorobutylene group is more preferable.
The monovalent organic group in Rx preferably has 2 to 30 carbon atoms, and examples thereof include an alkyl group, a cycloalkyl group which may have an oxygen atom in the ring, an aryl group, an aralkyl group, and an alkenyl group.
The alkyl group in Rx may have a substituent, and is preferably a linear or branched alkyl group having 1 to 20 carbon atoms. An oxygen atom, a sulfur atom, and/or a nitrogen atom may be included in the above-described alkyl chain.
Examples of the alkyl group having a substituent include a group in which a linear or branched alkyl group is substituted with a cycloalkyl group (for example, an adamantylmethyl group, an adamantylethyl group, a cyclohexylethyl group, and a camphor residue).
The cycloalkyl group in Rx may have a substituent, and is preferably a cycloalkyl group having 3 to 20 carbon atoms. In addition, an oxygen atom may be included in the ring of the cycloalkyl group.
The aryl group in Rx may have a substituent. The number of carbon atoms in the above-described aryl group is preferably 6 to 14.
The aralkyl group in Rx may have a substituent. The number of carbon atoms in the above-described aralkyl group is preferably 7 to 20.
The alkenyl group in Rx may have a substituent. Examples of the above-described alkenyl group include a group having a double bond at any position of the alkyl group mentioned as Rx.
In a case where B represents -N(Rx)Ry-, the divalent organic group in Ry is preferably an alkylene group. In addition, in this case, as a ring which can be formed by bonding Rx and Ry to each other, a 5- to 8-membered ring containing a nitrogen atom is preferable, and a 6-membered ring is more preferable. The nitrogen atom included in the ring may be a nitrogen atom other than the nitrogen atom directly bonded to X in -N(Rx)Ry-.
In a case where B represents -N(Rx)Ry-, it is preferable that R and Rx are bonded to each other to form a ring. By forming the ring, stability is improved, and storage stability of a composition using this is improved. The number of carbon atoms forming the ring is preferably 4 to 20, the ring may be a monocycle or a polycycle, and the ring may include an oxygen atom, a sulfur atom, and/or a nitrogen atom. The nitrogen atom included in the ring may be a nitrogen atom other than the nitrogen atom directly bonded to X in -N(Rx)Ry-.
Examples of the monocycle include a 4-membered ring, 5-membered ring, 6-membered ring, 7-membered ring, or 8-membered ring including a nitrogen atom. Examples of such a ring structure include a piperazine ring and a piperidine ring. Examples of the polycycle include a structure formed by a combination of two or three or more monocyclic structures. Each of the monocycle and the polycycle may have a substituent. As the above-described substituent, for example, a halogen atom, a hydroxyl group, a cyano group, a carboxy group, a carbonyl group, a cycloalkyl group (preferably having 3 to 10 carbon atoms), an aryl group (preferably having 6 to 14 carbon atoms), an alkoxy group

(preferably having 1 to 10 carbon atoms), an acyl group (preferably having 2 to 15 carbon atoms), an acyloxy group (preferably having 2 to 15 carbon atoms), an alkoxycarbonyl group (preferably having 2 to 15 carbon atoms), or an aminoacyl group (preferably having 2 to 20 carbon atoms) is preferable. If possible, these substituents may further have a substituent. Examples of a case where the aryl group and the cycloalkyl group further have a substituent include an alkyl group (preferably having 1 to 15 carbon atoms). Examples of a substituent further included in the aminoacyl group include an alkyl group (preferably having 1 to 15 carbon atoms).

The proton-accepting functional group in R is as described above, and as a partial structure thereof, for example, it is preferable to have crown ether, primary to tertiary amine, or a structure of a nitrogen-containing hetero ring (for example, pyridine, imidazole, pyrazine, and the like).

As the proton-accepting functional group, a functional group having a nitrogen atom is preferable, and a group having a primary to tertiary amino group or a nitrogen-containing heterocyclic group is more preferable. In these structures, it is preferable that all atoms adjacent to the nitrogen atom included in the structure are carbon atoms or hydrogen atoms. In addition, it is preferable that an electron-withdrawing functional group (a carbonyl group, a sulfonyl group, a cyano group, a halogen atom, or the like) is not directly connected to the nitrogen atom.

The monovalent organic group in such a monovalent organic group (group R) including the proton-accepting functional group preferably has 2 to 30 carbon atoms, and examples thereof include an alkyl group, a cycloalkyl group, an aryl group, an aralkyl group, and an alkenyl group, in which each group may have a substituent.

Examples of the alkyl group, the cycloalkyl group, the aryl group, the aralkyl group, and the alkenyl group in the alkyl group, the cycloalkyl group, the aryl group, the aralkyl group, and the alkenyl group, which are included in the proton-accepting functional group in R, include the same groups as the alkyl group, the cycloalkyl group, the aryl group, the aralkyl group, and the alkenyl group, which are exemplified as Rx.

Examples of the substituent which may be included in each group described above include a halogen atom, a hydroxyl group, a nitro group, a cyano group, a carboxy group, a carbonyl group, a cycloalkyl group (preferably having 3 to 10 carbon atoms; which may be partially substituted with a heteroatom or a group having a heteroatom (an ester group and the like)), an aryl group (preferably having 6 to 14 carbon atoms), an alkoxy group (preferably having 1 to 10 carbon atoms), an acyl group (preferably having 2 to 20 carbon atoms), an acyloxy group (preferably having 2 to 10 carbon atoms), an alkoxycarbonyl group (preferably having 2 to 20 carbon atoms), and an aminoacyl group (preferably having 2 to 20 carbon atoms). Examples of the substituent included in the cyclic group of the aryl group, the cycloalkyl group, and the like include an alkyl group (preferably having 1 to 20 carbon atoms). Examples of a substituent included in the aminoacyl group include an alkyl group (preferably having 1 to 20 carbon atoms).

The counter cation of [$C^+$] is preferably a sulfonium cation or an iodonium cation. As the sulfonium cation and the iodonium cation, for example, the sulfonium cation and the iodonium cation in the cation which may be included in the photoacid generator (specifically, the cation in the compound represented by Formula (ZaI) and the cation in the compound represented by Formula (ZaII)) can be used in the same manner.

<Compound (DC)>

The nitrogen-containing compound may be a low-molecular-weight compound (DC) (hereinafter, also referred to as "compound (DC)") which has a nitrogen atom and has a group eliminated by action of acid. The compound (DC) is preferably an amine derivative having, on the nitrogen atom, a group which is eliminated by the action of acid.

The group which is eliminated by action of acid is preferably an acetal group, a carbonate group, a carbamate group, a tertiary ester group, a tertiary hydroxyl group, or a hemiaminal ether group, and more preferably a carbamate group or a hemiaminal ether group.

A molecular weight of the compound (DC) is preferably 100 to 1,000, more preferably 100 to 700, and still more preferably 100 to 500.

The compound (DC) may have a carbamate group having a protective group on a nitrogen atom. The protective group constituting the carbamate group is preferably a group represented by Formula (c-1).

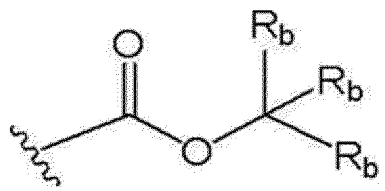
(c-1)

In Formula (c-1), Rb's each independently represent a hydrogen atom, an alkyl group (preferably having 1 to 10 carbon atoms), a cycloalkyl group (preferably having 3 to 30 carbon atoms), an aryl group (preferably having 3 to 30

carbon atoms), an aralkyl group (preferably having 1 to 10 carbon atoms), or an alkoxyalkyl group (preferably having 1 to 10 carbon atoms). Rb's may be linked to each other to form a ring.

The alkyl group, the cycloalkyl group, the aryl group, and the aralkyl group described above may be each independently substituted with a functional group such as a hydroxy group, a cyano group, an amino group, a pyrrolidino group, a piperidino group, a morpholino group, and an oxo group, an alkoxy group, or a halogen atom. The same applies to the alkoxyalkyl group represented by Rb.

Rb is preferably a linear or branched alkyl group, a cycloalkyl group, or an aryl group, and more preferably a linear or branched alkyl group or a cycloalkyl group.

Examples of the ring formed by linking two Rb's to each other include an alicyclic hydrocarbon, an aromatic hydrocarbon, a heterocyclic hydrocarbon, and a derivative thereof.

Examples of a specific structure of the group represented by Formula (c-1) include a structure described in paragraph [0466] of US2012/0135348A1.

It is preferable that the compound (DC) is a compound having a structure represented by Formula (6).

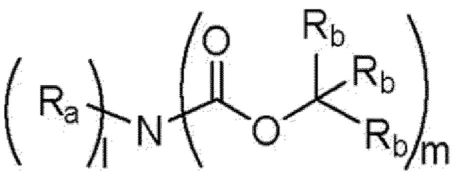 (6)

In Formula (6), 1 represents an integer of 0 to 2, m represents an integer of 1 to 3, and 1 + m satisfies 3. Ra represents a hydrogen atom, an alkyl group, a cycloalkyl group, an aryl group, or an aralkyl group. In a case where 1 is 2, two Ra's may be the same or different from each other, and two Ra's may be linked to each other to form a hetero ring with the nitrogen atom in the formula. The hetero ring may include a heteroatom other than the nitrogen atom in the formula.

Rb has the same meaning as Rb in Formula (c-1) described above, and a suitable aspect thereof is also the same.

The alkyl group, the cycloalkyl group, the aryl group, and the aralkyl group as Ra may be each independently substituted with a group same as the above-described group which may be substituted on the alkyl group, the cycloalkyl group, the aryl group, and the aralkyl group as Rb.

Examples of the alkyl group, the cycloalkyl group, the aryl group, and the aralkyl group (these groups may be substituted with the above-described group) of Ra include a group same as in the specific examples described above for Rb.

<Compound (DD)>

The compound (DD) is an onium salt compound having a nitrogen atom in a cationic moiety.

However, in a case where the compound (DD) generates an acid by irradiation with actinic ray or radiation, a pKa of the generated acid is smaller than a value obtained by adding 1.00 to the pKa of the acid generated from the photoacid generator A.

The compound (DD) is preferably a compound having, in the cationic moiety, a basic site containing a nitrogen atom.

The basic site is preferably an amino group and more preferably an aliphatic amino group. In addition, it is preferable that all of atoms adjacent to the nitrogen atom in the basic site are hydrogen atoms or carbon atoms. In addition, from the viewpoint of improving basicity, it is preferable that an electron-withdrawing functional group (a carbonyl group, a sulfonyl group, a cyano group, a halogen atom, or the like) is not directly connected to the nitrogen atom.

In addition, as the acid diffusion control agent, an onium salt which is a relatively weak acid to a photoacid-generating component (including the specific photoacid generator and the other photoacid generators) can also be used.

In a case of being used in a form of coexistence of the photoacid generator and the onium salt that generates an acid which is relatively weak with respect to the acid generated from the photoacid-generating component, the acid generated from the photoacid-generating component due to the irradiation with actinic ray or radiation collides with an onium salt having an unreacted weak-acid anion, so that salt exchange releases the weak acid to yield an onium salt with a strong-acid anion. In this process, since the strong acid is exchanged for the weak acid having a lower catalytic activity, the acid is apparently inactivated and the acid diffusion can be controlled.

As the onium salt which is a relatively weak acid to the photoacid-generating component, compounds represented by Formulae (d1-1) to (d1-3) are preferable.

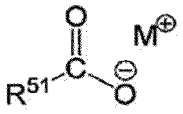

(d 1 − 1)

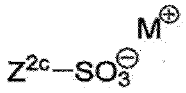

(d 1 − 2)

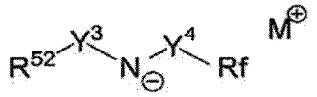

(d 1 − 3)

In Formula (d1-1), $R^{51}$ represents an organic group. The number of carbon atoms in the above-described organic group is preferably 1 to 30.

$Z^{2c}$ represents an organic group. The number of carbon atoms in the above-described organic group is preferably 1 to 30. However, in a case where the organic group represented by $Z^{2c}$ has a carbon atom adjacent to $SO^{3-}$ specified in the formula, this carbon atom ($\alpha$-carbon atom) does not have a fluorine atom and/or a perfluoroalkyl group as a substituent. The above-described $\alpha$-carbon atom is not a ring member atom of the cyclic structure, and it is preferably a methylene group. In addition, in a case where a $\beta$-position atom in $Z^{2c}$ relative to $SO_3^-$ is a carbon atom ($\beta$-carbon atom), the $\beta$-carbon atom also does not have a fluorine atom and/or a perfluoroalkyl group as a substituent.

$R^{52}$ represents an organic group (an alkyl group and the like), $Y^3$ represents $-SO_2-$, a linear, branched, or cyclic alkylene group, or an arylene group, $Y^4$ represents -CO- or $-SO_2-$, and Rf represents a hydrocarbon group having a fluorine atom (a fluoroalkyl group and the like).

$M^+$'s each independently represent an ammonium cation, a sulfonium cation, or an iodonium cation. Examples of $M^+$ in Formulae (d1-1) to (d1-3) include the above-described organic cations (for example, the organic cation represented by $M_{11}^+$ in Formula (Ia-1) described above).

As one aspect, these cations preferably have an acid-decomposable group. The acid-decomposable group is as described above.

A zwitterion may be used as the acid diffusion control agent. The acid diffusion control agent which is a zwitterion preferably has a carboxylate anion, and more preferably further has a sulfonium cation or an iodonium cation.

In the resist composition according to the embodiment of the present invention, a known acid diffusion control agent can be appropriately used. For example, as the acid diffusion control agent, known compounds described in paragraphs [0627] to [0664] of US2016/0070167A1, paragraphs [0095] to [0187] of US2015/0004544A1, paragraphs [0403] to [0423] of US2016/0237190A1, and paragraphs [0259] to [0328] of US2016/0274458A1 can be suitably used.

In a case where the resist composition contains the acid diffusion control agent, a content of the acid diffusion control agent (in a case of a plurality of types, the total thereof) is preferably 0.1% to 20.0% by mass, more preferably 0.1% to 15.0% by mass, still more preferably 0.1% to 10.0% by mass, and particularly preferably 1.0% to 10.0% by mass with respect to the total solid content of the resist composition.

The acid diffusion control agent may be used alone or in combination of two or more kinds thereof.

[Hydrophobic Resin]

The resist composition may contain a hydrophobic resin different from the acid-decomposable resin, in addition to the above-described acid-decomposable resin.

It is preferable that the hydrophobic resin is designed to be unevenly distributed on a surface of the resist film, and it is not necessary to have a hydrophilic group in the molecule as different from a surfactant, and is not necessary to contribute to uniform mixing of polar materials and non-polar materials.

Examples of an effect caused by the addition of the hydrophobic resin include a control of static and dynamic contact angles of a surface of the resist film with respect to water and suppression of outgas.

From the viewpoint of uneven distribution on the film surface layer, the hydrophobic resin preferably has any one or more of a fluorine atom, a silicon atom, and a $CH_3$ partial structure which is included in a side chain moiety of a resin, and more preferably has two or more kinds thereof. In addition, the above-described hydrophobic resin preferably has a hydrocarbon group having 5 or more carbon atoms. These groups may be included in the main chain of the resin or may be substituted in the side chain of the resin.

Examples of the hydrophobic resin include compounds described in paragraphs [0275] to [0279] of WO2020/004306A.

In a case where the resist composition contains the hydrophobic resin, a content thereof is preferably 0.01% to 20% by mass, more preferably 0.1% to 15% by mass, still more preferably 0.1% to 10% by mass, and particularly preferably 0.1% to 8.0% by mass with respect to the total solid content of the resist composition.

The hydrophobic resin may be used alone or in combination of two or more kinds thereof. In a case where two or more kinds thereof are used, a total content thereof is preferably within the suitable content range.

[Surfactant]

The resist composition may contain a surfactant.

In a case where the surfactant is contained, it is possible to form a pattern having more excellent adhesiveness and fewer development defects.

The surfactant is preferably a fluorine-based and/or silicon-based surfactant.

As the fluorine-based and/or silicon-based surfactant, for example, surfactants described in paragraphs [0218] and [0219] of WO2018/19395A can be used.

In a case where the resist composition contains a surfactant, a content thereof is preferably 0.0001% to 2% by mass, and more preferably 0.0005% to 1% by mass with respect to the total solid content of the resist composition.

The surfactant may be used alone or in combination of two or more kinds thereof. In a case where two or more kinds thereof are used, a total content thereof is preferably within the suitable content range.

[Solvent]

The resist composition may contain a solvent.

The solvent preferably includes at least one solvent of (M1) propylene glycol monoalkyl ether carboxylate or (M2) at least one selected from the group consisting of propylene glycol monoalkyl ether, lactic acid ester, acetic acid ester, alkoxypropionic acid ester, chain ketone, cyclic ketone, lactone, and alkylene carbonate. The solvent may further include a component other than the components (M1) and (M2).

The present inventors have found that, by using such a solvent and the above-described resin in combination, a pattern having a small number of development defects can be formed while improving coating property of the composition.

A reason for this is not always clear, but the present inventors have considered that, since these solvents have a good balance of solubility, boiling point, and viscosity of the above-described resin, unevenness of a film thickness of a composition film, generation of precipitates during spin coating, and the like can be suppressed.

Details of the component (M1) and the component (M2) are described in paragraphs [0218] to [0226] of WO2020/004306A.

In a case where the solvent further contains a component other than the components (M1) and (M2), a content of the component other than the components (M1) and (M2) is preferably 5% to 30% by mass with respect to the total amount of the solvent.

A content of the solvent in the resist composition is preferably set such that a concentration of solid contents is 30% by mass or less, more preferably set such that a concentration of solid contents is 10% by mass or less, and still more preferably set such that a concentration of solid contents is 2% by mass or less. The lower limit thereof is preferably set such that the concentration of solid contents is 0.05% by mass or more, more preferably set such that the concentration of solid contents is 0.1% by mass or more, and still more preferably set such that the concentration of solid contents is 0.5% by mass or more. In a case where the content thereof is within the above-described range, coating property of the resist composition can be further improved.

The content of the solvent is preferably 70% to 99.95% by mass, more preferably 90% to 99.9% by mass, and still more preferably 98% to 99.5% by mass with respect to the total mass of the resist composition.

The solvent may be used alone or in combination of two or more kinds thereof. In a case where two or more kinds thereof are used, a total content thereof is preferably within the suitable content range.

[Other Additives]

The resist composition may further contain a dissolution inhibiting compound, a dye, a plasticizer, a photosensitizer, a light absorbing agent, and/or a compound promoting a solubility in a developer (an alicyclic or aliphatic compound including a carboxylic acid group).

The resist composition may further contain a dissolution inhibiting compound. Here, the "dissolution inhibiting compound" is intended to be a compound having a molecular weight of 3000 or less, in which solubility in an organic developer decreases by decomposition due to action of acid.

The resist composition according to the embodiment of the present invention is also suitably used as a photosensitive composition for EUV light.

Since the EUV light has a wavelength of 13.5 nm and has a shorter wavelength than that of ArF (wavelength: 193 nm), the number of incident photons in a case of being exposed with the same sensitivity is small. Therefore, influence of "photon shot noise" in which the number of photons varies probabilistically is large, which causes deterioration of LER and bridge defects. In order to reduce the photon shot noise, there is a method of increasing the number of incident photons by increasing an exposure amount, but there is a trade-off with the demand for higher sensitivity.

In a case where an A value obtained by Expression (1) is high, absorption efficiency of EUV light and electron beams of the resist film formed from the resist composition is high, which is effective in reducing the photon shot noise. The A value represents the absorption efficiency of EUV light and electron beams of the resist film in terms of a mass proportion.

$$\text{Expression (1): } A = ([H] \times 0.04 + [C] \times 1.0 + [N] \times 2.1 + [O] \times 3.6 + [F] \times 5.6 + [S] \times 1.5 + [I] \times 39.5)/([H] \times 1 + [C] \times 12 + [N] \times 14 + [O] \times 16 + [F] \times 19 + [S] \times 32 + [I] \times 127)$$

The A value is preferably 0.120 or more. In a case where the A value is extremely high, the transmittance of EUV light and electron beams of the resist film is lowered and the optical image profile in the resist film is deteriorated, which results in difficulty in obtaining a good pattern shape, so that the upper limit is preferably 0.240 or less, and more preferably 0.220 or less.

In Expression (1), [H] represents a molar ratio of hydrogen atoms derived from a total solid content with respect to all atoms of the total solid content in the actinic ray-sensitive or radiation-sensitive resin composition, [C] represents a molar ratio of carbon atoms derived from the total solid content with respect to all atoms of the total solid content in the actinic ray-sensitive or radiation-sensitive resin composition, [N] represents a molar ratio of nitrogen atoms derived from the total solid content with respect to all atoms of the total solid content in the actinic ray-sensitive or radiation-sensitive resin composition, [O] represents a molar ratio of oxygen atoms derived from the total solid content with respect to all atoms of the total solid content in the actinic ray-sensitive or radiation-sensitive resin composition, [F] represents a molar ratio of fluorine atoms derived from the total solid content with respect to all atoms of the total solid content in the actinic ray-sensitive or radiation-sensitive resin composition, [S] represents a molar ratio of sulfur atoms derived from the total solid content with respect to all atoms of the total solid content in the actinic ray-sensitive or radiation-sensitive resin composition, and [I] represents a molar ratio of iodine atoms derived from the total solid content with respect to all atoms of the total solid content in the actinic ray-sensitive or radiation-sensitive resin composition.

For example, in a case where the resist composition contains the acid-decomposable resin, the compound (1), and the solvent, the acid-decomposable resin and the compound (1) correspond to the solid content. That is, all atoms of the total solid content correspond to a sum of all atoms derived from the acid-decomposable resin and all atoms derived from the compound (1). For example, [H] represents a molar ratio of hydrogen atoms derived from the total solid content with respect to all atoms in the total solid content, and by way of description based on the example above, [H] represents a molar ratio of a sum of hydrogen atoms derived from the acid-decomposable resin and hydrogen atoms derived from the compound (1) with respect to the sum of all atoms derived from the acid-decomposable resin and all atoms derived from the compound (1).

The A value can be calculated by computation of the structure of constituent components of the total solid content in the resist composition, and the ratio of the number of atoms contained in a case where the content is already known. In addition, even in a case where the constituent component is not known yet, it is possible to calculate a ratio of the number of constituent atoms by subjecting a resist film obtained after evaporating the solvent components of the resist composition to computation according to an analytic approach such as elemental analysis.

[Resist Film and Pattern Forming Method]

A procedure of the pattern forming method using the above-described resist composition preferably has the following steps.

Step 1: step of forming a resist film on a substrate using the resist composition
Step 2: step of exposing the resist film
Step 3: step of developing the exposed resist film using a developer

Hereinafter, the procedure of each of the above-described steps will be described in detail.

<Step 1: Resist Film Forming Step>

The step 1 is a step of forming a resist film on a substrate using the resist composition.

The definition of the resist composition is as described above.

Examples of a method for forming a resist film on a substrate using the resist composition include a method in which a resist composition is applied to a substrate.

In addition, it is preferable that the resist composition before the application is filtered through a filter, as desired.

A pore size of the filter is preferably 0.1 μm or less, more preferably 0.05 μm or less, and still more preferably 0.03 μm or less. In addition, the filter is preferably a polytetrafluoroethylene-made filter, a polyethylene-made filter, or a nylon-made filter.

The resist composition can be applied to a substrate (for example, silicon and silicon dioxide coating) as used in the manufacture of integrated circuit elements by a suitable application method such as an application using a spinner or a coater. The coating method is preferably a spin coating using a spinner. A rotation speed upon the spin coating using a spinner is preferably 1000 to 3000 rpm.

After the application of the resist composition, the substrate may be dried to form a resist film. In addition, various underlying films (an inorganic film, an organic film, or an antireflection film) may be formed on an underlayer of the resist film.

Examples of the drying method include a method of heating and drying. The heating can be carried out using a unit included in an ordinary exposure machine and/or development machine, and may also be carried out using a hot plate or the like. A heating temperature is preferably 80°C to 150°C, more preferably 80°C to 140°C, and still more preferably 80°C to 130°C. A heating time is preferably 30 to 1000 seconds, more preferably 60 to 800 seconds, and still more preferably 60 to 600 seconds.

From the viewpoint that a fine pattern having higher accuracy can be formed, a film thickness of the resist film is preferably 10 to 120 nm. Among these, in a case of performing EUV exposure, the film thickness of the resist film is more preferably 10 to 65 nm and still more preferably 15 to 50 nm.

A topcoat may be formed on an upper layer of the resist film using a topcoat composition.

It is preferable that the topcoat composition is not mixed with the resist film and can be uniformly applied to the upper layer of the resist film. The topcoat is not particularly limited, a topcoat known in the related art can be formed by the methods known in the related art, and for example, the topcoat can be formed based on the description in paragraphs [0072] to [0082] of JP2014-059543A.

For example, it is preferable that a topcoat including a basic compound as described in JP2013-61648A is formed on the resist film. Specific examples of the basic compound which can be included in the topcoat include a basic compound which may be included in the resist composition.

In addition, it is also preferable that the topcoat includes a compound which includes at least one group or bond selected from the group consisting of an ether bond, a thioether bond, a hydroxyl group, a thiol group, a carbonyl bond, and an ester bond.

<Step 2: Exposing Step>

The step 2 is a step of exposing the resist film.

Examples of an exposing method include a method in which the formed resist film is irradiated with actinic ray or radiation through a predetermined mask.

Examples of the actinic ray or radiation include infrared light, visible light, ultraviolet light, far ultraviolet light, extreme ultraviolet light, X-rays, and electron beam.

Far ultraviolet light is preferable as a wavelength of the actinic ray or radiation. The wavelength of the far ultraviolet light is preferably 250 nm or less, more preferably 220 nm or less, and still more preferably 1 to 200 nm. Specific examples thereof include a KrF excimer laser (248 nm), an ArF excimer laser (193 nm), an $F_2$ excimer laser (157 nm), EUV light (13 nm), X-rays, and electron beams.

It is preferable to perform baking (heating) before performing development and after the exposure. The baking accelerates a reaction in the exposed portion, and the sensitivity and the pattern shape are improved.

A heating temperature is preferably 80°C to 150°C, more preferably 80°C to 140°C, and still more preferably 80°C to 130°C.

A heating time is preferably 10 to 1000 seconds, more preferably 10 to 180 seconds, and still more preferably 30 to 120 seconds.

The heating can be carried out using a unit included in an ordinary exposure machine and/or development machine, and may also be performed using a hot plate or the like.

This step is also referred to as post exposure bake (PEB).

<Step 3: Developing Step>

The step 3 is a step of developing the exposed resist film using a developer to form a pattern.

The developer may be either an alkali developer or a developer including an organic solvent (hereinafter, also referred to as "organic developer").

Examples of a developing method include a method in which the substrate is immersed in a tank filled with a developer for a certain period of time (a dipping method), a method in which a development is performed by heaping a

developer up onto the surface of the substrate by surface tension, and then leaving it to stand for a certain period of time (a puddle method), a method in which a developer is sprayed on the surface of the substrate (a spraying method), and a method in which a developer is continuously jetted onto the substrate rotating at a constant rate while scanning a developer jetting nozzle at a constant rate (a dynamic dispensing method).

In addition, after the step of performing the development, a step of stopping the development may be carried out while replacing the solvent with another solvent.

A developing time is not particularly limited as long as it is a period of time where the non-exposed portion of the resin is sufficiently dissolved, and is preferably 10 to 300 seconds and more preferably 20 to 120 seconds.

A temperature of the developer is preferably 0°C to 50°C and more preferably 15°C to 35°C.

As the alkali developer, it is preferable to use an aqueous alkali solution including an alkali. Examples of the type of the aqueous alkali solution include an aqueous alkali solution including a quaternary ammonium salt typified by tetramethylammonium hydroxide, an inorganic alkali, a primary amine, a secondary amine, a tertiary amine, an alcoholamine, a cyclic amine, or the like. Among these, the alkali developer is preferably aqueous solutions of the quaternary ammonium salts typified by tetramethylammonium hydroxide (TMAH). An appropriate amount of alcohols, a surfactant, or the like may be added to the alkali developer. An alkali concentration of the alkali developer is usually 0.1% to 20% by mass. In addition, a pH of the alkali developer is usually 10.0 to 15.0. A content of water in the alkali developer is preferably 51% to 99.95% by mass.

The organic developer is preferably a developer containing at least one organic solvent selected from the group consisting of a ketone-based solvent, an ester-based solvent, an alcohol-based solvent, an amide-based solvent, an ether-based solvent, and a hydrocarbon-based solvent.

A plurality of the above-described solvents may be mixed, or the solvent may be used in admixture with a solvent other than those described above or water. With respect to the total mass of the developer, a moisture content in the entire developer is preferably less than 50% by mass, more preferably less than 20% by mass, and still more preferably less than 10% by mass, and it is particularly preferable that the entire developer contains substantially no water.

A content of the organic solvent with respect to the organic developer is preferably 50% to 100% by mass, more preferably 80% to 100% by mass, still more preferably 90% to 100% by mass, and particularly preferably 95% to 100% by mass with respect to the total mass of the developer.

<Other Steps>

It is preferable that the above-described pattern forming method includes a step of performing washing using a rinsing liquid after the step 3.

Examples of the rinsing liquid used in the rinsing step after the step of performing development using an alkali developer include pure water. An appropriate amount of a surfactant may be added to the pure water.

An appropriate amount of a surfactant may be added to the rinsing liquid.

The rinsing liquid used in the rinsing step after the developing step with an organic developer is not particularly limited as long as the rinsing liquid does not dissolve the pattern, and a solution including a common organic solvent can be used. As the rinsing liquid, a rinsing liquid containing at least one organic solvent selected from the group consisting of a hydrocarbon-based solvent, a ketone-based solvent, an ester-based solvent, an alcohol-based solvent, an amide-based solvent, and an ether-based solvent is preferably used.

A method for the rinsing step is not particularly limited, and examples thereof include a method in which the rinsing liquid is continuously jetted onto the substrate rotated at a constant rate (a spin coating method), a method in which the substrate is immersed in a tank filled with the rinsing liquid for a certain period of time (a dipping method), and a method in which the rinsing liquid is sprayed on the surface of the substrate (a spraying method).

In addition, the pattern forming method according to the embodiment of the present invention may include a heating step (postbaking) after the rinsing step. By this step, the developer and the rinsing liquid remaining between and inside the patterns are removed by baking. In addition, this step also has an effect that a resist pattern is annealed and the surface roughness of the pattern is improved. The heating step after the rinsing step is usually performed at 40°C to 250°C (preferably 90°C to 200°C) for usually 10 seconds to 3 minutes (preferably 30 seconds to 2 minutes).

In addition, an etching treatment on the substrate may be carried out using the formed pattern as a mask. That is, the substrate (or the underlayer film and the substrate) may be processed using the pattern formed in the step 3 as a mask to form a pattern on the substrate.

A method for processing the substrate (or the underlayer film and the substrate) is not particularly limited, but a method in which a pattern is formed on a substrate by subjecting the substrate (or the underlayer film and the substrate) to dry etching using the pattern formed in the step 3 as a mask is preferable. Oxygen plasma etching is preferable as the dry etching.

It is preferable that various materials (for example, the solvent, the developer, the rinsing liquid, a composition for forming the antireflection film, a composition for forming the topcoat, and the like) used in the resist composition and

the pattern forming method according to the embodiment of the present invention do not include impurities such as metals. A content of the impurities included in these materials is preferably 1 ppm by mass or less, more preferably 10 ppb by mass or less, still more preferably 100 parts per trillion (ppt) by mass or less, particularly preferably 10 ppt by mass or less, and most preferably 1 ppt by mass or less with respect to the total solid content of the resist composition or the various materials. Here, examples of the metal impurities include Na, K, Ca, Fe, Cu, Mg, Al, Li, Cr, Ni, Sn, Ag, As, Au, Ba, Cd, Co, Pb, Ti, V, W, and Zn.

Examples of a method for removing the impurities such as metals from the various materials include filtration using a filter. Details of the filtration using a filter are described in paragraph [0321] of WO2020/004306A.

In addition, examples of a method for reducing the impurities such as metals included in the various materials include a method of selecting raw materials having a low content of metals as raw materials constituting the various materials, a method of subjecting raw materials constituting the various materials to filter filtration, and a method of performing distillation under the condition for suppressing the contamination as much as possible by, for example, lining the inside of a device with TEFLON (registered trademark).

In addition to the filter filtration, removal of the impurities by an adsorbing material may be performed, or a combination of filter filtration and an adsorbing material may be used. As the adsorbing material, known adsorbing materials can be used, and for example, inorganic adsorbing materials such as silica gel and zeolite and organic adsorbing materials such as activated carbon can be used. It is necessary to prevent the incorporation of impurities such as metals in the production process in order to reduce the metal impurities included in the above-described various materials. Sufficient removal of the metal impurities from a production device can be confirmed by measuring the content of metal components included in a washing solution used to wash the production device. A content of the metal components included in the washing solution after the use is preferably 100 parts per trillion (ppt) by mass or less, more preferably 10 ppt by mass or less, and still more preferably 1 ppt by mass or less.

A conductive compound may be added to an organic treatment liquid such as the rinsing liquid in order to prevent breakdown of chemical liquid pipes and various parts (a filter, an O-ring, a tube, or the like) due to electrostatic charging, and subsequently generated electrostatic discharging. Examples of the conductive compound include methanol. From the viewpoint that preferred development characteristics or rinsing characteristics are maintained, an addition amount thereof is preferably 10% by mass or less and more preferably 5% by mass or less.

For members of the chemical liquid pipe, for example, various pipes coated with stainless steel (SUS), or a polyethylene, polypropylene, or a fluororesin (a polytetrafluoroethylene resin, a perfluoroalkoxy resin, or the like) that has been subjected to an antistatic treatment can be used. In the same manner, for the filter or the O-ring, polyethylene, polypropylene, or a fluororesin (polytetrafluoroethylene, a perfluoroalkoxy resin, or the like) that has been subjected to an antistatic treatment can be used.

[Method for Manufacturing Electronic Device]

In addition, the present invention further relates to a method for manufacturing an electronic device, including the above-described pattern forming method, and an electronic device manufactured by this manufacturing method.

The electronic device according to the embodiment of the present invention is suitably mounted on electric and electronic apparatus (for example, home appliances, office automation (OA)-related equipment, media-related equipment, optical equipment, telecommunication equipment, and the like).

Examples

Hereinbelow, the present invention will be described in more detail with reference to Examples.

The materials, the amounts of materials used, the proportions, the treatment details, the treatment procedure, and the like shown in Examples below may be appropriately modified as long as the modifications do not depart from the spirit of the present invention. Therefore, the scope of the present invention should not be construed as being limited to Examples shown below.

[Each Component of Resist Composition]

Each component contained in the resist composition used in Examples and Comparative Examples is shown below.

[Resin]

Resins (A-1 to A-22 (corresponding to the resin A) and a-1 to a-9) used for preparing the resist composition are shown below.

In Table 1, the column of "Molar ratio" indicates a content (% by mole) of each repeating unit with respect to all repeating units.

The column of "Mw" indicates a weight-average molecular weight.

The column of "Mw/Mn" indicates a dispersity.

[Table 1]

| | Repeating unit 1 | | Repeating unit 2 | | Repeating unit 3 | | Repeating unit 4 | | Repeating unit 5 | | Mw | Mw/Mn |
|---|---|---|---|---|---|---|---|---|---|---|---|---|
| | Type | Molar ratio | Type | Molar ratio | Type | Molar ratio | Type | Molar ratio | Type | Molar ratio | | |
| Resin A-1 | M-1 | 40 | M-29 | 10 | M-37 | 30 | M-14 | 20 | - | - | 7000 | 1.70 |
| Resin A-2 | M-2 | 40 | M-30 | 5 | M-38 | 20 | M-15 | 35 | - | - | 8000 | 1.60 |
| Resin A-3 | M-3 | 30 | M-31 | 10 | M-39 | 30 | M-16 | 30 | - | - | 6000 | 1.60 |
| Resin A-4 | M-4 | 35 | M-32 | 5 | M-40 | 30 | M-17 | 25 | M-54 | 5 | 6000 | 1.70 |
| Resin A-5 | M-5 | 50 | M-33 | 20 | M-41 | 20 | M-18 | 10 | - | - | 11000 | 1.60 |
| Resin A-6 | M-6 | 20 | M-34 | 15 | M-42 | 20 | M-19 | 45 | - | - | 8000 | 1.55 |
| Resin A-7 | M-7 | 40 | M-35 | 10 | M-43 | 30 | M-20 | 20 | - | - | 9000 | 1.70 |
| Resin A-8 | M-8 | 20 | M-36 | 15 | M-44 | 10 | M-21 | 55 | - | - | 11000 | 1.60 |
| Resin A-9 | M-9 | 30 | M-31 | 10 | M-45 | 30 | M-17 | 20 | M-55 | 10 | 6000 | 1.60 |
| ResinA-10 | M-10 | 25 | M-33 | 10 | M-46 | 35 | M-18 | 20 | M-55 | 10 | 10000 | 1.60 |
| Resin A-11 | M-11 | 20 | M-29 | 5 | M-47 | 30 | M-24 | 45 | - | - | 8000 | 1.70 |
| Resin A-12 | M-12 | 20 | M-30 | 10 | M-48 | 10 | M-23 | 60 | - | - | 9000 | 1.65 |
| Resin A-13 | M-13 | 20 | M-31 | 15 | M-49 | 30 | M-26 | 35 | - | - | 8000 | 1.75 |
| ResinA-14 | M-1 | 30 | M-32 | 5 | M-50 | 25 | M-27 | 35 | M-53 | 5 | 13000 | 1.60 |
| ResinA-15 | M-1 | 30 | M-33 | 20 | M-51 | 40 | M-28 | 10 | - | - | 6000 | 1.75 |
| ResinA-16 | M-1 | 30 | - | - | M-38 | 40 | M-26 | 30 | - | - | 5000 | 1.50 |
| ResinA-17 | M-1 | 20 | - | - | M-37 | 25 | M-27 | 50 | M-55 | 5 | 13000 | 1.50 |
| Resin A-18 | M-1 | 30 | M-31 | 10 | M-38 | 30 | M-26 | 30 | - | - | 6000 | 1.55 |
| ResinA-19 | M-2 | 40 | - | - | M-39 | 50 | M-14 | 10 | - | - | 12000 | 1.65 |
| Resin A-20 | M-2 | 50 | - | - | M-40 | 40 | M-15 | 10 | - | - | 10000 | 1.50 |
| Resin A-21 | M-1 | 30 | - | - | M-41 | 30 | M-18 | 20 | M-25 | 20 | 6000 | 1.70 |
| Resin A-22 | M-2 | 40 | M-34 | 10 | M-42 | 10 | M-22 | 20 | M-28 | 20 | 12000 | 1.50 |
| Resin a-1 | M-1 | 30 | - | - | - | - | M-24 | 70 | - | - | 6000 | 1.65 |
| Resin a-2 | M-1 | 30 | - | - | - | - | M-27 | 70 | - | - | 7000 | 1.55 |

(continued)

| | Repeating unit 1 | | Repeating unit 2 | | Repeating unit 3 | | Repeating unit 4 | | Repeating unit 5 | | Mw | Mw/Mn |
|---|---|---|---|---|---|---|---|---|---|---|---|---|
| | Type | Molar ratio | Type | Molar ratio | Type | Molar ratio | Type | Molar ratio | Type | Molar ratio | | |
| Resin a-3 | M-7 | 20 | - | - | - | - | M-25 | 80 | - | - | 11000 | 1.50 |
| Resin a-4 | M-7 | 20 | - | - | - | - | M-26 | 80 | - | - | 7000 | 1.70 |
| Resin a-5 | M-9 | 30 | M-30 | 10 | - | - | M-14 | 60 | - | - | 10000 | 1.55 |
| Resin a-6 | M-9 | 20 | M-31 | 5 | - | - | M-28 | 75 | - | - | 9000 | 1.75 |
| Resin a-7 | M-1 | 30 | - | - | M-52 | 30 | M-14 | 40 | - | - | 7000 | 1.65 |
| Resin a-8 | M-1 | 30 | - | - | M-37 | 70 | - | - | - | - | 9000 | 1.75 |
| Resin a-9 | M-1 | 30 | - | - | - | - | M-14 | 70 | - | - | 9000 | 1.75 |

Structures of the monomer corresponding to each repeating unit in the resins are shown below.

M-37 to M-51 correspond to the compound (1).

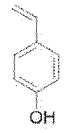
M-1
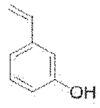
M-2
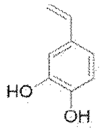
M-3
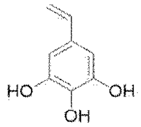
M-4
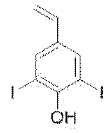
M-5
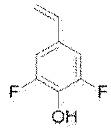
M-6
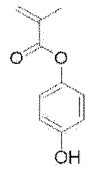
M-7

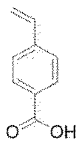
M-8
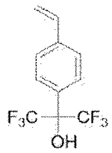
M-9
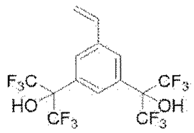
M-10
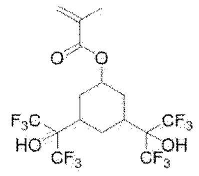
M-11
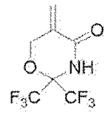
M-12
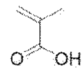
M-13

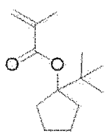
M-14
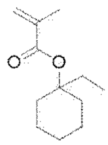
M-15
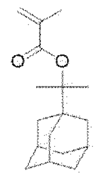
M-16
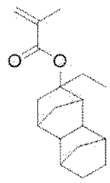
M-17
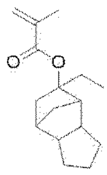
M-18
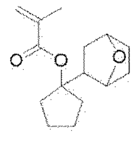
M-19

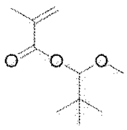
M-20
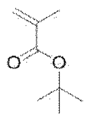
M-21
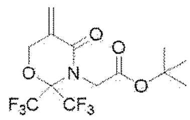
M-22
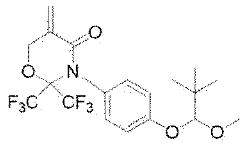
M-23
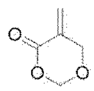
M-24

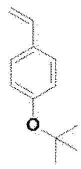
M-25
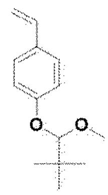
M-26
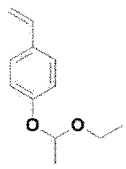
M-27
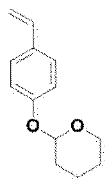
M-28

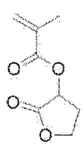
M-29
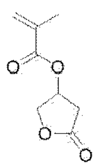
M-30
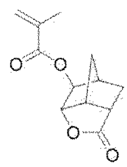
M-31
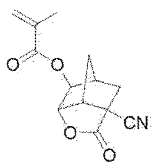
M-32
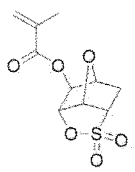
M-33
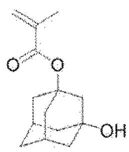
M-34

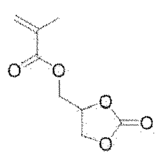
M-35
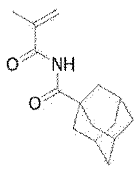
M-36

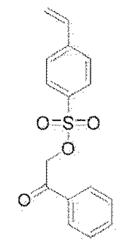
M-37

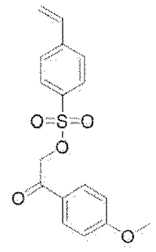
M-38

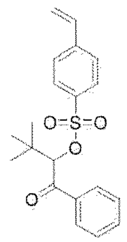
M-39

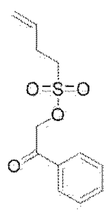
M-40

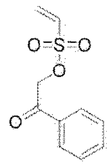
M-41

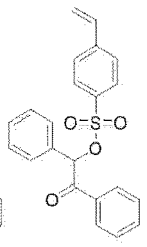
M-42

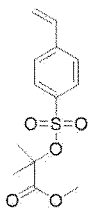
M-43

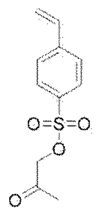
M-44

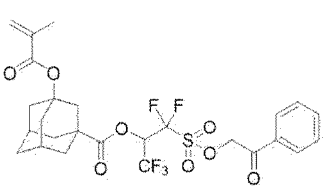
M-45

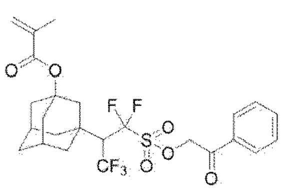
M-46

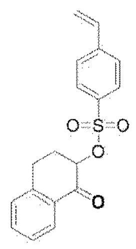
M-47

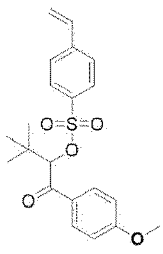
M-48

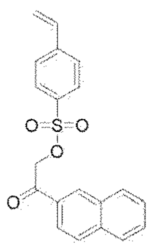
M-49

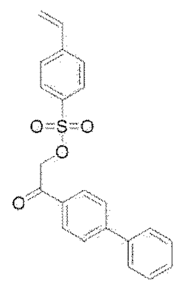
M-50

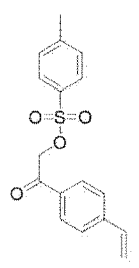
M-51

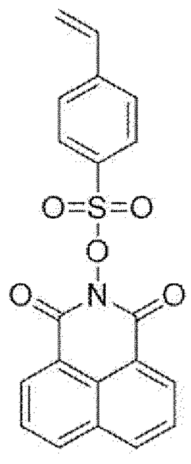
M-52

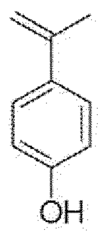
M-53

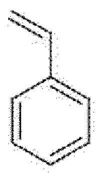
M-54

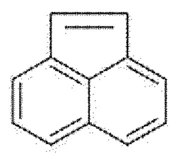
M-55

<Synthesis of Resin A-1>

Cyclohexanone (62 g) was heated to 85°C under a nitrogen stream. While stirring this liquid, a mixed solution (100.7 g) of a cyclohexanone solution (10% by mass) of a monomer (25 g) represented by Formula (M-1), a monomer (8.6 g) represented by Formula (M-29), a monomer (46 g) represented by Formula (M-37), a monomer (21 g) represented by Formula (M-14), cyclohexanone (248 g), and dimethyl 2,2'-azobisisobutyrate [V-601, manufactured by FUJIFILM Wako Pure Chemical Corporation] was added dropwise thereto over 3 hours to obtain a reaction solution. After completion of the dropwise addition, the reaction solution was further stirred at 85°C for 3 hours. The obtained reaction solution was cooled, reprecipitated with a large amount of a mixed solvent of ethyl acetate/heptane = 1/9 (mass ratio), and filtered, and the obtained solid was vacuum-dried to obtain the resin A-1 (83 g). All the synthesis of the above-described resin

A-1 was carried out under a yellow light.

In addition, resins other than the resin A-1 were synthesized according to the synthesis method of the resin A-1.

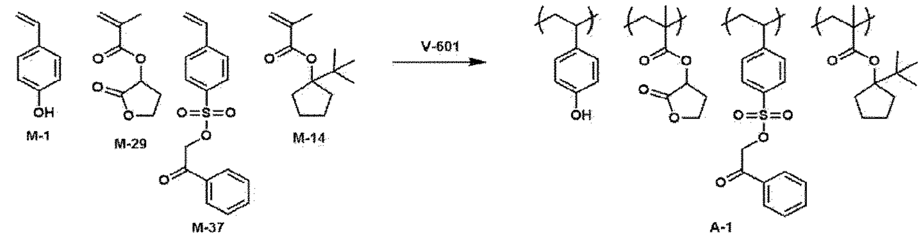

M-1 M-29 M-37 M-14 A-1

[Photoacid Generator]

Structures of photoacid generators (B-1 to B-11 (corresponding to the compound (1)) and b-1 to b-10) used for preparing the resist composition are shown below.

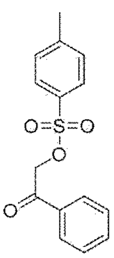
B-1

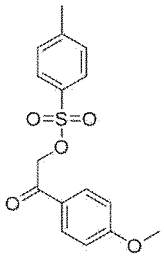
B-2

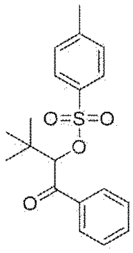
B-3

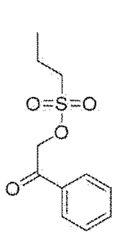
B-4

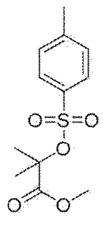
B-5

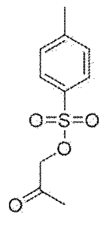
B-6

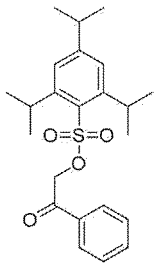
B-7

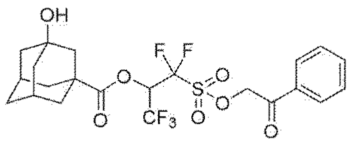
B-8

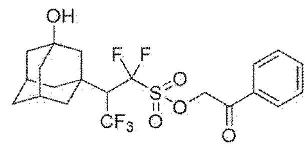
B-9

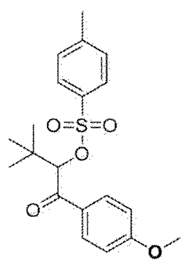
B-10

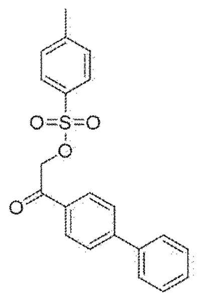
B-11

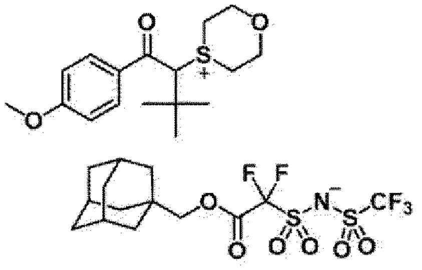

b-1

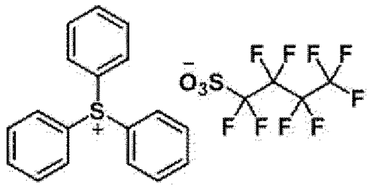

b-2

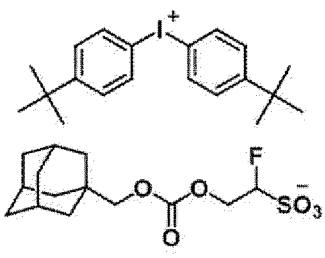

b-3

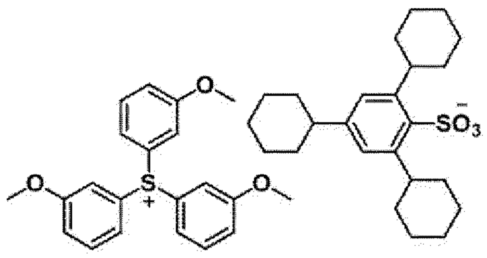

b-4

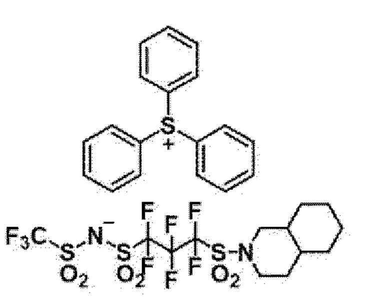

b-5

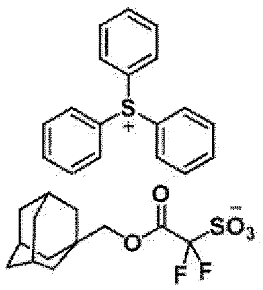

b-6

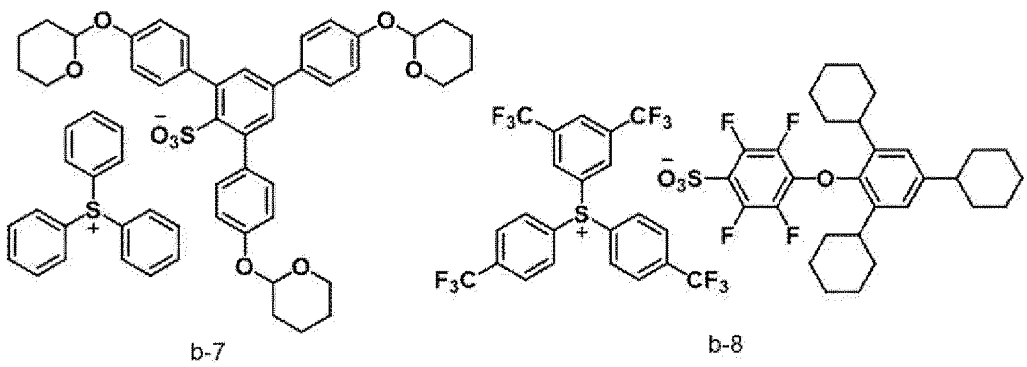

b-7

b-8

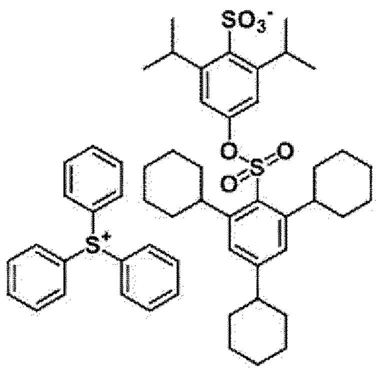

b-9

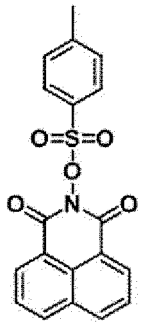

b-10

[Acid Diffusion Control Agent]

Structures of acid diffusion control agents used for preparing the resist composition are shown below.

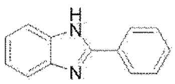

C-1

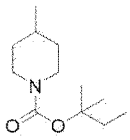

C-2

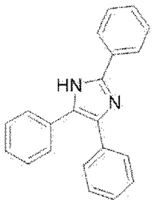

C-3

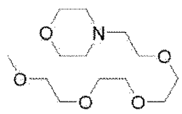

C-4

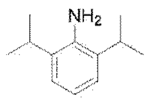

C-5

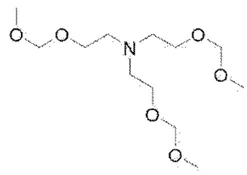

C-6

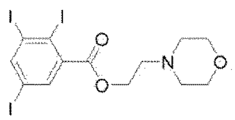

C-7

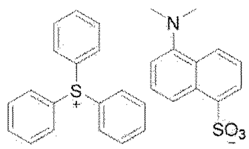

C-8

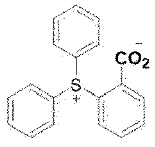

C-9

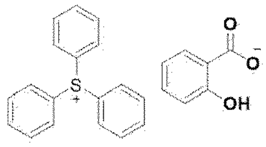

C-10

[Hydrophobic Resin]

Structures of hydrophobic resins used for preparing the resist composition are shown below.

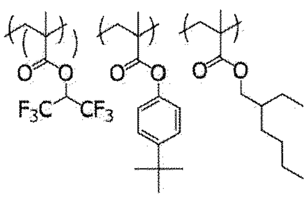

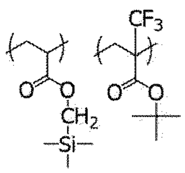

**D-2**

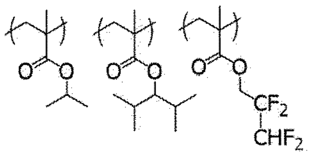

**D-3**

**D-1**

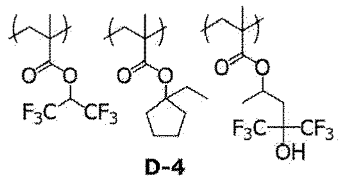

D-4

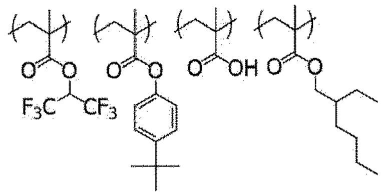

D-5

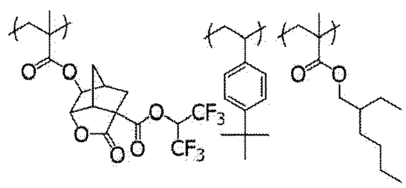

D-6

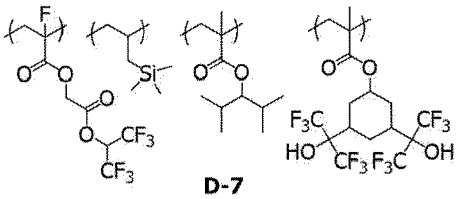

D-7

A compositional ratio (mass ratio; corresponding in order from the left) of each repeating unit of the above-described hydrophobic resin, a weight-average molecular weight (Mw), and a dispersity (Mw/Mn) are shown below.

The hydrophobic resin was synthesized according to the above-described synthesis method (Synthesis Example 1) of the resin A-1.

[Table 2]

| | Mass ratio of repeating unit | | | | Mw | Mw/Mn |
|---|---|---|---|---|---|---|
| D-1 | 50 | 45 | 5 | - | 6500 | 1.52 |
| D-2 | 50 | 50 | - | - | 25000 | 1.65 |
| D-3 | 30 | 65 | 5 | - | 22000 | 1.55 |
| D-4 | 40 | 40 | 20 | - | 12000 | 1.68 |
| D-5 | 40 | 50 | 5 | 5 | 5500 | 1.49 |
| D-6 | 90 | 8 | 2 | - | 12000 | 1.63 |
| D-7 | 20 | 30 | 40 | 10 | 13000 | 1.55 |

[Surfactant]

Structures of surfactants used for preparing the resist composition are shown below.

W-1: MEGAFACE R08 (manufactured by DIC Corporation, fluorine- and silicon-based surfactant)
W-2: MEGAFACE F176 (manufactured by DIC Corporation, fluorine-based surfactant)
W-3: TROYSOL S-366 (manufactured by Troy Corporation, fluorine-based surfactant)
W-4: PF656 (manufactured by OMNOVA Solutions Inc., fluorine-based surfactant)

[Solvent]

Solvents used for preparing the resist composition are shown below.

S-1: Propylene glycol monomethyl ether acetate (PGMEA)
S-2: Propylene glycol monomethyl ether (PGME)
S-3: Propylene glycol monoethyl ether (PGEE)
S-4: Cyclohexanone
S-5: Cyclopentanone
S-6: 2-Heptanone
S-7: Ethyl lactate
S-8: γ-Butyrolactone

S-9: Propylene carbonate

[Preparation of Resist Composition]

Each component shown in the following table was mixed so that the concentration of solid contents was 1.4% by mass. Next, the obtained mixed solution was passed through and filtered with a polyethylene filter having a pore diameter of 0.02 μm to prepare each resist composition.

"Solid content" means all components excluding the solvent.

In the table, the column of "Amount" indicates a content (% by mass) of each solid content component with respect to the total solid content.

The column of "Mixing ratio" in "Solvent" indicates that a mixing ratio (mass ratio) of each solvent.

[Table 3]

| Resist composition | Resin | | Photoacid generator | | Acid diffusion control agent | | Hydrophobic resin | | Surfactant | | Solvent | |
|---|---|---|---|---|---|---|---|---|---|---|---|---|
| | Type | Amount | Type | Amount | Type | Amount | Type | Amount | Type | Amount | Type | Mixed ratio (mass ratio) |
| R-1 | A-1 | 89.9 | b-4 | 5.0 | C-1 | 5.0 | - | - | W-1 | 0.1 | S-1/S-2/S-7 | 20/20/60 |
| R-2 | A-2 | 80.0 | B-2 | 10.0 | C-2 | 5.0 | D-5 | 5.0 | - | - | S-1/S-2 | 30/70 |
| R-3 | A-3 | 90.0 | B-8 | 5.0 | C-3 | 5.0 | - | - | - | - | S-3/S-5 | 20/80 |
| R-4 | A-4 | 94.9 | - | - | C-4 | 5.0 | - | - | W-1 | 0.1 | S-2/S-6 | 50/50 |
| R-5 | A-5 | 80.0 | B-8 | 10.0 | C-5 | 5.0 | D-3 | 5.0 | - | - | S-1/S-2/S-7 | 20/20/60 |
| R-6 | A-6 | 85.0 | B-3 | 5.0 | C-6 | 5.0 | D-6 | 5.0 | - | - | S-1/S-2/S-7 | 20/20/60 |
| R-7 | A-7 | 94.9 | - | - | C-7 | 5.0 | - | - | W-2 | 0.1 | S-2/S-6 | 50/50 |
| R-8 | A-8 | 85.0 | B-5 | 5.0 | C-8 | 10.0 | - | - | - | - | S-1/S-2 | 30/70 |
| R-9 | A-9 | 89.9 | - | - | C-9 | 10.0 | - | - | W-3 | 0.1 | S-1/S-2/S-7 | 20/20/60 |
| R-10 | A-10 | 85.0 | B-11 | 5.0 | C-10 | 10.0 | - | - | - | - | S-1/S-2 | 30/70 |
| R-11 | A-11 | 85.0 | b-9 | 5.0 | C-2 | 5.0 | D-2 | 5.0 | - | - | S-2/S-6 | 50/50 |
| R-12 | A-12 | 95.0 | - | - | C-1 | 5.0 | - | - | - | - | S-1/S-2/S-7 | 20/20/60 |
| R-13 | A-13 | 80.0 | b-5 | 10.0 | C-3 | 5.0 | D-4 | 5.0 | - | - | S-1/S-8 | 50/50 |
| R-14 | A-14 | 95.0 | - | - | C-6 | 5.0 | - | - | - | - | S-1/S-2 | 50/50 |
| R-15 | A-15 | 90.0 | b-3 | 5.0 | C-2 | 5.0 | - | - | - | - | S-1/S-2 | 30/70 |
| R-16 | A-16 | 94.9 | - | - | C-3 | 5.0 | - | - | W-4 | 0.1 | S-1/S-2 | 30/70 |
| R-17 | A-17 | 85.0 | b-2 | 10.0 | C-1 | 5.0 | - | - | - | - | S-1/S-2 | 30/70 |
| R-18 | A-18 | 95.0 | - | - | C-8 | 5.0 | - | - | - | - | S-1/S-2/S-7 | 20/20/60 |
| R-19 | A-19 | 95.0 | - | - | C-4 | 5.0 | - | - | - | - | S-2/S-6 | 50/50 |
| R-20 | A-20 | 90.0 | - | - | C-5 | 5.0 | D-1 | 5.0 | - | - | S-1/S-2 | 30/70 |
| R-21 | A-21 | 84.9 | B-6 | 10.0 | C-7 | 5.0 | - | - | W-3 | 0.1 | S-1/S-4 | 20/80 |
| R-22 | A-22 | 95.0 | - | - | C-9 | 5.0 | - | - | - | - | S-1/S-2 | 50/50 |
| R-23 | a-1 | 60.0 | B-1 | 30.0 | C-10 | 10.0 | - | - | - | - | S-1/S-2 | 50/50 |
| R-24 | a-2 | 70.0 | B-10 | 25.0 | C-6 | 5.0 | - | - | - | - | S-1/S-8 | 50/50 |

(continued)

| Resist composition | Resin | | Photoacid generator | | Acid diffusion control agent | | Hydrophobic resin | | Surfactant | | Solvent | |
|---|---|---|---|---|---|---|---|---|---|---|---|---|
| | Type | Amount | Type | Amount | Type | Amount | Type | Amount | Type | Amount | Type | Mixed ratio (mass ratio) |
| R-25 | a-3 | 70.0 | B-4 | 20.0 | C-1 | 5.0 | D-6 | 5.0 | - | - | S-1/S-9 | 30/70 |
| R-26 | a-4 | 69.9 | B-7 | 25.0 | C-7 | 5.0 | - | - | W-4 | 0.1 | S-1/S-8 | 50/50 |
| R-27 | a-5 | 60.0 | B-9 | 30.0 | C-1 | 5.0 | D-7 | 5.0 | - | - | S-1/S-2 | 30/70 |
| R-28 | a-6 | 70.0 | B-8 | 25.0 | C-10 | 5.0 | - | - | - | - | S-1/S-2 | 30/70 |
| R-29 | A-11 | 74.9 | b-8 | 20.0 | C-1 | 5.0 | - | - | W-1 | 0.1 | S-1/S-2 | 30/70 |
| R-30 | A-12 | 94.9 | - | - | C-4 | 5.0 | - | - | W-2 | 0.1 | S-1/S-2 | 30/70 |
| R-31 | A-13 | 90.0 | - | - | C-1 | 5.0 | D-1 | 5.0 | - | - | S-1/S-2 | 30/70 |
| R-32 | A-14 | 89.9 | b-6 | 5.0 | C-2 | 5.0 | - | - | W-1 | 0.1 | S-1/S-8 | 50/50 |
| R-33 | A-15 | 90.0 | b-7 | 5.0 | C-4 | 5.0 | - | - | - | - | S-1/S-2 | 30/70 |
| R-34 | A-16 | 95.0 | - | - | C-8 | 5.0 | - | - | - | - | S-1/S-2 | 30/70 |
| R-35 | A-17 | 89.9 | b-1 | 5.0 | C-2 | 5.0 | - | - | W-2 | 0.1 | S-1/S-2 | 30/70 |
| R-36 | A-18 | 85.0 | b-4 | 10.0 | C-3 | 5.0 | - | - | - | - | S-1/S-2 | 30/70 |
| r-1 | a-7 | 95.0 | - | - | C-1 | 5.0 | - | - | - | - | S-1/S-2 | 30/70 |
| r-2 | a-8 | 100.0 | - | - | - | - | - | - | - | - | S-1/S-2 | 30/70 |
| r-3 | a-9 | 75.0 | b-10 | 20.0 | C-1 | 5.0 | - | - | - | - | S-1/S-2 | 30/70 |

[Test]

[Pattern Formation (1): EB Exposure and Alkali Development]

A 6-inch silicon wafer (or chrome wafer) was subjected to a hexamethyldisilazane (HMDS) treatment. Each of the resist compositions shown in the following table was applied onto the surface-treated silicon wafer obtained above using a spin coater Mark 8 (manufactured by Tokyo Electron Limited), and dried on a hot plate at a temperature of 130°C for 300 seconds to obtain each resist film having a film thickness of 100 nm. In addition, in the above-described production method of the resist film, a chrome substrate may be used instead of the silicon wafer.

The obtained resist film was subjected to pattern exposure with an exposure mask (line:space = 1:1) using an electron beam drawing apparatus (manufactured by Advantest Corporation, F7000S; acceleration voltage: 50 KeV). Next, the resist film was heated on a hot plate at a temperature of 100°C for 60 seconds, immersed in a 2.38% by mass tetramethylammonium (TMAH) aqueous solution for 60 seconds, and then rinsed with water for 30 seconds. Finally, the silicon wafer was rotated at a rotation speed of 4,000 rpm for 30 seconds, and baked and dried at a temperature of 95°C for 60 seconds to obtain a silicon wafer having a predetermined pattern.

<Sensitivity>

While changing an exposure amount (amount of electron beam irradiation), a line width of the line-and-space pattern was measured, and an exposure amount at which the line width reached 50 nm was determined and defined as a sensitivity (Eop, $\mu C/cm^2$). As the value of the sensitivity is smaller, the sensitivity is better.

<Resolution>

The minimum dimension of a pattern (line:space = 1:1) which could be resolved without collapsing at the exposure amount showing the above-described sensitivity (Eop) was obtained using a length-measuring scanning electron microscope (SEM, S-9380II, manufactured by Hitachi, Ltd.) and defined as a resolution (L/S) (nm). As the value of the resolution is smaller, the resolution is better.

<LWR (Roughness Performance)>

A line width of a pattern resolved to have a line width of 50 nm and line:space =1:1 at the exposure amount showing the above-described sensitivity (Eop) was measured at an arbitrary point from an upper part of the pattern using a length-measuring scanning electron microscope (SEM, S-9380II, manufactured by Hitachi, Ltd.). Variation of each obtained line width was evaluated as $3\sigma$ (nm). As the value of $3\sigma$ is smaller, the LWR is better.

<LWR After Aging Storage>

Each of the resist compositions obtained above was stored at 23°C for 1 year, and LWR was evaluated in the same procedure as in <LWR (Roughness Performance)> described above to obtain $3\sigma$ (nm), except that the stored resist composition was used for the LWR test, and it was defined as LWR (nm) after aging storage.

<Result>

In the table, each description indicates the following.

In the column of "Resin" in "$R^4$ is aromatic ring group", a case where the resin had a residue formed by removing one hydrogen atom from the compound (1) in which $R^4$ was an aromatic ring group is denoted as "A", and otherwise, it was denoted as "B".

In the column of "Photoacid generator" in "$R^4$ is aromatic ring group", a case where $R^4$ in the compound represented by Formula (1) (photoacid generator) represented an aromatic ring group is denoted as "A", and otherwise, it was denoted as "B".

In the column of "Nitrogen-containing basic compound", a case where the resist composition contained a nitrogen-containing basic compound is denoted as "A", and otherwise, it was denoted as "B".

In the column of "Content of repeating unit b of 15% by mole or more", a case where the content of the repeating unit b was 15% by mole or more with respect to all repeating units is denoted as "A", and otherwise, it was denoted as "B".

In the column of "Formulae (M4) and (M5)", a case where the repeating unit b included at least one selected from the group consisting of the repeating units represented by Formulae (M4) and (M5) is denoted as "A", and otherwise, it was denoted as "B".

[Table 4]

| | Resist composition | Resin | Photoacid generator | $R^4$ is aromatic ring group | | Nitrogen-containing basic compound | Content of repeating unit b of 15% by mole or more | Formula (M4) and Formula (M5) | Sensitivity [μC/cm²] | Resolution [nm] | LWR [nm] | LWR after aging storage [nm] |
|---|---|---|---|---|---|---|---|---|---|---|---|---|
| | | | | Resin | Photoacid generator | | | | | | | |
| Example 1a | R-1 | A-1 | b-4 | A | B | A | A | B | 57.5 | 25 | 4.4 | 4.5 |
| Example 2a | R-2 | A-2 | B-2 | A | A | A | A | B | 55.0 | 26 | 4.5 | 4.5 |
| Example 3a | R-3 | A-3 | B-8 | A | A | A | A | B | 54.8 | 27 | 4.4 | 4.4 |
| Example 4a | R-4 | A-4 | - | A | B | A | A | B | 57.9 | 28 | 4.5 | 4.5 |
| Example 5a | R-5 | A-5 | B-8 | A | A | A | B | B | 55.0 | 30 | 4.6 | 4.6 |
| Example 6a | R-6 | A-6 | B-3 | A | A | A | A | B | 55.2 | 26 | 4.5 | 4.5 |
| Example 7a | R-7 | A-7 | - | B | B | A | A | A | 62.3 | 25 | 4.1 | 4.1 |
| Example 8a | R-8 | A-8 | B-5 | B | B | A | A | B | 61.5 | 27 | 4.4 | 4.4 |
| Example 9a | R-9 | A-9 | - | A | B | B | A | B | 58.5 | 26 | 4.5 | 4.8 |
| Example 10a | R-10 | A-10 | B-11 | A | A | B | A | B | 56.0 | 25 | 4.5 | 4.9 |
| Example IIa | R-11 | A-11 | b-9 | A | B | A | A | B | 57.0 | 28 | 4.4 | 4.5 |
| Example 12a | R-12 | A-12 | - | A | B | A | A | B | 56.4 | 28 | 4.4 | 4.4 |
| Example 13a | R-13 | A-13 | b-5 | A | B | A | A | A | 58.5 | 25 | 4.1 | 4.1 |
| Example 14a | R-14 | A-14 | - | A | B | A | A | A | 56.8 | 25 | 4.0 | 4.1 |
| Example 15a | R-15 | A-15 | b-3 | A | B | A | B | A | 59.1 | 30 | 4.2 | 42 |
| Example 16a | R-16 | A-16 | - | A | B | A | A | A | 58.3 | 27 | 4.0 | 4.0 |
| Example 17a | R-17 | A-17 | b-2 | A | B | A | A | A | 57.4 | 27 | 4.1 | 4.1 |
| Example 18a | R-18 | A-18 | - | A | B | A | A | A | 58.4 | 25 | 4.0 | 4.1 |
| Example 19a | R-19 | A-19 | - | A | B | A | B | B | 59.4 | 30 | 4.7 | 4.7 |
| Example 20a | R-20 | A-20 | - | A | B | A | B | B | 57.8 | 30 | 4.6 | 4.6 |
| Example 21a | R-21 | A-21 | B-6 | A | B | A | A | B | 57.8 | 26 | 4.5 | 4.5 |
| Example 22a | R-22 | A-22 | - | A | B | B | A | A | 59.1 | 28 | 4.1 | 5.0 |

(continued)

| | Resist composition | Resin | Photoacid generator | $R^4$ is aromatic ring group | | Nitrogen-containing basic compound | Content of repeating unit b of 15% by mole or more | Formula (M4) and Formula (M5) | Sensitivity [$\mu C/cm^2$] | Resolution [nm] | LWR [nm] | LWR after aging storage [nm] |
|---|---|---|---|---|---|---|---|---|---|---|---|---|
| | | | | Resin | Photoacid generator | | | | | | | |
| Example 23a | R-23 | a-1 | B-1 | B | A | B | A | B | 58.7 | 29 | 4.5 | 4.9 |
| Example 24a | R-24 | a-2 | B-10 | B | A | A | A | A | 59.6 | 29 | 4.1 | 4.1 |
| Example 25a | R-25 | a-3 | B-4 | B | A | A | A | B | 56.9 | 25 | 4.5 | 4.6 |
| Example 26a | R-26 | a-4 | B-7 | B | A | A | A | A | 57.1 | 27 | 4.1 | 4.1 |
| Example 27a | R-27 | a-5 | B-9 | B | A | A | A | B | 59.8 | 26 | 4.5 | 4.5 |
| Example 28a | R-28 | a-6 | B-8 | B | A | B | A | A | 57.4 | 28 | 4.1 | 4.9 |
| Example 29a | R-29 | A-11 | b-8 | A | B | A | A | B | 56.7 | 25 | 4.4 | 4.4 |
| Example 30a | R-30 | A-12 | - | A | B | A | A | B | 56.8 | 29 | 4.4 | 4.4 |
| Example 31a | R-31 | A-13 | - | A | B | A | A | A | 58.1 | 25 | 4.1 | 4.2 |
| Example 32a | R-32 | A-14 | b-6 | A | B | A | A | A | 58.0 | 28 | 4.0 | 4.0 |
| Example 33a | R-33 | A-15 | b-7 | A | B | A | B | A | 57.4 | 30 | 4.2 | 42 |
| Example 34a | R-34 | A-16 | - | A | B | A | A | A | 59.3 | 27 | 4.0 | 4.0 |
| Example 35a | R-35 | A-17 | b-1 | A | B | A | A | A | 56.6 | 25 | 4.0 | 4.0 |
| Example 36a | R-36 | A-18 | b-4 | A | B | A | A | A | 58.7 | 26 | 4.1 | 4.1 |
| Comparative Example la | r-l | a-7 | - | - | - | - | - | - | 80.9 | 50 | 6.0 | 6.1 |
| Comparative Example 2a | r-2 | a-8 | - | - | - | - | - | - | 76.5 | 50 | 6.2 | 6.2 |
| Comparative Example 3a | r-3 | a-9 | b-10 | - | - | - | - | - | 77.4 | 45 | 6.0 | 6.1 |

From the results shown in the table, it was confirmed that the desired effects of the present invention could be obtained in the case of EB exposure and alkali development using the resist composition according to the embodiment of the present invention.

In a case where the resin had a residue formed by removing one hydrogen atom from the compound (1) in which $R^4$ was an aromatic ring group or the $R^4$ in the compound (1) represented an aromatic ring group, it was confirmed that the sensitivity was more excellent (comparison between Examples 1a and the like and Examples 7a and 8a). In addition, in a case where the resin had a residue formed by removing one hydrogen atom from the compound (1) in which $R^4$ was an aromatic ring group and the $R^4$ in the compound (1) represented an aromatic ring group, it was confirmed that the sensitivity was still more excellent (comparison between Examples 1a and the like and Examples 2a, 3a, 5a, 6a, 10a, and 22a to 28a).

In a case where the resist composition further contained a nitrogen-containing basic compound, it was confirmed that the LWR after aging storage was more excellent (comparison between Examples 1a and the like and Examples 9a, 10a, 22a, 23a, and 28a).

In a case where the content of the repeating unit b was 15% by mole or more with respect to all repeating units, it was confirmed that the resolution and LWR were more excellent (comparison between Examples 1a and the like and Examples 5a, 15a, 19a, 20a, and 33a).

In a case where the repeating unit b included at least one selected from the group consisting of the repeating unit represented by Formula (M4) and the repeating unit represented by (M5), it was confirmed that the LWR was more excellent (comparison between Examples 7a, 13a to 18a, 22a, 24a, 26a, 28a, and 31a to 36a, and Examples 1a and the like).

[Pattern Formation (2): EUV Exposure and Alkali Development]

A 6-inch silicon wafer (or chrome wafer) was subjected to a hexamethyldisilazane (HMDS) treatment. Each of the resist compositions shown in the following table was applied onto the surface-treated silicon wafer obtained above using a spin coater Mark 8 (manufactured by Tokyo Electron Limited), and dried on a hot plate at a temperature of 130°C for 300 seconds to obtain each resist film having a film thickness of 100 nm. In addition, in the above-described production method of the resist film, a chrome substrate may be used instead of the silicon wafer.

Using an EUV exposure device (manufactured by Exitech Ltd., Micro Exposure Tool, numerical aperture (NA): 0.3, Quadrupole, outer sigma: 0.68, inner sigma: 0.36), the obtained resist film was subjected to pattern exposure with an exposure mask (line:space = 1/1).

Next, the resist film was heated on a hot plate at a temperature of 100°C for 90 seconds, immersed in a 2.38% by mass tetramethylammonium (TMAH) aqueous solution for 60 seconds, and then rinsed with water for 30 seconds. Finally, the silicon wafer was rotated at a rotation speed of 4,000 rpm for 30 seconds, and baked and dried at a temperature of 95°C for 60 seconds to obtain a silicon wafer having a predetermined pattern.

Using the obtained pattern, each of the items of sensitivity, resolution (L/S), LWR (roughness performance), and LWR after aging storage was evaluated in the same manner as in [Pattern Formation (1): EB Exposure and Alkali Development] described above.

<Result>

In the table, each description is the same as in Table 4.

[Table 5]

| | Resist composition | Resin | Photoacid generator | $R^4$ is aromatic ring group | | Nitrogen-containing basic compound | Content of repeating unit b of 15% by mole or more | Formula (M4) and Formula (M5) | Sensitivity [$\mu C/cm^2$] | Resolution [nm] | LWR [nm] | LWR after aging storage [nm] |
|---|---|---|---|---|---|---|---|---|---|---|---|---|
| | | | | Resin | Photoacid generator | | | | | | | |
| Example 1b | R-1 | A-1 | b-4 | A | B | A | A | B | 20.1 | 25 | 4.5 | 4.5 |
| Example 2b | R-2 | A-2 | B-2 | A | A | A | A | B | 19.0 | 26 | 4.5 | 4.5 |
| Example 3b | R-3 | A-3 | B-8 | A | A | A | A | B | 18.9 | 25 | 4.6 | 4.7 |
| Example 4b | R-4 | A-4 | - | A | B | A | A | B | 20.9 | 28 | 4.5 | 4.5 |
| Example 5b | R-5 | A-5 | B-8 | A | A | A | B | B | 19.2 | 31 | 4.7 | 4.7 |
| Example 6b | R-6 | A-6 | B-3 | A | A | A | A | B | 19.0 | 27 | 4.6 | 4.5 |
| Example 7b | R-7 | A-7 | - | B | B | A | A | A | 25.6 | 25 | 4.2 | 4.2 |
| Example 8b | R-8 | A-8 | B-5 | B | B | A | A | B | 24.9 | 25 | 4.5 | 4.5 |
| Example 9b | R-9 | A-9 | - | A | B | B | A | B | 22.3 | 27 | 4.5 | 4.8 |
| Example 10b | R-10 | A-10 | B-11 | A | A | B | A | B | 19.1 | 25 | 4.6 | 5.0 |
| Example 11b | R-11 | A-11 | b-9 | A | B | A | A | B | 20.6 | 28 | 4.5 | 4.5 |
| Example 12b | R-12 | A-12 | - | A | B | A | A | B | 20.9 | 27 | 4.6 | 4.6 |
| Example 13b | R-13 | A-13 | b-5 | A | B | A | A | A | 20.8 | 26 | 4.1 | 4.2 |
| Example 14b | R-14 | A-14 | - | A | B | A | A | A | 21.1 | 25 | 4.0 | 4.1 |
| Example 15b | R-15 | A-15 | b-3 | A | B | A | B | A | 21.9 | 32 | 4.3 | 4.2 |
| Example 16b | R-16 | A-16 | - | A | B | A | A | A | 21.7 | 27 | 4.1 | 4.0 |
| Example 17b | R-17 | A-17 | b-2 | A | B | A | A | A | 22.3 | 25 | 4.2 | 4.2 |
| Example 18b | R-18 | A-18 | - | A | B | A | A | A | 20.4 | 25 | 4.2 | 4.1 |
| Example 19b | R-19 | A-19 | - | A | B | A | B | B | 20.4 | 31 | 4.7 | 4.7 |
| Example 20b | R-20 | A-20 | - | A | B | A | B | B | 21.6 | 33 | 4.7 | 4.7 |
| Example 21b | R-21 | A-21 | B-6 | A | B | A | A | B | 22.8 | 26 | 4.5 | 4.5 |
| Example 22b | R-22 | A-22 | - | A | B | B | A | A | 21.0 | 25 | 4.2 | 4.9 |

(continued)

| | Resist composition | Resin | Photoacid generator | $R^4$ is aromatic ring group | | Nitrogen-containing basic compound | Content of repeating unit b of 15% by mole or more | Formula (M4) and Formula (M5) | Sensitivity [μC/cm$^2$] | Resolution [nm] | LWR [nm] | LWR after aging storage [nm] |
|---|---|---|---|---|---|---|---|---|---|---|---|---|
| | | | | Resin | Photoacid generator | | | | | | | |
| Example 23b | R-23 | a-1 | B-1 | B | A | B | A | B | 21.8 | 29 | 4.6 | 4.9 |
| Example 24b | R-24 | a-2 | B-10 | B | A | A | A | A | 22.2 | 29 | 4.2 | 43 |
| Example 25b | R-25 | a-3 | B-4 | B | A | A | A | B | 21.1 | 25 | 4.5 | 4.6 |
| Example 26b | R-26 | a-4 | B-7 | B | A | A | A | A | 21.7 | 27 | 4.2 | 42 |
| Example 27b | R-27 | a-5 | B-9 | B | A | A | A | B | 21.7 | 26 | 4.5 | 4.5 |
| Example 28b | R-28 | a-6 | B-8 | B | A | B | A | A | 21.9 | 25 | 4.1 | 4.9 |
| Example 29b | R-29 | A-11 | b-8 | A | B | A | A | B | 22.3 | 25 | 4.6 | 4.6 |
| Example 30b | R-30 | A-12 | - | A | B | A | A | B | 22.4 | 27 | 4.5 | 4.5 |
| Example 31b | R-31 | A-13 | - | A | B | A | A | A | 20.4 | 25 | 4.2 | 43 |
| Example 32b | R-32 | A-14 | b-6 | A | B | A | A | A | 20.5 | 28 | 4.0 | 4.0 |
| Example 33b | R-33 | A-15 | b-7 | A | B | A | B | A | 22.9 | 32 | 4.3 | 4.2 |
| Example 34b | R-34 | A-16 | - | A | B | A | A | A | 21.7 | 25 | 4.0 | 4.0 |
| Example 35b | R-35 | A-17 | b-1 | A | B | A | A | A | 22.9 | 26 | 4.0 | 4.1 |
| Example 36b | R-36 | A-18 | b-4 | A | B | A | A | A | 20.9 | 26 | 4.1 | 4.1 |
| Comparative Example 1b | r-l | a-7 | - | - | - | - | - | - | 40.6 | 45 | 6.0 | 6.1 |
| Comparative Example 2b | r-2 | a-8 | - | - | - | - | - | - | 43.5 | 50 | 6.3 | 6.3 |
| Comparative Example 3b | r-3 | a-9 | b-10 | - | - | - | - | - | 45.7 | 50 | 6.2 | 6.2 |

From the results shown in the table, it was confirmed that the desired effects of the present invention could be obtained in the case of EUV exposure and alkali development using the resist composition according to the embodiment of the present invention.

In a case where the resin had a residue formed by removing one hydrogen atom from the compound (1) in which $R^4$ was an aromatic ring group or the $R^4$ in the compound (1) represented an aromatic ring group, it was confirmed that the sensitivity was more excellent (comparison between Examples 1b and the like and Examples 7b and 8b). In addition, in a case where the resin had a residue formed by removing one hydrogen atom from the compound (1) in which $R^4$ was an aromatic ring group and the $R^4$ in the compound (1) represented an aromatic ring group, it was confirmed that the sensitivity was still more excellent (comparison between Examples 1b and the like and Examples 2b, 3b, 5b, 6b, 10b, and 22b to 28b).

In a case where the resist composition further contained a nitrogen-containing basic compound, it was confirmed that the LWR after aging storage was more excellent (comparison between Examples 1b and the like and Examples 9b, 10b, 22b, 23b, and 28b).

In a case where the content of the repeating unit b was 15% by mole or more with respect to all repeating units, it was confirmed that the resolution and LWR were more excellent (comparison between Examples 1b and the like and Examples 5b, 15b, 19b, 20b, and 33b).

In a case where the repeating unit b included at least one selected from the group consisting of the repeating unit represented by Formula (M4) and the repeating unit represented by (M5), it was confirmed that the LWR was more excellent (comparison between Examples 7b, 13b to 18b, 22b, 24b, 26b, 28b, and 31b to 36b, and Examples 1b and the like).

## Claims

1. An actinic ray-sensitive or radiation-sensitive resin composition comprising:

a resin which is decomposed by action of acid to increase polarity,
wherein at least one of a requirement that the actinic ray-sensitive or radiation-sensitive resin composition further contains a compound represented by Formula (1), or
a requirement that the resin which is decomposed by action of acid to increase polarity has a residue formed by removing one hydrogen atom from the compound represented by Formula (1) is satisfied,

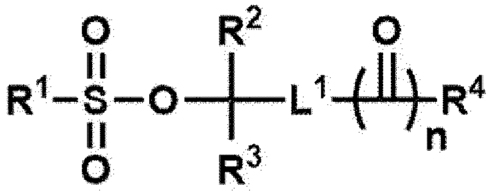

(1)

in Formula (1), $R^1$ and $R^4$ each independently represent a substituent, $R^2$ and $R^3$ each independently represent a hydrogen atom or a substituent, $L^1$ represents a single bond or a divalent linking group, and n represents an integer of 1 or more.

2. The actinic ray-sensitive or radiation-sensitive resin composition according to claim 1,

wherein the residue includes at least one selected from the group consisting of a group represented by Formula (2a) and a group represented by Formula (2b),

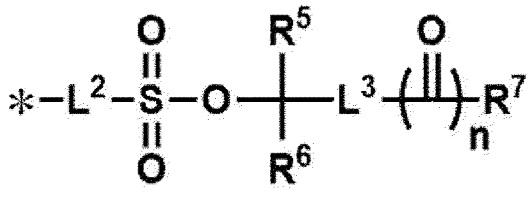

(2a)

in Formula (2a), $R^5$ and $R^6$ each independently represent a hydrogen atom or a substituent, $R^7$ represents a substituent, $L^2$ represents a divalent linking group, $L^3$ represents a single bond or a divalent linking group, n represents an integer of 1 or more, and * represents a bonding position,

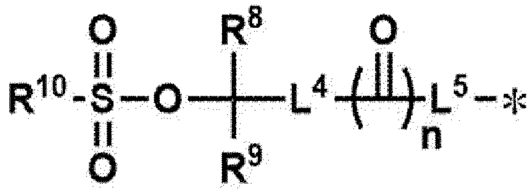

(2b)

in Formula (2b), $R^8$ and $R^9$ each independently represent a hydrogen atom or a substituent, $R^{10}$ represents a substituent, $L^4$ represents a single bond or a divalent linking group, $L^5$ represents a divalent linking group, n represents an integer of 1 or more, and * represents a bonding position.

**3.** The actinic ray-sensitive or radiation-sensitive resin composition according to claim 1,
wherein $R^4$ represents an aromatic ring group.

**4.** The actinic ray-sensitive or radiation-sensitive resin composition according to any one of claims 1 to 3, further comprising:
a nitrogen-containing basic compound.

**5.** The actinic ray-sensitive or radiation-sensitive resin composition according to any one of claims 1 to 4,

wherein the resin which is decomposed by action of acid to increase polarity includes a repeating unit b having a group which is decomposed by action of acid to increase polarity, and
a content of the repeating unit b is 15% by mole or more with respect to all repeating units.

**6.** The actinic ray-sensitive or radiation-sensitive resin composition according to any one of claims 1 to 5,

wherein the resin which is decomposed by action of acid to increase polarity includes a repeating unit b having a group which is decomposed by action of acid to increase polarity, and
the repeating unit b includes at least one selected from the group consisting of repeating units represented by Formulae (M1) to (M5),

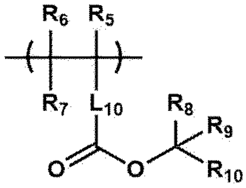

(M1)

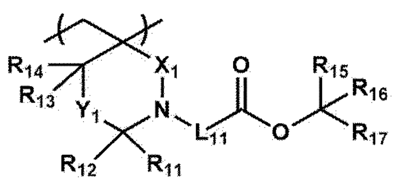

(M2)

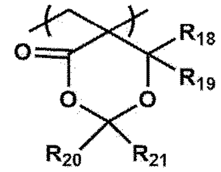

(M3)

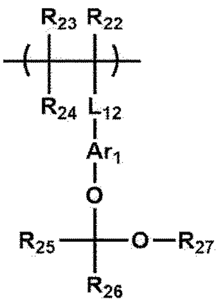

(M4)

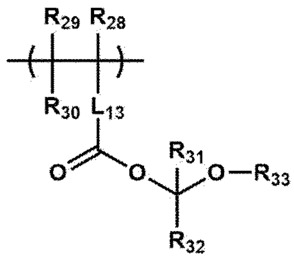

(M5)

in Formula (M1), $R_5$ to $R_7$ each independently represent a hydrogen atom, an alkyl group, a cycloalkyl group, a halogen atom, a cyano group, or an alkoxycarbonyl group, $L_{10}$ represents a single bond or a divalent linking group, and $R_8$ to $R_{10}$ each independently represent an alkyl group, a cycloalkyl group, an aryl group, an aralkyl group, or an alkenyl group, where at least two of $R_8$ to $R_{10}$ may be bonded to each other to form a ring,
in Formula (M2), $R_{11}$ to $R_{14}$ each independently represent a hydrogen atom or an organic group, where at least one of $R_{11}$ or $R_{12}$ represents an organic group, $X_1$ represents -CO-, -SO-, or $-SO_2-$, $Y_1$ represents -O-, -S-, -SO-, $-SO_2-$, or $-NR_{34}-$, $R_{34}$ represents a hydrogen atom or an organic group, $L_{11}$ represents a single bond or a divalent linking group, and $R_{15}$ to $R_{17}$ each independently represent an alkyl group, a cycloalkyl group, an aryl group, an aralkyl group, or an alkenyl group, where at least two of $R_{15}$ to $R_{17}$ may be bonded to each other to form a ring,
in Formula (M3), $R_{18}$ and $R_{19}$ each independently represent a hydrogen atom or an organic group, and $R_{20}$ and $R_{21}$ each independently represent a hydrogen atom, an alkyl group, a cycloalkyl group, an aryl group, an aralkyl group, or an alkenyl group, where at least two of $R_{18}$ to $R_{21}$ may be bonded to each other to form a ring,
in Formula (M4), $R_{22}$ to $R_{24}$ each independently represent a hydrogen atom, an alkyl group, a cycloalkyl group, a halogen atom, a cyano group, or an alkoxycarbonyl group, $L_{12}$ represents a single bond or a divalent linking group, An represents an aromatic ring group, and $R_{25}$ to $R_{27}$ each independently represent a hydrogen atom, an alkyl group, a cycloalkyl group, an aryl group, an aralkyl group, or an alkenyl group, where at least two of $R_{25}$ to $R_{27}$ may be bonded to each other to form a ring,
in Formula (M5), $R_{28}$ to $R_{30}$ each independently represent a hydrogen atom, an alkyl group, a cycloalkyl group, a halogen atom, a cyano group, or an alkoxycarbonyl group, $L_{13}$ represents a single bond or a divalent linking group, $R_{31}$ and $R_{32}$ each independently represent a hydrogen atom, an alkyl group, a cycloalkyl group, an aryl group, an aralkyl group, or an alkenyl group, and $R_{33}$ represents an alkyl group, a cycloalkyl group, an aryl group, an aralkyl group, or an alkenyl group, where at least two of $R_{31}$ to $R_{33}$ may be bonded to each other to form a ring.

**7.** The actinic ray-sensitive or radiation-sensitive resin composition according to claim 6,
wherein the repeating unit b includes at least one selected from the group consisting of the repeating unit represented by Formula (M4) and the repeating unit represented by Formula (M5).

**8.** A resist film formed of the actinic ray-sensitive or radiation-sensitive resin composition according to any one of claims 1 to 7.

**9.** A pattern forming method comprising:

a step of forming a resist film on a substrate using the actinic ray-sensitive or radiation-sensitive resin composition according to any one of claims 1 to 7;
a step of exposing the resist film; and
a step of developing the exposed resist film using a developer.

**10.** A method for manufacturing an electronic device, comprising:
the pattern forming method according to claim 9.

**INTERNATIONAL SEARCH REPORT**

International application No.
**PCT/JP2021/047175**

**A. CLASSIFICATION OF SUBJECT MATTER**

***C07C 309/67***(2006.01)i; ***C07C 309/68***(2006.01)i; ***C07C 309/73***(2006.01)i; ***C08F 220/12***(2006.01)i; ***G03F 7/004***(2006.01)i; ***G03F 7/039***(2006.01)i; ***G03F 7/20***(2006.01)i
FI: G03F7/004 503A; G03F7/039 601; G03F7/004 501; C08F220/12; G03F7/20 521; C07C309/73; C07C309/67; C07C309/68

According to International Patent Classification (IPC) or to both national classification and IPC

**B. FIELDS SEARCHED**

Minimum documentation searched (classification system followed by classification symbols)

C07C309/67; C07C309/68; C07C309/73; C08F220/12; G03F7/004; G03F7/039; G03F7/20

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 4-215661 A (FUJITSU LTD) 06 August 1992 (1992-08-06) claims, example 3 | 1-10 |
| X | JP 2002-236359 A (FUJI PHOTO FILM CO LTD) 23 August 2002 (2002-08-23) claims, example 1, etc. | 1-10 |
| X | JP 2002-236358 A (FUJI PHOTO FILM CO LTD) 23 August 2002 (2002-08-23) claims, example 1, etc. | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 March 2022** | **22 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/047175**

| Patent document cited in search report | Publication date (day/month/year) | Patent family member(s) | Publication date (day/month/year) |
|---|---|---|---|
| JP 4-215661 A | 06 August 1992 | (Family: none) | |
| JP 2002-236359 A | 23 August 2002 | TW 571178 B<br>KR 10-2002-0070779 A | |
| JP 2002-236358 A | 23 August 2002 | TW 571178 B<br>KR 10-2002-0070779 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008111120 A
- WO 2018193954 A
- WO 2020004306 A
- WO 2020066824 A
- WO 2017154345 A
- US 20120135348 A1
- US 20160070167 A1
- US 20150004544 A1
- US 20160237190 A1
- US 20160274458 A1
- WO 201819395 A
- JP 2014059543 A
- JP 2013061648 A


### Non-patent literature cited in the description


- *Materials Letters,* 2008, vol. 62, 3152
- Prediction of polymer properties. Marcel Dekker Inc, 1993